# EUROPEAN PATENT APPLICATION

(11) **EP 4 279 500 A1**
(43) Date of publication of application: **22.11.2023**
(21) Application number: 22739521.7
(22) Date of filing: 17.01.2022
(51) Int. Cl.: C07K 14/00, C07K 16/00, A61K 39/395, A61K 47/64, A61P 35/00, A61P 43/00

(54) **COMPOUND OR SALT THEREOF, AND ANTIBODY PRODUCED USING SAME**

(30) Priority: 18.01.2021 JP 2021005762
(71) Applicant: AJINOMOTO CO., INC., Chuo-ku Tokyo 104-8315 (JP)
(72) Inventor: HATADA, Noriko, Kawasaki-shi, Kanagawa 210-8681 (JP); YAMADA, Kei, Kawasaki-shi, Kanagawa 210-8681 (JP); MATSUDA, Yutaka, Kawasaki-shi, Kanagawa 210-8681 (JP); FUJII, Tomohiro, Kawasaki-shi, Kanagawa 210-8681 (JP)
(74) Representative: Mewburn Ellis LLP
(86) International application number: PCT/JP2022/001358
(87) International publication number: WO 2022/154116

(57) **Abstract**

The present invention provides a compound which facilitates regioselective modification of an antibody with a functional substance and control of the bonding ratio of the antibody to the functional substance within a desired range. More specifically, the present invention provides a compound or salt thereof represented by the following formula (I): 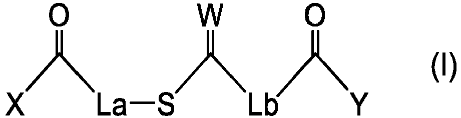 wherein
X indicates a leaving group,
Y indicates an affinity peptide having a binding region to a CH2 domain in an immunoglobulin unit containing two heavy chains and two light chains,
O indicates an oxygen atom,
S indicates a sulfur atom,
W indicates an oxygen atom or a sulfur atom,
La indicates a first linker,
Lb indicates a second linker, and
the total number of atoms constituting a main chain in the first linker and atoms constituting a main chain in the second linker are 5 to 7; and antibodies which can be prepared by using the same.

## Description

### TECHNICAL FIELD

The present invention relates to a compound or salt thereof, and antibody obtained by using the same, and the like.

### BACKGROUND ART

In recent years, research and development of an antibody-drug conjugate (ADC) have been actively conducted. An ADC, as implied by the name, is a medicine in which a drug (e.g., an anti-cancer agent) is conjugated with an antibody and has a direct cytotoxic activity on cancer cells and the like. A typical ADC is T-DM1 (trade name: Kadcyla (registered trademark)) which is jointly developed by Immunogen and Roche.

ADCs including T-DM1 have had the problem of their nonuniformity from the beginning of their development. That is, a small compound drug is randomly reacted with about 70 to 80 lysine residues in an antibody, and thus a drug antibody ratio (DAR) and a conjugation position are not constant. It is known that such a random conjugation method normally provides a DAR within a range of 0 to 8, producing a plurality of antibody medicines having different numbers of bonds of a drug. In recent years, it has been reported that when the number of bonds and the bond positions of a drug of an ADC are changed, pharmacokinetics, and a releasing rate and effects of the drug change. Given these circumstances, next-generation ADCs are required to control the number and positions of a drug to be conjugated. It is believed that when the number and positions are fixed, the problems of expected efficacy, variations in conjugation medicines, and lot difference, or what is called regulation, will be solved.

Although methods for regioselectively modifying antibodies are being investigated worldwide, most of them are methods of modification using genetic engineering techniques or enzymes. For the genetic engineering methods of modification, problems have been pointed out such as reductions in the expression efficiency of antibodies themselves (reductions in total yield when ADCs are prepared), although regioselectivity and number selectivity can be controlled. In addition, there is a problem in that it takes long years to construct an antibody expression system and the like.

In recent years, a chemical conjugation by affinity peptide (CCAP) method which can regioselectively modify an antibody by chemical synthetic technique has recently been developed (Patent Literature 1). This method has succeeded in regioselective modification of antibodies by a method that reacts a peptide reagent in which an NHS-activated ester and a drug are coupled with an affinity peptide with an antibody. However, in the ADC produced by this method, the antibody and the drug are bonded via a linker containing a peptide moiety. The peptide moiety has potential immunogenicity and is susceptible to hydrolysis in the blood. Therefore, the ADC produced by this method has room for improvement in that it contains a peptide moiety in the linker.

As an improved method of the above C-CAP method, techniques which can prepare an antibody which does not contain a peptide moiety as a linker and has a functional substance (e.g., a drug) regioselectively by a chemical synthesis method using a certain compound containing an affinity peptide have been reported (Patent Literature 2 to 6). Avoiding the use of linkers containing peptide moieties is desirable in clinical applications. In these techniques, plural positions corresponding to various amino acid residues in CH2 and CH3 domains (e.g., lysine residues, tyrosine residues, serine residues, and threonine residue) have been proposed as the positions of amino acid residues in antibodies that can be regioselectively modified with drugs. However, it is not necessarily easy to regioselectively modify an antibody with a functional substance and control the bonding ratio of the antibody to the functional substance within a desired range.

### PRIOR ART REFERENCES

### Patent Literature

Patent Literature 1: WO2016/186206
Patent Literature 2: WO2018/199337
Patent Literature 3: WO2019/240287
Patent Literature 4: WO2019/240288
Patent Literature 5: WO2020/009165
Patent Literature 6: WO2020/090979

### DISCLOSURE OF INVENTION

### PROBLEM TO BE SOLVED BY THE INVENTION

An object of the present invention is to provide tehniques for regioselectively modifying an antibody with a functional substance and control the bonding ratio of the antibody to the functional substance within a desired range.

### MEANS FOR SOLVING PROBLEM

As a result of diligent studies, the present inventors have found that by selecting the lysine residue at the position 288/290 of a heavy chain in an immunoglobulin unit as a modification position of an antibody, and by using a specific compound as a compound which can regioselectively modify the lysine residue, the antibody can be regioselectively modified with the functional substance and the bonding ratio between the antibody and the functional substance (number of functional substances/immunoglobulin unit) can be highly controled within a desired range (1.5 to 2.5). Such a specific compound corresponds to a comound in which the total number of atoms constituting a main chain connecting the reactive portion with the lysine residue at the 288/290 position of the heavy chain in the immunoglobulin unit [X (leaving group)-C=O] and the binding portion with the affinity peptide [O=C-Y (affinity peptide)] is 7 to 9 (that is, in the compound represented by formula (I), the compound in which the total number of atoms constituting a main chain in a first linker and atoms constituting a main chain in a second linker are 5 to 7). Based on such findings, the present inventors have succeeded in developing a compound or salt thereof represented by formula (I), and a reagent for derivatizing the antibody, which comprising the same. The present inventors also found tht by using a compound or salt thereof represented by formula (I), a specific antibody, that is, an antibody in which the lysine residue at the position 288/290 in the antibody are regioselectively modified with a modifying group, and the bonding ratio between the antibody and the modifying group (number of modifying group/immunoglobulin unit) is highly controled within a desired range (1.5 to 2.5) (an antibody intermediate, a thiol group-introduced antibody, and a conjugate of an antibody and a functional substance) can be prepared and have completed the present invention.

That is, the present invention is the following.
[1] A compound or salt thereof represented by formula (I).
[2] The compound or salt thereof of [1], wherein the leaving group is selected from the following:
   (A) R-S wherein R indicates a hydrogen atom, a monovalent hydrocarbon group which may have a substituent, or a monovalent heterocyclic group which may have a substituent, and S indicates a sulfur atom;
   (B) R-O wherein R indicates a hydrogen atom, a monovalent hydrocarbon group which may have a substituent, or a monovalent heterocyclic group which may have a substituent, and O indicates an oxygen atom;
   (C) R_{A}-(R_{B}-)N wherein R_{A} and R_{B} each independently indicate a hydrogen atoms, a monovalent hydrocarbon group which may have a substituent, or a monovalent heterocyclic group which may have a substituent, and N indicates a nitrogen atom; or
   (d) a halogen atom.
[3] The compound or salt thereof of [1] or [2], wherein the immunoglobulin unit is a human immunoglobulin unit.
[4] The compound or salt thereof of any of [1] to [3], wherein the immunoglobulin unit is human IgG.
[5] The compound or salt thereof of any of [1] to [4],
   wherein the affinity peptide contains a lysine residue and forms an amide bond with a carbonyl group (C=O) adjacent to Y via an amino group in a side chain of the lysine residue.
[6] The compound or salt thereof of [1] to [5], wherein the affinity peptide is the following:
   (A) Affinity peptide comprising the amino acid sequence of CQRRFYEALHDPNLNEEQRNARIRSIKDDC (SEQ ID NO: 1);
   (B) An affinity peptide comprising an amino acid sequence comprising a mutation of 1 to 5 amino acid residues which is selected from the group consisting of substitution, insertion, deletion and addition of amino acid residues in the amino acid sequence of CQRRFYEALHDPNLNEEQRNARIRSIKDDC (SEQ ID NO: 1),
   wherein the lysine residue at the position 27 and the two cysteine residues at the positions 1 and 30 are maintained.
[7] The compound or salt thereof of [6], wherein the affinity peptide (B) is selected from the group consisting of the following:
   (A) Affinity peptide comprising the amino acid sequence of FNMQCQRRFYEALHDPNLNEEQRNARIRSIKDDC (SEQ ID NO: 2);
   (B) Affinity peptide comprising the amino acid sequence of FNMQCQRRFYEALHDPNLNEEQRNARIRSIKEDC (SEQ ID NO: 3);
   (C) Affinity peptide comprising the amino acid sequence of FNMQCQRRFYEALHDPNLNEEQRNARIRSIKEEC (SEQ ID NO: 4);
   (D) Affinity peptide comprising the amino acid sequence of NMQCQRRFYEALHDPNLNEEQRNARIRSIKEEC (SEQ ID NO: 5);
   (E) Affinity peptide comprising the amino acid sequence of MQCQRRFYEALHDPNLNEEQRNARIRSIKEEC (SEQ ID NO: 6); and
   (f) Affinity peptide comprising the amino acid sequence of QCQRRFYEALHDPNLNEEQRNARIRSIKEEC (SEQ ID NO: 7).
[8] The compound or salt thereof of [6] or [7], wherein the N-terminal and C-terminal amino acid residues in the affinity peptide may be protected, and the two thiol groups in side chains of the two cysteine residues (C) in the affinity peptide may be linked by a disulfide bond, or via a linker.
[9] The compound or salt thereof of any of [1] to [8], wherein the compound represented by formula (I) is represented by formula (I').
[10] The compound or salt thereof of [9], wherein the compound represented by formula (I') is represented by formula (I' ').
[11] A reagent for derivatizing an antibody, which comprises a compound or salt thereof represented by formula (I) .
[12] The reagent of [11], wherein the compound represented by formula (I) is represented by formula (I').
[13] The reagent according to [12], wherein the compound represented by formula (I') is represented by formula (I").
[14] An antibody intermediate or salt thereof comprising a structural unit represented by formula (II).
[15] The antibody intermediate or salt thereof of [14], wherein the antibody intermediate is a human antibody intermediate.
[16] The antibody intermediate or salt thereof of [14] or [15], wherein the antibody intermediate is a human IgG intermediate.
[17] The antibody intermediate or salt thereof of any of [14] to [16], wherein the structural unit represented by formula (II) is represented by formula (II').
[18] The antibody intermediate or salt thereof of [17], wherein the structural unit represented by formula (II') is represented by formula (II'').
[19] A thiol group-introduced antibody derivative or salt thereof, which comprises a structural unit represented by formula (III).
[20] The thiol group-introduced antibody derivative or salt thereof of [19], wherein a specific amino acid residue other than the lysine residue present at the position 288/290 in the two heavy chains is further modified.
[21] The thiol group-introduced antibody derivative or salt thereof of [20], wherein the specific amino acid residue is a lysine residue present at the position 246/248 in the two heavy chains.
[22] The thiol group-introduced antibody derivative or salt thereof of any of [19] to [21], wherein the structural unit represented by formula (III) is formula (III').
[23] The thiol group-introduced antibody derivative or salt thereof of [22], wherein the structural unit represented by formula (III') is formula (III'').
[24] A conjugate of an antibody and a functional substance or a salt thereof, which comprises a structural unit represented by formula (IV).
[25] The conjugate or salt thereof of [24], wherein a specific amino acid residue other than the lysine residue present at the position 288/290 in the two heavy chains is further modified.
[26] The conjugate or salt thereof of [25], wherein the specific amino acid residue is a lysine residue present at position 246/248 in two heavy chains.
[27] The conjugate or salt thereof of any of [24] to [26], wherein the structural unit represented by formula (IV) is represented by formula (IV').
[28] The conjugate or salt thereof of [27], wherein the structural unit represented by formula (IV') is represented by formula (IV").
[29] A compound or salt thereof represented by formula (V).
[30] The compound or salt thereof of [29], wherein the compound represented by formula (V) is represented by formula (V').
[31] The compound or salt thereof of [30], wherein the compound represented by formula (V') is represented by formula (V'').
[32] The compound represented by formula (V'') is formula (V''-1) or (V''-2).
[33] A compound or salt thereof represented by formula (VI) .
[34] The compound or salt thereof of [33], wherein the leaving group having a leaving ability which is higher than that of the leaving group X is a pentafluorophenyloxy group, a tetrafluorophenyloxy group, a paranitrophenyloxy group, or an N-succinimidyloxy group.
[35] The compound or salt thereof of [33] or [34], wherein the compound represented by formula (VI) is represented by formula (VI').
[36] The compound or salt thereof of [35], wherein the compound represented by formula (VI') is represented by formula (VI'').
[37] The compound or salt thereof of [36], wherein the compound represented by formula (VI'') is represented by formula (VI''-1) or (VI''-2).
[38] A method for producing an antibody intermediate or salt thereof, which comprises reacting a compound or salt thereof represented by formula (I) with an antibody comprising the immunoglobulin unit to give an antibody intermediate or salt thereof represented by formula (II).
[39] A method for producing a thiol group-introduced antibody derivative or salt thereof, which comprises
   (1) reacting a compound or salt thereof represented by formula (I) with an antibody comprising the immunoglobulin unit to give an antibody intermediate or salt thereof represented by formula (II); and
   (2) subjecting the antibody intermediate or salt thereof to a thioester cleavage reaction to give a thiol group-introduced antibody derivative or salt thereof comprising a structural unit represented by formula (III).
[40] A method for producing a conjugate of an antibody and a functional substance or a salt thereof, which comprises
   (1) reacting a compound or salt thereof represented by formula (I) with an antibody comprising the immunoglobulin unit to give an antibody intermediate or salt thereof represented by formula (II);
   (2) subjecting the antibody intermediate or salt thereof to a thioester cleavage reaction to give a thiol group-introduced antibody derivative or salt thereof comprising a structural unit represented by formula (III); and
   (3) reacting the thiol group-introduced antibody derivative or salt thereof with a functional substance to give a conjugate of an antibody and a functional substance or a salt thereof, which comprises a structural unit represented by formula (IV).
[41] The method of any of [38] to [40], further comprising reacting a compound or salt thereof represented by formula (VI) with the affinity peptide having a binding region to the CH2 domain in the immunoglobulin unit containing two heavy chains and two light chains to give the compound or salt thereof represented by the formula (I).
[42] The method of any of [38] to [40], further comprising (1') reacting a compound or salt thereof represented by formula (V) with a carboxyl group modifying reagent to give a compound or salt thereof represented by formula (VI); and (2') reacting the compound or salt thereof represented by the formula (VI) with an immunoglobulin unit containing two heavy chains and two light chains to give a compound or salt thereof represented by formula (I).
[43] A method for producing a compound or salt thereof represented by formula (VI), which comprises reacting a compound or salt thereof represented by formula (V) with a carboxyl group modifying reagent to give the compound or salt thereof represented by formula (VI).
[44] A method for producing a thiol group-introduced antibody derivative or salt thereof, which comprises subjecting an antibody intermediate or salt thereof comprising a structural unit represented by formula (II) to a thioester cleavage reaction to give a thiol group-introduced antibody derivative or salt thereof, which comprises a structural unit represented by formula (III).
[45] A method for producing a conjugate of an antibody and a functional substance or a salt thereof, which comprises
   (1) subjecting an antibody intermediate or salt thereof comprising a structural unit represented by formula (II) to a thioester cleavage reaction to give a thiol group-introduced antibody derivative or salt thereof, which comprises a structural unit represented by formula (III); and
   (2) reacting the thiol group-introduced antibody derivative or salt thereof with a functional substance to give the conjugate of the antibody and the functional substance or a salt thereof which is represented by formula (IV).
[46] A method for producing a conjugate of an antibody and a functional substance or a salt thereof, which comprises reacting a thiol group-introduced antibody derivative or salt thereof, which comprises a structural unit represented by formula (III) with a functional substance to give a conjugate of an antibody and a functional substance or a salt thereof, which comprises a structural unit represented by formula (IV).

### Effect of Invention

The compound or salt thereof represented by formula (I) can specifically and highly modify the lysine residue at the position 288/290 of a heavy chain in an immunoglobulin unit so as to be the average ratio of the bonds between the immunoglobulin unit and the affinity peptide-containing group (the number of affinity peptide-containing groups/immunoglobulin unit) in a desired range (1.5 to 2). Therefore, the compound or salt thereof represented by formula (I) is useful as a reagent for derivatizing an antibody.

In addition, the compound or salt thereof represented by formula (I) can provide an antibody intermediate or salt thereof represented by formula (II) in which the lysine residue at the position 288/290 of the heavy chain in the immunoglobulin unit is specifically modified with an affinity peptide-containing group, and the average ratio of the bonds between the immunoglobulin unit and the affinity peptide-containing group (number of affinity peptide-containing group/immunoglobulin unit) is highly controlled within a desired range.

Furthermore, an antibody prepared by using the antibody intermediate or salt thereof represented by formula (II) as a raw material can inherit the regioselectivity and the average bonding ratio of the antibody intermediate or salt thereof. Therefore, a thiol group-introduced antibody derivative or salt thereof represented by formula (III) and a conjugate of an antibody and a functional substance represented by the formula (IV) or a salt thereof, which has the above regioselectivity and the average bonding ratio can be provided.

Also provided are compounds or salts thereof represented by formulae (V) and (VI) which are synthetic intermediates that enable efficient production of the compound or salt thereof represented by formula (I).

### BRIEF DESCRIPTION OF DRAWINGS

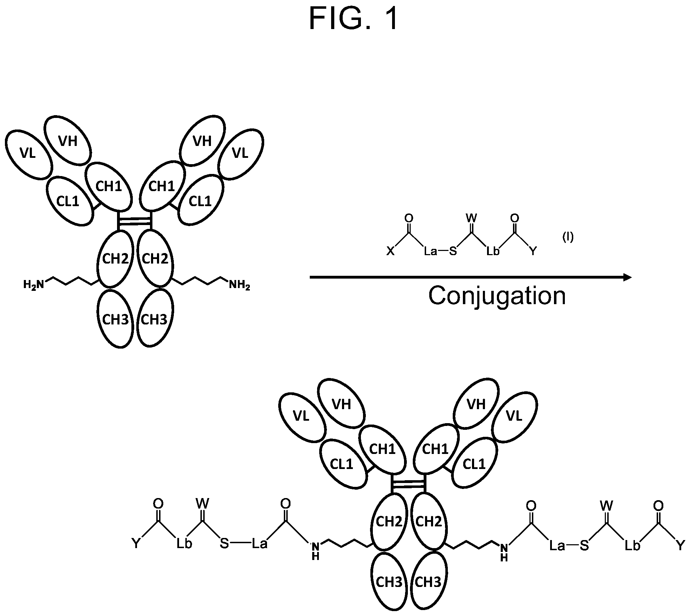

FIG. 1 is a schematic diagram (1) showing the concept of modification of an immunoglobulin unit with the compound of the present invention or salt thereof represented by the formula (I). First, the compound of the present invention or salt thereof represented by the formula (I) associates with the CH2 domain in the immunoglobulin unit via an affinity peptide (Y). Next, the compound of the present invention or salt thereof represented by the formula (I) reacts with a side chain of a specific amino acid residue in the CH2 domain (In the figure, an amino group in a side chain of a lysine residue) via an activated carbonyl group having a leaving group (X).

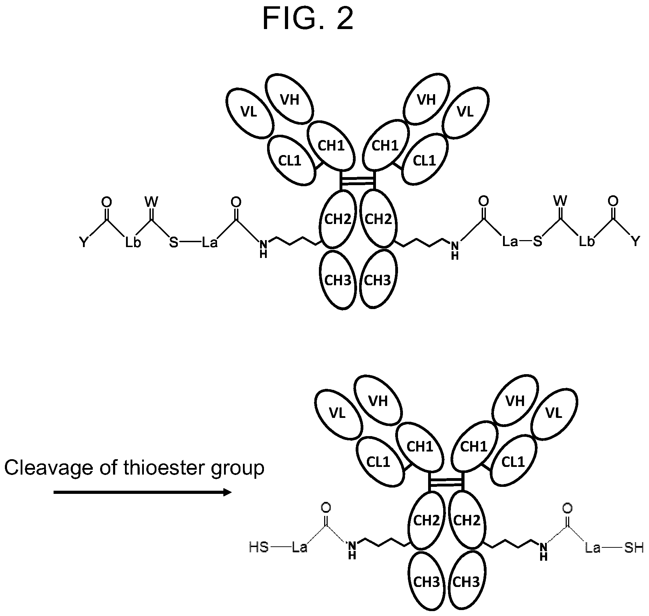

FIG. 2 is a schematic diagram (2) showing the concept of modification of an immunoglobulin unit with the compound of the present invention or salt thereof represented by the formula (I). Cleavage of the thioester group produces a thiol group-introduced antibody derivative or a salt thereof.

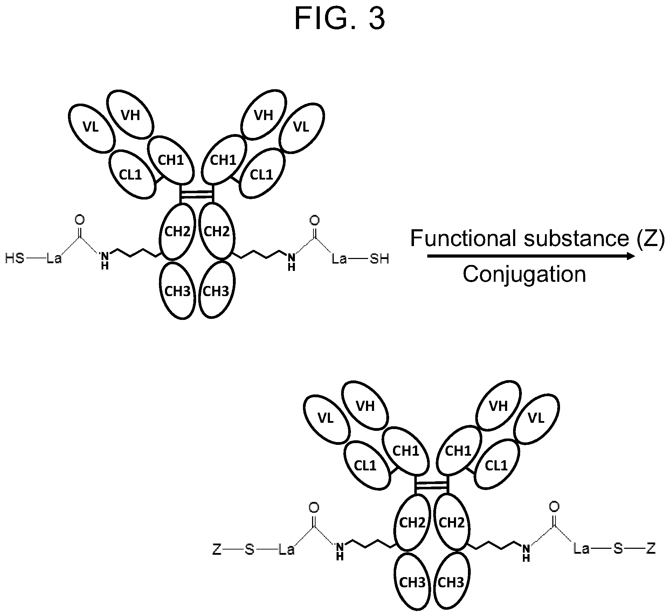

FIG. 3 is a schematic diagram (3) showing the concept of modification of an immunoglobulin unit with the compound of the present invention or salt thereof represented by the formula (I). The reaction of a thiol group with a functional substance (Z) in a thiol group-introduced antibody derivative or a salt thereof produces a conjugate of an antibody and a functional substance or a salt thereof.

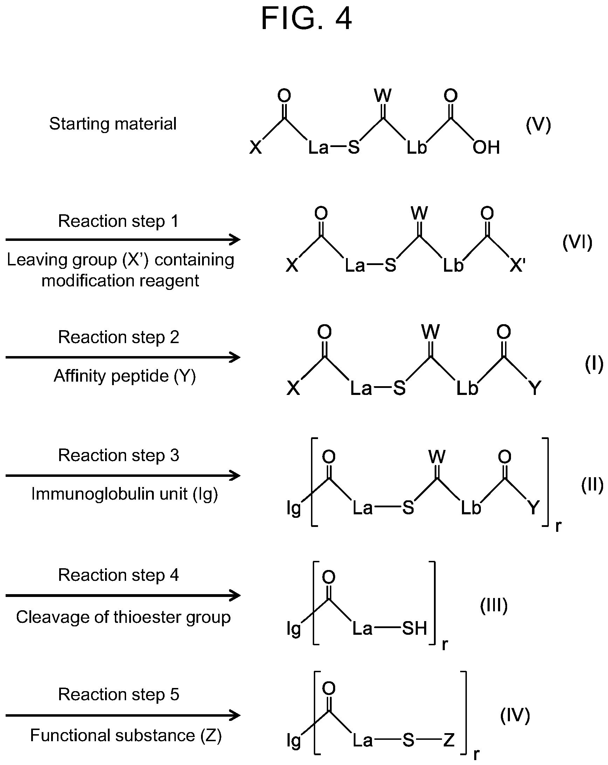

FIG. 4 is a diagram showing an outline of an embodiment of the present invention.

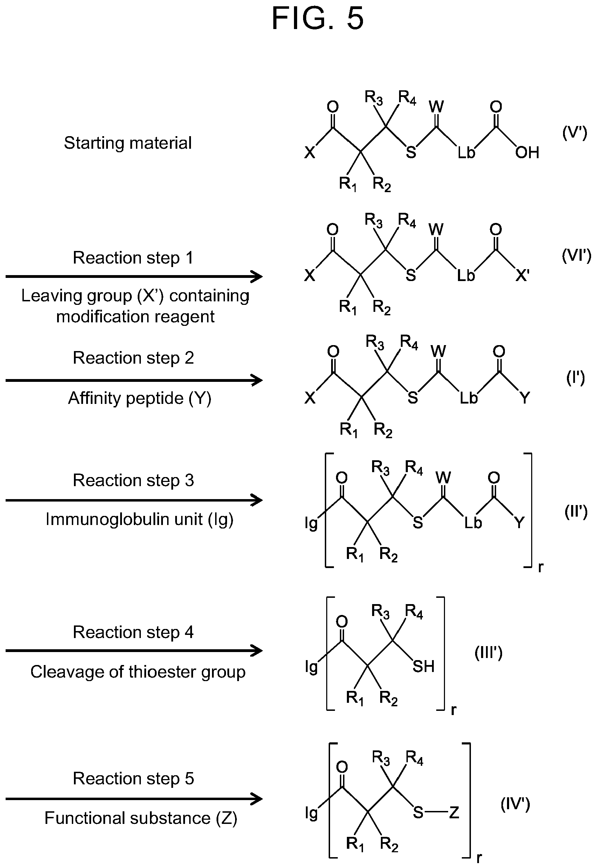

FIG. 5 is a diagram showing an outline of a preferred embodiment of the present invention.

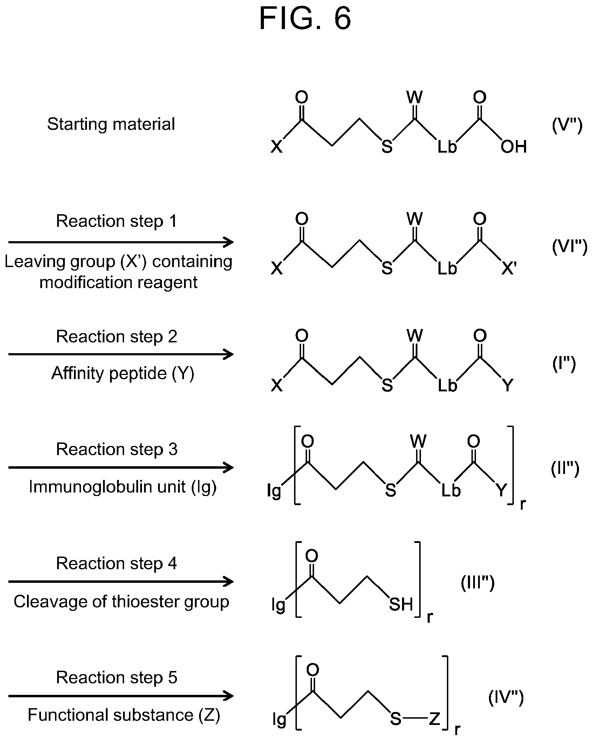

FIG. 6 is a diagram showing an outline of a more preferable embodiment of the present invention.
FIG. 7 is a diagram showing ESI-TOFMS analysis of specific modification (number of introduced binding peptides) of trastuzumab (anti-HER2 IgG antibody) (Example 1) .
FIG. 8 is a diagram showing ESI-TOFMS analysis of specific modification (heavy chain selectivity) of trastuzumab (Example 1).
FIG. 9 is a diagram showing (1) the amino acid sequence of the heavy chain of trastuzumab (SEQ ID NO: 8) and (2) the amino acid sequence of the light chain of trastuzumab (SEQ ID NO: 9).
FIG. 10 shows MS spectrum (measured value: m/z 577.03606, theoretical value: 577.03557, tetravalent) of the peptide fragment for a peptide consisting of 18 amino acid residues FNWYVDGVEVHNAKTTKPR (SEQ ID NO: 10) containing a site of modification of trussumab to a lysine residue by trypsin digestion (a thiol-introduced product (+145.09 Da) which has been carbamidomethylated with iodoacetamide) (Example 1-9-4).
FIG. 11 shows CID spectrum of a product ion of m/z 682.13 (theoretical value: 682.01) corresponding to trivalent y16, which indicates the modification of the lysine residue at position 288/290 of the human IgG heavy chain in EU numbering (Example 1-9-4).
FIG. 12 shows the results of searching for a peptide fragment containing a modification to a lysine residue (thiol-introduced product which has been carbamidemethylated with iodoacetamide (+145.09Da)) in a trypsin digested product of trastuzumab using BioPharma Finder (Example 1-9-4). The horizontal axis shows the identified lysine residue, and the vertical axis shows the Integrity.
FIG. 13 is a diagram showing ESI-TOFMS analysis of specific modification of trastuzumab (number of introduced binding peptides) (Example 2).
FIG. 14 is a diagram showing ESI-TOFMS analysis of specific modification (heavy chain selectivity) of trastuzumab (Example 2).
FIG. 15 shows MS spectrum (measured value: m/z 577.03571, theoretical value: 577.03557, tetravalent) of the peptide fragment for a peptide consisting of 18 amino acid residues FNWYVDGVEVHNAKTTKPR (SEQ ID NO: 10) containing a site of modification of trussumab to a lysine residue by trypsin digestion (a thiol-introduced product (+145.09 Da) which has been carbamidomethylated with iodoacetamide) (Example 2-7-4).
FIG. 16 shows CID spectrum of a product ion of m/z 682.41 (theoretical value: 682.01) corresponding to trivalent y16, which indicates the modification of the lysine residue at position 288/290 of the human IgG heavy chain in EU numbering (Example 2-7-4).
FIG. 17 shows the results of searching for a peptide fragment containing a modification to a lysine residue (thiol-introduced product which has been carbamidemethylated with iodoacetamide (+145.09Da)) in a trypsin digested product of trastuzumab using BioPharma Finder (Example 2-7-4). The horizontal axis shows the identified lysine residue, and the vertical axis shows the Integrity.
FIG. 18 is a diagram showing ESI-TOFMS analysis of specific modification of trastuzumab (number of introduced binding peptides) (Example 3).
FIG. 19 is a diagram showing ESI-TOFMS analysis of specific modification (heavy chain selectivity) of trastuzumab (Example 3).
FIG. 20 is a diagram showing ESI-TOFMS analysis of specific modification of trastuzumab (number of introduced binding peptides) (Example 4).
FIG. 21 is a diagram showing ESI-TOFMS analysis of specific modification (heavy chain selectivity) of trastuzumab (Example 4).
FIG. 22 is a diagram showing ESI-TOFMS analysis of specific modification of trastuzumab (number of introduced binding peptides) (Example 5).
FIG. 23 is a diagram showing ESI-TOFMS analysis of specific modification (heavy chain selectivity) of trastuzumab (Example 5).
FIG. 24 is a diagram showing ESI-TOFMS analysis of specific modification of trastuzumab (number of introduced binding peptides) (Example 6).
FIG. 25 is a diagram showing ESI-TOFMS analysis of specific modification (heavy chain selectivity) of trastuzumab (Example 6).
FIG. 26 is a diagram showing ESI-TOFMS analysis of specific modification of trastuzumab (number of introduced binding peptides) (Example 7).
FIG. 27 is a diagram showing ESI-TOFMS analysis of specific modification (heavy chain selectivity) of trastuzumab (Example 7).
FIG. 28 shows MS spectrum (measured value: m/z 769.04506, theoretical value: 769.04482, trivalent) of the peptide fragment for a peptide consisting of 18 amino acid residues FNWYVDGVEVHNAKTTKPR (SEQ ID NO: 10) containing a site of modification of trussumab to a lysine residue by trypsin digestion (a thiol-introduced product (+145.09 Da) which has been carbamidomethylated with iodoacetamide) (Example 7-6-4).
FIG. 29 shows CID spectrum of a product ion of m/z 1022.71 (theoretical value: 1022.51) corresponding to divalent y16, which indicates the modification of the lysine residue at position 288/290 of the human IgG heavy chain in EU numbering (Example 7-6-4).
FIG. 30 shows the results of searching for a peptide fragment containing a modification to a lysine residue (thiol-introduced product which has been carbamidemethylated with iodoacetamide (+145.09Da)) in a trypsin digested product of trastuzumab using BioPharma Finder (Example 7-6-4). The horizontal axis shows the identified lysine residue, and the vertical axis shows the Integrity.
FIG. 31 is a diagram showing ESI-TOFMS analysis of specific modification of trastuzumab (number of introduced binding peptides) (Example 8).
FIG. 32 is a diagram showing ESI-TOFMS analysis of specific modification (heavy chain selectivity) of trastuzumab (Example 8).
FIG. 33 is a diagram showing ESI-TOFMS analysis of specific modification of trastuzumab (number of introduced binding peptides) (Example 9).
FIG. 34 is a diagram showing ESI-TOFMS analysis of specific modification (heavy chain selectivity) of trastuzumab (Example 9).

### EMBODIMENTS FOR CARRYING OUT THE INVENTION

### 1.Definitions of general terms

In the present invention, the term "antibody" is as follows. The term "immunoglobulin unit" corresponds to a divalent monomer unit that is a basic constituent element of such an antibody, and is a unit comprising two heavy chains and two light chains. Therefore, definitions, examples, and preferred examples of the origin, type (polyclonal or monoclonal, isotype, and full-length antibody or antibody fragment), antigen, position of a lysine residue, and regioselectivity of the immunoglobulin unit are similar to those of the antibody described below.

The origin of the antibody is not particularly limited, and for example, the antibody may be derived from an animal such as a mammal or a bird (e.g., a domestic fowl). The immunoglobulin unit is preferably derived from a mammal. Examples of such a mammal include primates (e.g., humans, monkeys, and chimpanzees), rodents (e.g., mice, rats, guinea pigs, hamsters, and rabbits), pets (e.g., dogs and cats), domestic animals (e.g., cows, pigs, and goats), and work animals (e.g., horses and sheep). Primates and rodents are preferred, and humans are more preferred.

The type of antibody may be a polyclonal antibody or a monoclonal antibody. The antibody may be a divalent antibody (e.g., IgG, IgD, or IgE) or a tetravalent or higher antibody (e.g., IgA antibody or IgM antibody). The antibody is preferably a monoclonal antibody. Examples of the monoclonal antibody include chimeric antibodies, humanized antibodies, human antibodies, antibodies with a certain sugar chain added (e.g., an antibody modified so as to have a sugar chain-binding consensus sequence such as an N-type sugar chain-binding consensus sequence), bi-specific antibodies, Fc region proteins, and Fc-fusion proteins. Examples of the isotype of the monoclonal antibody include IgG (e.g., IgG1, IgG2, IgG3, and IgG4), IgM, IgA, IgD, IgE, and IgY. In the present invention, as the monoclonal antibody, a full-length antibody or a variable region, and an antibody fragment comprising a CH1 domain and a CH2 domain can be used, but a full-length antibody is preferred. The antibody is preferably a human IgG monoclonal antibody, and more preferably a human IgG full-length monoclonal antibody.

As an antigen of the antibody, any antigen can be used. Examples of such an antigen include a protein [which comprises an oligopeptide and a polypeptide, and may be a protein modified with a biomolecule such as a sugar (e.g., a glycoprotein)], a sugar chain, a nucleic acid, and a small compound. The antibody may be preferably an antibody with a protein as an antigen. Examples of the protein include cell membrane receptors, cell membrane proteins other than cell membrane receptors (e.g., extracellular matrix proteins), ligands, and soluble receptors.

More specifically, the protein as the antigen of the antibody may be a disease target protein. Examples of the disease target protein include the following.

### (1) Cancerous Region

PD-L1, GD2, PDGFRα (a platelet-derived growth factor receptor), CD22, HER2, phosphatidyl serine (PS), EpCAM, fibronectin, PD-1, VEGFR-2, CD33, HGF, gpNMB, CD27, DEC-205, folic acid receptors, CD37, CD19, Trop2, CEACAM5, S1P, HER3, IGF-1R, DLL4, TNT-1/B, CPAAs, PSMA, CD20, CD105 (Endoglin), ICAM-1, CD30, CD16A, CD38, MUC1, EGFR, KIR2DL1, KIR2DL2, NKG2A, tenascin-C, IGF (insulin-like growth factor), CTLA-4, mesothelin, CD138, c-Met, Ang2, VEGF-A, CD79b, ENPD3, folic acid receptor α, TEM-1, GM2, Glypican 3, macrophage inhibitory factor, CD74, Notch1, Notch2, Notch3, CD37, TLR-2, CD3, CSF-1R, FGFR2b, HLA-DR, GM-CSF, EphA3, B7-H3, CD123, gpA33, Frizzled7 receptor, DLL4, VEGF, RSPO, LIV-1, SLITRK6, Nectin-4, CD70, CD40, CD19, SEMA4D (CD100), CD25, MET, Tissue Factor, IL-8, EGFR, cMet, KIR3DL2, Bst1 (CD157), P-Cadherin, CEA, GITR, TAM (tumor associated macrophage), CEA, DLL4, Ang2, CD73, FGFR2, CXCR4, LAG-3, GITR, Fucosyl GM1, IGF-1, Angiopoietin 2, CSF-1R, FGFR3, OX40, BCMA, ErbB3, CD137 (4-1BB), PTK7, EFNA4, FAP, DR5, CEA, Ly6E, CA6, CEACAM5, LAMP1, tissue factor, EPHA2, DR5, B7-H3, FGFR4, FGFR2, α2-PI, A33, GDF15, CAIX, CD166, ROR1, GITR, BCMA, TBA, LAG-3, EphA2, TIM-3, CD-200, EGFRvIII, CD16A, CD32B, PIGF, Axl, MICA/B, Thomsen-Friedenreich, CD39, CD37, CD73, CLEC12A, Lgr3, transferrin receptors, TGFβ, IL-17, 5T4, RTK, Immune Suppressor Protein, NaPi2b, Lewis blood group B antigen, A34, Lysil-Oxidase, DLK-1, TROP-2, α9 Integrin, TAG-72 (CA72-4), and CD70.

(2) Autoimmune Diseases and Inflammatory Diseases IL-17, IL-6R, IL-17R, INF-α, IL-5R, IL-13, IL-23, IL-6, ActRIIB, β7-Integrin, IL-4αR, HAS, Eotaxin-1, CD3, CD19, TNF-α, IL-15, CD3ε, Fibronectin, IL-1β, IL-1α, IL-17, TSLP (Thymic Stromal Lymphopoietin), LAMP (Alpha4 Beta 7 Integrin), IL-23, GM-CSFR, TSLP, CD28, CD40, TLR-3, BAFF-R, MAdCAM, IL-31R, IL-33, CD74, CD32B, CD79B, IgE (immunoglobulin E), IL-17A, IL-17F, C5, FcRn, CD28, TLR4, MCAM, B7RP1, CXCR1/2 Ligands, IL-21, Cadherin-11, CX3CL1, CCL20, IL-36R, IL-10R, CD86, TNF-α, IL-7R, Kv1.3, α9 Integrin, and LIFHT.

### (3) Brain or Nerve Diseases

CGRP, CD20, β amyloid, β amyloid protofibril, Calcitonin Gene-Related Peptide Receptor, LINGO (Ig Domain Containing 1), α Synuclein, extracellular tau, CD52, insulin receptors, tau protein, TDP-43, SOD1, TauC3, and JC virus.

### (4) Infectious Diseases

Clostridium Difficile toxin B, cytomegalovirus, RS viruses, LPS, S. Aureus Alpha-toxin, M2e protein, Psl, PcrV, S. Aureus toxin, influenza A, Alginate, Staphylococcus aureus, PD-L1, influenza B, Acinetobacter, F-protein, Env, CD3, enteropathogenic Escherichia coli, Klebsiella, and Streptococcus pneumoniae.

### (5) Hereditary Rare Diseases

amyloid AL, SEMA4D (CD100), insulin receptors, ANGPTL3, IL4, IL13, FGF23, adrenocorticotropic hormone, transthyretin, and huntingtin.

### (6) Eye Diseases

Factor D, IGF-1R, PGDFR, Ang2, VEGF-A, CD-105 (Endoglin), IGF-1R, and β amyloid.

### (7) Bone and Orthopedic Region

Sclerostin, Myostatin, Dickkopf-1, GDF8, RNAKL, HAS, and Siglec-15

### (8) Blood Diseases

vWF, Factor IXa, Factor X, IFNγ, C5, BMP-6, Ferroportin, and TFPI

### (9) Other Diseases

BAFF (B cell activating factor), IL-1β, PCSK9, NGF, CD45, TLR-2, GLP-1, TNFR1, C5, CD40, LPA, prolactin receptors, VEGFR-1, CB1, Endoglin, PTH1R, CXCL1, CXCL8, IL-1β, AT2-R, and IAPP.

Specific examples of the monoclonal antibody include specific chimeric antibodies (e.g., rituximab, basiliximab, infliximab, cetuximab, siltuximab, dinutuximab, and altertoxaximab), specific humanized antibodies (e.g., daclizumab, palivizumab, trastuzumab, alemtuzumab, omalizumab, efalizumab, bevacizumab, natalizumab (IgG4), tocilizumab, eculizumab (IgG2), mogamulizumab, pertuzumab, obinutuzumab, vedolizumab, pembrolizumab (IgG4), mepolizumab, elotuzumab, daratumumab, ixekizumab (IgG4), reslizumab (IgG4), and atezolizumab), and specific human antibodies (e.g., adalimumab (IgG1), panitumumab, golimumab, ustekinumab, canakinumab, ofatumumab, denosumab (IgG2), ipilimumab, belimumab, raxibacumab, ramucirumab, nivolumab, dupilumab (IgG4), secukinumab, evolocumab (IgG2), alirocumab, necitumumab, brodalumab (IgG2), and olaratumab) (cases not referring to the IgG subtype indicate that they are IgG1).

The positions of a lysine residue in the antibody and the position of a constant region of a heavy chain (e.g., CH2 domain) are in accordance with EU numbering (see, http://www.imgt.org/IMGTScientificChart/Numbering/Hu_IGHGnb er. html). Therefore, when human IgG is a target, a lysine residue at position 288 corresponds to an amino acid residue at position 58 of a human IgG CH2 region, and a lysine residue at position 290 corresponds to an amino acid residue at position 60 of a human IgG CH2 region. The notation at position 288/290 indicates that a lysine residue at position 288 or position 290 is a target.

According to the present invention, a lysine residue at position 288/290 in an antibody can be regioselectively modified. In the present specification, "regioselective" or "regioselectivity" refers to a state in which even though a specific amino acid residue is not present locally in a specific region in the antibody, a certain structural unit capable of binding to the specific amino acid residue in the antibody is present locally in a specific region in the antibody. Consequently, expressions related to regioselectivity such as "regioselectively having," "regioselective binding," and "binding with regioselectivity" mean that the possession rate or the binding rate of a certain structural unit in the target region comprising one or more specific amino acid residues is higher at a significant level than the possession rate or the binding rate of the structural unit in the non-target region comprising a plurality of amino acid residues of the same type as the specific amino acid residues in the target region. Such regioselectivity may be 50% or more, preferably 60% or more, more preferably 70% or more, even more preferably 80% or more, and particularly preferably 90% or more, 95% or more, 96% or more, 97% or more, 98% or more, 99% or more, 99.5% or more, or 100%. According to the present invention, the lysine residue at position 288/290 can be regioselectively modified without utilizing a linker containing a peptide. The peptide moiety has potential immunogenicity and is susceptible to hydrolysis in a blood. Therefore, avoiding the use of a linker containing a peptide moiety is desirable in clinical applications.

In the present invention, as long as a lysine residue at position 288/290 in an antibody is regioselectively modified, a specific lysine residue at another position may be further regioselectively modified. For example, a method for regioselectively modifying a specific amino acid residue at a predetermined position in an antibody is described in WO 2018/199337 A, WO 2019/240288 A, WO 2019/240287 A, and WO 2020/090979 A. As such a specific amino acid residue, an amino acid residue (e.g., a lysine residue, an aspartic acid residue, a glutamic acid residue, an asparagine residue, a glutamine residue, a threonine residue, a serine residue, a tyrosine residue, or a cysteine residue) having a side chain that is easily modified (e.g., an amino group, a carboxy group, an amide group, a hydroxy group, or a thiol group) can be used. However, a lysine residue having a side chain comprising an amino group, a tyrosine residue having a side chain comprising a hydroxy group, a serine residue, a threonine residue, or a cysteine residue having a side chain comprising a thiol group may be preferred, a lysine residue may be more preferred, a lysine residue at position 246/248, and a lysine residue at position 317 may be even more preferred, and a lysine residue at position 246/248 are particulary preferred. The notation at position 246/248 indicates that the lysine residue at position 246 or 248 is target.

In the present invention, examples of the term "salt" include salts with inorganic acids, salts with organic acids, salts with inorganic bases, salts with organic bases, and salts with amino acids. Examples of salts with inorganic acids include salts with hydrogen chloride, hydrogen bromide, phosphoric acid, sulfuric acid, and nitric acid. Examples of salts with organic acids include salts with formic acid, acetic acid, trifluoroacetic acid, lactic acid, tartaric acid, fumaric acid, oxalic acid, maleic acid, citric acid, succinic acid, malic acid, benzenesulfonic acid, and p-toluenesulfonic acid. Examples of salts with inorganic bases include salts with alkali metals (e.g., sodium and potassium), alkaline-earth metals (e.g., calcium and magnesium), other metals such as zinc and aluminum, and ammonium. Examples of salts with organic bases include salts with trimethylamine, triethylamine, propylenediamine, ethylenediamine, pyridine, ethanolamine, monoalkyl ethanolamine, dialkyl ethanolamine, diethanolamine, and triethanolamine. Examples of salts with amino acids include salts with basic amino acids (e.g., arginine, histidine, lysine, and ornithine) and acidic amino acids (e.g., aspartic acid and glutamic acid). The salt is preferably a salt with an inorganic acid (e.g., hydrogen chloride) or a salt with an organic acid (e.g., trifluoroacetic acid).

### 1. Compound or salt

The present invention provide a compound or salt thereof represented by the following formula (I): 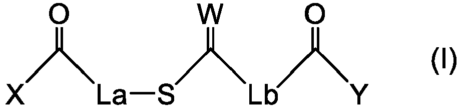 wherein
X indicates a leaving group,
Y indicates an affinity peptide having a binding region to a CH2 domain in an immunoglobulin unit containing two heavy chains and two light chains,
O indicates an oxygen atom,
S indicates a sulfur atom,
W indicates an oxygen atom or a sulfur atom,
La indicates a first linker,
Lb indicates a second linker, and
the total number of atoms constituting a main chain in the first linker and atoms constituting a main chain in the second linker are 5 to 7.

In formula (I) and other formulae presented in relation to the present invention, -(hyphen) indicates that two units (e.g., atoms or groups) present on both sides thereof are covalently bonded to each other. Therefore, in the formula (I), X is covalently bonded to the carbon atom constituting the carbonyl group, La is covalently bonded to the carbon atom constituting the carbonyl group and S, and S is covalently bonded to La and the carbon atom constituting the carbonyl group, Lb is covalently bonded to the two carbon atoms constituting the two carbonyl groups present on both sides, and Y is covalently bonded to a carbon atom constitutes the carbonyl group.

The leaving group represented by X is a group that can be eliminated by a reaction between a carbon atom of a carbonyl group adjacent to X and an amino group. A person skilled in the art can appropriately design such a leaving group. Examples of such leaving groups include:
(a) R-S wherein R indicates a hydrogen atom, a monovalent hydrocarbon group which may have a substituent, or a monovalent heterocyclic group which may have a substituent, and S indicates a sulfur atom;
(b) R-O wherein R indicates a hydrogen atom, a monovalent hydrocarbon group which may have a substituent, or a monovalent heterocyclic group which may have a substituent, and O indicates an oxygen atom;
(c) R_{A}-(R_{B}-)N wherein R_{A} and R_{B} each independently indicate a hydrogen atoms, a monovalent hydrocarbon group which may have a substituent, or a monovalent heterocyclic group which may have a substituent, and N indicates a nitrogen atom; or
(d) a halogen atom.

Preferably, the leaving group represented by X may be:
(a) R-S wherein R indicates a hydrogen atom, a monovalent hydrocarbon group which may have a substituent, or a monovalent heterocyclic group which may have a substituent, and S indicates a sulfur atom;
(b) R-O wherein R indicates a hydrogen atom, a monovalent hydrocarbon group which may have a substituent, or a monovalent heterocyclic group which may have a substituent, and O indicates an oxygen atom; or
(c) R_{A}-(R_{B}-)N wherein R_{A} and R_{B} each independently indicate a hydrogen atoms, a monovalent hydrocarbon group which may have a substituent, or a monovalent heterocyclic group which may have a substituent, and N indicates a nitrogen atom.

More preferably, the leaving group represented by X may be:
(a) R-S wherein R indicates a hydrogen atom, a monovalent hydrocarbon group which may have a substituent, or a monovalent heterocyclic group which may have a substituent, and S indicates a sulfur atom; or
(b) R-O wherein R indicates a hydrogen atom, a monovalent hydrocarbon group which may have a substituent, or a monovalent heterocyclic group which may have a substituent, and O indicates an oxygen atom.

Even more preferably, the leaving group represented by X may be:
(a) R-S wherein R indicates a monovalent hydrocarbon group which may have a substituent, or a monovalent heterocyclic group which may have a substituent, and S indicates a sulfur atom.

Particularly preferably, the leaving group represented by X may be:
(a) R-S wherein R indicates a monovalent hydrocarbon group (e.g., phenyl) which may have a substituent, and S indicates a sulfur atom.

Examples of the halogen atom include a fluorine atom, a chlorine atom, a bromine atom, and an iodine atom.

Examples of the monovalent hydrocarbon group include a monovalent chain hydrocarbon group, a monovalent alicyclic hydrocarbon group, and a monovalent aromatic hydrocarbon group.

The monovalent chain hydrocarbon group means a hydrocarbon group comprising only a chain structure and does not comprise any cyclic structure in a main chain thereof. Note that the chain structure may be linear or branched. Examples of the monovalent chain hydrocarbon group include alkyl, alkenyl, and alkynyl. The alkyl, alkenyl, and alkynyl may be linear or branched.

The alkyl is preferably C₁₋₁₂ alkyl, more preferably C₁₋₆ alkyl, and even more preferably C₁₋₄ alkyl. When the alkyl has a substituent, the number of carbon atoms does not comprise the number of carbon atoms of the substituent. Examples of the C₁₋₁₂ alkyl include methyl, ethyl, n-propyl, i-propyl, n-butyl, s-butyl, isobutyl, t-butyl, pentyl, hexyl, heptyl, octyl, nonyl, decyl, and dodecyl.

The alkenyl is preferably C₂₋₁₂ alkenyl, more preferably C₂₋₆ alkenyl, and even more preferably C₂₋₄ alkenyl. When the alkenyl has a substituent, the number of carbon atoms does not comprise the number of carbon atoms of the substituent. Examples of the C₂₋₁₂ alkenyl include vinyl, propenyl, and n-butenyl.

The alkynyl is preferably C₂₋₁₂ alkynyl, more preferably C₂₋₆ alkynyl, and even more preferably C₂₋₄ alkynyl. When the alkynyl has a substituent, the number of carbon atoms does not comprise the number of carbon atoms of the substituent. Examples of the C₂₋₁₂ alkynyl include ethynyl, propynyl, and n-butynyl.

The monovalent chain hydrocarbon group is preferably alkyl.

The monovalent alicyclic hydrocarbon group means a hydrocarbon group comprising only an alicyclic hydrocarbon as a cyclic structure and not comprising any aromatic ring, in which the alicyclic hydrocarbon may be monocyclic or polycyclic. Note that the monovalent alicyclic hydrocarbon group is not necessarily required to comprise only an alicyclic hydrocarbon but may comprise a chain structure in part thereof. Examples of the monovalent alicyclic hydrocarbon group include cycloalkyl, cycloalkenyl, and cycloalkynyl, which may be monocyclic or polycyclic.

The cycloalkyl is preferably C₃₋₁₂ cycloalkyl, more preferably C₃₋₆ cycloalkyl, and even more preferably C₅₋₆ cycloalkyl. When the cycloalkyl has a substituent, the number of carbon atoms does not comprise the number of carbon atoms of the substituent. Examples of the C₃₋₁₂ cycloalkyl include cyclopropyl, cyclobutyl, cyclopentyl, and cyclohexyl.

The cycloalkenyl is preferably C₃₋₁₂ cycloalkenyl, more preferably C₃₋₆ cycloalkenyl, and even more preferably C₅₋₆ cycloalkenyl. When the cycloalkenyl has a substituent, the number of carbon atoms does not comprise the number of carbon atoms of the substituent. Examples of the C₃₋₁₂ cycloalkenyl include cyclopropenyl, cyclobutenyl, cyclopentenyl, and cyclohexenyl.

The cycloalkynyl is preferably C₃₋₁₂ cycloalkynyl, more preferably C₃₋₆ cycloalkynyl, and even more preferably C₅₋₆ cycloalkynyl. When the cycloalkynyl has a substituent, the number of carbon atoms does not comprise the number of carbon atoms of the substituent. Examples of the C₃₋₁₂ cycloalkynyl include cyclopropynyl, cyclobutynyl, cyclopentynyl, and cyclohexynyl.

The monovalent alicyclic hydrocarbon group is preferably cycloalkyl.

The monovalent aromatic hydrocarbon group means a hydrocarbon group comprising an aromatic cyclic structure. Note that the monovalent aromatic hydrocarbon group is not necessarily required to comprise only an aromatic ring and may comprise a chain structure or alicyclic hydrocarbon in part thereof, in which the aromatic ring may be monocyclic or polycyclic. The monovalent aromatic hydrocarbon group is preferably C₆₋₁₂ aryl, more preferably C₆₋₁₀ aryl, and even more preferably C₆ aryl. When the monovalent aromatic hydrocarbon group has a substituent, the number of carbon atoms does not comprise the number of carbon atoms of the substituent. Examples of the C₆₋₁₂ aryl include phenyl and naphthyl.

The monovalent aromatic hydrocarbon group is preferably phenyl.

Among these groups, the monovalent hydrocarbon group is preferably alkyl, cycloalkyl, or aryl.

The monovalent heterocyclic group refers to a group obtained by removing one hydrogen atom from a heterocycle of a heterocyclic compound. The monovalent heterocyclic group is a monovalent aromatic heterocyclic group or a monovalent nonaromatic heterocyclic group. The monovalent heterocyclic group preferably comprises one or more selected from the group consisting of an oxygen atom, a sulfur atom, a nitrogen atom, a phosphorus atom, a boron atom, and a silicon atom and more preferably comprises one or more selected from the group consisting of an oxygen atom, a sulfur atom, and a nitrogen atom as a hetero atom comprised in the heterocyclic group.

The monovalent aromatic heterocyclic group is preferably a C₁₋₁₅ aromatic heterocyclic group, more preferably a C₁₋₉ aromatic heterocyclic group, and even more preferably a C₁₋₆ aromatic heterocyclic group. When the monovalent aromatic heterocyclic group has a substituent, the number of carbon atoms does not comprise the number of carbon atoms of the substituent. Examples of the monovalent aromatic heterocyclic group include pyrrolyl, furanyl, thiophenyl, pyridinyl, pyridazinyl, pyrimidinyl, pyrazinyl, triazinyl, pyrazolyl, imidazolyl, thiazolyl, isothiazolyl, oxazolyl, isoxazolyl, triazolyl, tetrazolyl, indolyl, purinyl, anthraquinolyl, carbazonyl, fluorenyl, quinolinyl, isoquinolinyl, quinazolinyl, and phthalazinyl.

The monovalent nonaromatic heterocyclic group is preferably a C₂₋₁₅ nonaromatic heterocyclic group, more preferably a C₂₋₉ nonaromatic heterocyclic group, and even more preferably a C₂₋₆ nonaromatic heterocyclic group. When the monovalent nonaromatic heterocyclic group has a substituent, the number of carbon atoms does not comprise the number of carbon atoms of the substituent. Examples of the monovalent nonaromatic heterocyclic group include oxiranyl, aziridinyl, azetidinyl, oxetanyl, thietanyl, pyrrolidinyl, dihydrofuranyl, tetrahydrofuranyl, dioxolanyl, tetrahydrothiophenyl, pyrolinyl, imidazolidinyl, oxazolidinyl, piperidinyl, dihydropyranyl, tetrahydropyranyl, tetrahydrothiopyranyl, morpholinyl, thiomorpholinyl, piperazinyl, dihydrooxazinyl, tetrahydrooxazinyl, dihydropyrimidinyl, and tetrahydropyrimidinyl.

Among these groups, the monovalent heterocyclic group is preferably a five-membered or six-membered heterocyclic group.

The number of "substituents" in "monovalent hydrocarbon group which may have a substituent" and "monovalent heterocyclic group which may have a substituent" represented by R, R_{A}, and R_{B} may be, for example, 1 to 5, preferably 1 to 3, and more preferably 1 or 2. Examples of the substituent include:
(i) a halogen atom;
(ii) a monovalent hydrocarbon group;
(iii) a monovalent heterocyclic group;
(iv) an aralkyl;
(v) Rₐ-O-, Rₐ-C(=O)-, Rₐ-O-C(=O)-, or Rₐ-C(=O)-O-, (where Rₐ represents a hydrogen atom or a monovalent hydrocarbon group);
(vi) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a monovalent hydrocarbon group); and
(vii) a nitro group, a sulfate group, a sulfonate group, a cyano group, and a carboxyl group.

Definitions, examples, and preferred examples of the halogen atom, the monovalent hydrocarbon group, and the monovalent heterocyclic group in the substituent are similar to those explained for the above R, R_{A}, and R_{B}.

The aralkyl refers to arylalkyl. Definitions, examples, and preferred examples of the aryl and the alkyl in the arylalkyl are as described above. The aralkyl is preferably C₃₋₁₅ aralkyl. Examples of such an aralkyl include benzoyl, phenethyl, naphthylmethyl, and naphthylethyl.

Preferably, the substituent may be:
(i) a halogen atom;
(ii) a C₁₋₁₂ alkyl, a C₁₋₁₂ phenyl, or a C₁₋₁₂ naphthyl;
(iii) a C₃₋₁₅ aralkyl;
(iv) a 5- or 6-membered heterocycle;
(v) Rₐ-O-, Rₐ-C(=O)-, Rₐ-O-C(=O)-, or Rₐ-C(=O)-O-, (where Rₐ represents a hydrogen atom or a C₁₋₁₂ alkyl);
(vi) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a C₁₋₁₂ alkyl); or
(vii) the same groups as listed in the above (vii).

More preferably, the substituent may be:
(i) a halogen atom;
(ii) a C₁₋₁₂ alkyl;
(iii) Rₐ-O-, Rₐ-C(=O)-, Rₐ-O-C(=O)-, or Rₐ-C(=O)-O-, (where Rₐ represents a hydrogen atom or a C₁₋₁₂ alkyl);
(iv) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a C₁₋₁₂ alkyl); or
(v) the same groups as listed in the above (vii).

Even more preferably, the substituent may be:
(i) a halogen atom;
(ii) a C₁₋₆ alkyl;
(iii) Rₐ-O-, Rₐ-C(=O)-, Rₐ-O-C(=O)-, or Rₐ-C(=O)-O-, (where Rₐ represents a hydrogen atom or a C₁₋₆ alkyl);
(iv) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a C₁₋₆ alkyl); or
(v) the same groups as listed in the above (vii).

Particularly preferably, the substituent may be:
(i) a halogen atom;
(ii) a C₁₋₄ alkyl;
(iii) Rₐ-O-, Rₐ-C(=O)-, Rₐ-O-C(=O)-, or Rₐ-C(=O)-O-, (where Rₐ represents a hydrogen atom or a C₁₋₄ alkyl);
(iv) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a C₁₋₄ alkyl); or
(v) the same groups as listed in the above (vii).

The affinity peptide indicated by Y has a binding region in a CH2 domain in an immunoglobulin unit containing two heavy chains and two light chains. As the affinity peptide, any peptide having a binding region in the CH2 domain in the immunoglobulin unit can be used. The affinity peptide may contain an amino acid residue containing a side chain containing a moiety capable of bonding with a carbonyl group (C=O) adjacent to Y (e.g., amino group, hydroxy group) and form an amide bond with a carbonyl group (C=O) adjacent to Y via an amino group in the side chain of the lysine residue. Preferably, the affinity peptide may contain a lysine residue and form an amide bond with a carbonyl group (C=O) adjacent to Y via an amino group in the side chain of the lysine residue. For such affinity peptides, various affinity peptides disclosed in WO2016/186206, WO2018/199337, WO2019/240288, WO2019/240287 and WO2020/090979 and literature cited in these international publications can be used.

Preferably, the affinity peptide may be:
(A) Affinity peptide comprising the amino acid sequence of CQRRFYEALHDPNLNEEQRNARIRSIKDDC (SEQ ID NO: 1);
(B) An affinity peptide comprising an amino acid sequence comprising a mutation of 1 to 5 amino acid residues which is selected from the group consisting of substitution, insertion, deletion and addition of amino acid residues in the amino acid sequence of CQRRFYEALHDPNLNEEQRNARIRSIKDDC (SEQ ID NO: 1),
wherein the lysine residue at the position 27 and the two cysteine residues at the positions 1 and 30 are maintained.

Since the affinity peptide containing the amino acid sequence of SEQ ID NO: 1 is a suitable peptide having a binding region in the CH2 domain in the immunoglobulin unit, it is preferable to use such an affinity peptide in the present invention.

The N-terminal and C-terminal amino acid residues of the affinity peptide may be protected. Further, the thiol groups of the side chains of the two cysteine residues (C) in the affinity peptide may be linked by a disulfide bond or via a linker.

Preferably, the affinity peptide (B) may be selected from the group consisting of:
(A) Affinity peptide comprising the amino acid sequence of FNMQCQRRFYEALHDPNLNEEQRNARIRSIKDDC (SEQ ID NO: 2);
(B) Affinity peptide comprising the amino acid sequence of FNMQCQRRFYEALHDPNLNEEQRNARIRSIKEDC (SEQ ID NO: 3);
(C) Affinity peptide comprising the amino acid sequence of FNMQCQRRFYEALHDPNLNEEQRNARIRSIKEEC (SEQ ID NO: 4);
(D) Affinity peptide comprising the amino acid sequence of NMQCQRRFYEALHDPNLNEEQRNARIRSIKEEC (SEQ ID NO: 5);
(E) Affinity peptide comprising the amino acid sequence of MQCQRRFYEALHDPNLNEEQRNARIRSIKEEC (SEQ ID NO: 6); and
(f) Affinity peptide comprising the amino acid sequence of QCQRRFYEALHDPNLNEEQRNARIRSIKEEC (SEQ ID NO: 7).

The N-terminal and C-terminal amino acid residues of the affinity peptide may be protected. Further, the thiol groups of the side chains of the two cysteine residues (C) in the affinity peptide may be linked by a disulfide bond or via a linker.

At least two separated cysteine residues in each amino acid sequence of the affinity peptide can form a cyclic peptide by a disulfide bond. Alternatively, in the above peptide, the thiol groups in the two cysteine residues may be linked by a carbonyl group-containing linker represented by the following.

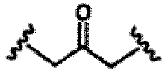

The broken line portion of the carbonyl group-containing linker represented above means the bonding portion with the thiol group. The linker is more stable to reduction reactions and the like than ordinary disulfide bonds. Such peptides can be prepared, for example, by the methods described in WO2016/186206.

The amino acids constituting the affinity peptide may be either L-form or D-form, and L-form is preferable (in the examples, all the amino acid residues constituting the peptide are L-form). The affinity peptide may be linked to the compound of formula (I) or a salt thereof by modifying a specific amino acid residue with a cross-linking agent. Examples of such a specific amino acid residue include a lysine residue, an aspartic acid residue, and a glutamic acid residue; preferred is a lysine residue. Examples of the cross-linking agent include cross-linking agents comprising preferably two or more succinimidyl groups such as disuccinimidyl glutarate (DSG) and disuccinimidyl suberate (DSS); cross-linking agents comprising preferably two or more imide acid portions such as dimethyl adipimidate·2HCl (DMA), dimethyl pimelimidate·2HCl (DMP), and dimethyl suberimidate·2HCl (DMS); and cross-linking agents having an SS bond such as dimethyl 3,3'-dithiobispropionimidate·2HCl (DTBP) and dithiobis(succinimidyl propionate) (DSP) (e.g., WO 2016/186206).

In the affinity peptide, a terminal amino group and a terminal carboxy group of the peptide may be protected. Examples of a protecting group for the N-terminal amino group include an alkylcarbonyl group (an acyl group) (e.g., an acetyl group, a propoxy group, and a butoxycarbonyl group such as a tert-butoxycarbonyl group), an alkyloxycarbonyl group (e.g., a fluorenylmethoxycarbonyl group), an aryloxycarbonyl group, and an arylalkyl(aralkyl)oxycarbonyl group (e.g., a benzyloxycarbonyl group). The protecting group for the N-terminal amino group is preferably an acetyl group. Examples of a protecting group for the C-terminal carboxy group include a group capable of forming an ester or an amide. Examples of the group capable of forming an ester or an amide include an alkyloxy group (e.g., methyloxy, ethyloxy, propyloxy, butyloxy, pentyloxy, and hexyloxy), an aryloxy group (e.g., phenyloxy and naphthyloxy), an aralkyloxy group (e.g., benzyloxy), and an amino group. The protecting group for the C-terminal carboxy group is preferably an amino group.

The first and second linkers represented by La and Lb, respectively, are divalent groups, as can be seen from the chemical structure of formula (I). The total number of atoms constituting the main chain in the first linker and the number of atoms constituting the main chain in the second linker are 5 to 7. By using the first linker and the second linker having such an atomic number, the lysine residue at the 288/290 position of the heavy chain in the immunoglobulin unit can be regioselectively modified with an affinity peptide-containing group, and the average ratio of the binding between the antibody and the affinity peptide-containing group can be easy to be highly controled in a desired range (1.5 to 2.5). In the present invention, the average ratio of the binding between the antibody and a predetermined group (e.g., affinity peptide-containing group) can be confirmed by analyzing the MS analysis data with DA calculator (Agilent's software).

In light of the fact that the total number of atoms constituting the main chain in the first linker and atoms constituting the main chain in the second linker are 5 to 7, the number of atoms constituting the main chain in the first linker is 1 to 6, and the number of atoms constituting the main chain in the second linker is 1 to 6. More specifically, the relationship between the number of atoms constituting the main chain in the first linker and the second linker is as follows.

**Table 1. Number of atoms constituting main chain**

| La | Lb |
|---|---|
| 1 | 4 - 6 |
| 2 | 3 - 5 |
| 3 | 2 - 4 |
| 4 | 1 - 3 |
| 5 | 1, or 2 |
| 6 | 1 |

The main chains in the first linker and the second linker are composed of a chain structure, a cyclic structure, or a structure containing a combination thereof. When the main chain portion has a chain structure comprising no cyclic structure, the number of carbon atoms of the main chain portion can be determined by counting the number of carbon atoms in the chain structure. On the other hand, when the main chain portion has a structure comprising a cyclic structure, the number of predetermined carbon atoms constituting the cyclic structure can be determined by counting the number of carbon atoms in the main chain portion. Specifically, the number of carbon atoms of the main chain portion in the cyclic structure can be determined by counting the number of carbon atoms of the shortest route linking two bonds in the cyclic structure (see, e.g., the following thick routes (a) to (d)). When the main chain has a structure comprising a combination of a chain structure and a cyclic structure, the number of atoms of the main chain can be determined by adding the number of atoms in the chain structure not comprising the cyclic structure to the number of atoms of the shortest route linking two bonds in the cyclic structure. 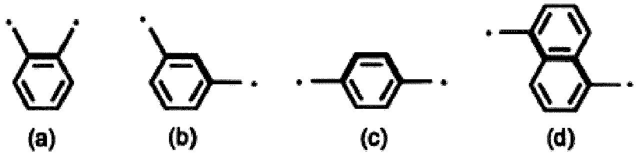 • is a bond.

In the case of (a), the shortest route is the thick route, and thus the number of atoms in the divalent cyclic structure counted as the number of carbon atoms of the main chain portion is two.

In the case of (b), the shortest route is the thick route, and thus the number of atoms in the divalent cyclic structure counted as the number of carbon atoms of the main chain portion is three.

In the case of (c), both routes are the shortest routes (equidistant), and thus the number of carbon atoms in the divalent cyclic structure counted as the number of carbon atoms in the main chain portion is four.

In the case of (d), a route of a condensation site is the shortest routes, and thus the number of carbon atoms in the divalent cyclic structure counted as the number of atoms in the main chain is four.

The main chains in the first linker and the second linker are set so that the number of atoms constituting the main chain as described above is 1 to 6. Therefore, the main chains in the first linker and the second linker are a divalent linear hydrocarbon group, a divalent cyclic hydrocarbon group, a divalent heterocyclic group, -C(=O)-, and -C(=S)-, -NR_{d}- (R_{d} indicates a hydrogen atom or a substituent), -O-, -S-, or a group consisting of a combination of two or more of these (e.g., 2 or 3).

The divalent linear hydrocarbon group is a linear alkylene, a linear alkenylene, or a linear alkynylene.

The linear alkylene is a C₁₋₆ linear alkylene, and is preferably a C₁₋₄ linear alkylene. Examples of the linear alkylene include methylene, ethylene, n-propylene, n-butylene, n-pentylene, and n-hexylene.

The linear alkenylene is a C₂₋₆ linear alkenylene, and is preferably a C₂₋₄ linear alkenylene. Examples of the linear alkenylene include ethylenylene, n-propynylene, n-butenylene, n-pentenylene, and n-hexenylene.

The linear alkynylene is a C₂₋₆ linear alkynylene, and is preferably a C₂₋₄ linear alkynylene. Examples of the linear alkynylene include ethynylene, n-propynylene, n-butynylene, n-pentynylene, and n-hexynylene.

The divalent linear hydrocarbon group is preferably a linear alkylene.

The divalent cyclic hydrocarbon group is an arylene or a divalent nonaromatic cyclic hydrocarbon group.

The arylene is preferably a C₆₋₁₄ arylene, more preferably a C₆₋₁₀ arylene, and particularly preferably a C₆ arylene. Examples of the arylene include phenylene, naphthylene, and anthracenylene.

The divalent nonaromatic cyclic hydrocarbon group is preferably a C₃₋₁₂ monocyclic or polycyclic divalent nonaromatic cyclic hydrocarbon group, more preferably a C₄₋₁₀ monocyclic or polycyclic divalent nonaromatic cyclic hydrocarbon group, and particularly preferably a C₅₋₈ monocyclic divalent nonaromatic cyclic hydrocarbon group. Examples of the divalent nonaromatic cyclic hydrocarbon group include cyclopropylene, cyclobutylene, cyclopentylene, cyclohexylene, cycloheptylene, and cyclooctylene.

The divalent cyclic hydrocarbon group is preferably an arylene.

The divalent heterocyclic group is a divalent aromatic heterocyclic group or a divalent nonaromatic heterocyclic group. The divalent heterocyclic group preferably comprises, as a hetero atom forming a heterocycle, one or more selected from the group consisting of an oxygen atom, a sulfur atom, a nitrogen atom, a phosphorous atom, a boron atom, and a silicon atom and more preferably comprises one or more selected from the group consisting of an oxygen atom, a sulfur atom, and a nitrogen atom.

The divalent aromatic heterocyclic group is preferably a C₃₋₁₅ divalent aromatic heterocyclic group, more preferably a C₃₋₉ divalent aromatic heterocyclic group, and particularly preferably a C₃₋₆ divalent aromatic heterocyclic group. Examples of the divalent aromatic heterocyclic group include pyrrolediyl, furandiyl, thiophenediyl, pyridinediyl, pyridazinediyl, pyrimidinediyl, pyrazinediyl, triazinediyl, pyrazolediyl, imidazolediyl, thiazolediyl, isothiazolediyl, oxazolediyl, isoxazolediyl, triazolediyl, tetrazolediyl, indolediyl, purinediyl, anthraquinonediyl, carbazolediyl, fluorenediyl, quinolinediyl, isoquinolinediyl, quinazolinediyl, and phthalazinediyl.

The divalent nonaromatic heterocyclic group is preferably a C₃₋₁₅ nonaromatic heterocyclic group, more preferably a C₃₋₉ nonaromatic heterocyclic group, and particularly preferably a C₃₋₆ nonaromatic heterocyclic group. Examples of the divalent nonaromatic heterocyclic group include pyrroldionediyl, pyrrolinedionediyl, oxiranediyl, aziridinediyl, azetidinediyl, oxetanediyl, thietanediyl, pyrrolidinediyl, dihydrofurandiyl, tetrahydrofurandiyl, dioxolanediyl, tetrahydrothiophenediyl, pyrrolinediyl, imidazolidinediyl, oxazolidinediyl, piperidinediyl, dihydropyrandiyl, tetrahydropyrandiyl, tetrahydrothiopyrandiyl, morpholinediyl, thiomorpholinediyl, piperazinediyl, dihydrooxazinediyl, tetrahydrooxazinediyl, dihydropyrimidinediyl, and tetrahydropyrimidinediyl.

The divalent heterocyclic group is preferably a divalent aromatic heterocyclic group.

W indicates an oxygen atom or a sulfur atom, preferably an oxygen atom.

La and Lb indicate a first linker and a second linker, respectively. The main chain in the first linker and the second linker may be composed of only carbon atoms, or may be composed of a combination of carbon atoms and heteroatoms (e.g., oxygen atom, nitrogen atom, sulfur atom). From the viewpoint of easiness of organic synthesis and improvement of stability, they may be composed of only carbon atoms. In certain cases, the main chains in the first and second linkers are divalent linear hydrocarbon groups, divalent cyclic hydrocarbon groups, divalent heterocyclic groups, -C(=O)-, -C(=S)- or a combination of two or more of these (e.g., 2 to 4, preferably 2 or 3).

In a specific embodiment, the main chain in the first linker represented by La is preferably set so that the number of atoms constituting the main chain as described above is 2 to 4. In this case, the main chain in the second linker represented by Lb is preferably set so that the number of atoms constituting the main chain as described above is 1 to 5.

In another specific embodiment, the main chain in the first linker represented by La is preferably set so that the number of atoms constituting the main chain as described above is two. In this case, the main chain in the second linker represented by Lb is preferably set so that the number of atoms constituting the main chain as described above is 3 to 5.

The main chain in the first linker represented by La is preferably composed of a divalent linear hydrocarbon group, a divalent cyclic hydrocarbon group, or a divalent heterocyclic group. Since La is a part contained in the thiol group-introduced antibody derivative and the conjugate of the antibody and the functional substance, it is preferable that the thiol group-introduced antibody derivative and the conjugate do not contain -C(=O)- and - C(=S)- which are relatively less stable (that is, more reactive) than the divalent linear hydrocarbon group, divalent cyclic hydrocarbon and divalent heterocyclic group. La is a portion contained in the thiol group-introduced antibody derivative and it is preferable that La has a low steric hindrance so as not to interfere with the reaction between the thiol group of the thiol group-introduced antibody derivative and the functional substance. In light of the above issue and the ease of organic synthesis, it is more preferable that the main chain in the first linker represented by La is composed of a divalent linear hydrocarbon group having 2 to 4 carbon atoms. The main chain in the first linker represented by La is even more preferably an ethylene group, a propylene group, or a butylene group, and particularly preferably an ethylene group.

On the other hand, since the main chain in the second linker indicated by Lb is contained in the antibody intermediate and since it is not contained in the thiol group-introduced antibody derivative produced from the antibody intermediate and the conjugate of the antibody and the functional substance, the stability of the main chain is unlikely to be a problem. Further, when a thiol group-introduced antibody derivative is produced from an antibody intermediate, the thiol group generated by cleaving between the thio group and the carbonyl group in the thiocarbonyl group (S-C=O) remains in the antibody but the second linker-containing structural unit represented by C(=W)-Lb-C(=O)-Y does not remain in the antibody. Therefore, this second linker can be decomposed in the production without any problem. That is, for the main chain in the second linker unlike the main chain in the first linker, its stability is less likely to be a problem. Therefore, the main chain in the second linker can be preferably composed of a divalent linear hydrocarbon group, a divalent cyclic hydrocarbon group, a divalent heterocyclic group, -C(=O)-, -C(=S)-, -NR_{d}- (R_{d} indicates a hydrogen atom or a substituent), -O-, -S-, or a combination of two or more of these (e.g., 2 or 3). The number of atoms constituting the main chain in the second linker is preferably 1 to 5, and more preferably 3 to 5.

Preferably, the main chain in the second linker may be composed of a divalent linear hydrocarbon group, a divalent cyclic hydrocarbon group, -C(=O)-, -C(=S)-, -NR_{d}-(R_{d} indicates a hydrogen atom or a substituent), -O-, -S-, or a combination of two or more of these (e.g., 2 or 3) in light of the ease of synthesis and the like. The number of atoms constituting the main chain in the second linker is preferably 1 to 5, and more preferably 3 to 5.

More preferably, the main chain in the second linker may be composed of a divalent linear hydrocarbon group, a divalent cyclic hydrocarbon group, -C(=O)-, - C(=S)-, -O-, -S-, or a combination of two or more of these (e.g., 2 or 3). The number of atoms constituting the main chain in the second linker is preferably 1 to 5, and more preferably 3 to 5.

Eaven more preferably, the main chain in the second linker may be composed of a linear alkylene, arylene, -C(=O)-, -C(=S)-, -O-, -S-, or a combination of two or more of these (e.g., 2 or 3). The number of atoms constituting the main chain in the second linker is preferably 1 to 5, and more preferably 3 to 5.

Particularly preferably, the main chain in the second linker may be composed of propylene or m-phenylene.

The groups constituting the main chain in the first linker and the second linker may have, for example, 1 to 5, preferably 1 to 3, and more preferably 1 or 2 substituents. Further, R_{d}, which is one of the groups constituting the main chain in the first linker and the second linker, may indicate a substituent as described above. Examples of such substituents include:
(i') a halogen atom;
(ii') a monovalent hydrocarbon group;
(iii') an aralkyl;
(iv') a monovalent heterocycle;
(v') Rₑ-O-, Rₑ-C(=O)-, Rₑ-O-C(=O)-, or Rₑ-C(=O)-O-, (where Rₑ represents a hydrogen atom or a monovalent hydrocarbon group);
(vi') NR_{f}R_{g}-, NR_{f}R_{g}-C(=O)-, NR_{f}R_{g}-C(=O)-O-, or R_{f}-C(=O)-NR_{g}-, (where R_{f} and R_{g} are the same as or different from each other, and each represent a hydrogen atom or a monovalent hydrocarbon group); or
(vii') Nitro group, sulfate group, sulfonic acid group, cyano group, and carboxyl group.

Definitions, examples, and preferred examples of the halogen atoms, monovalent hydrocarbon groups, aralkyl, and monovalent heterocyclic groups in the substituents are similar to that of the halogen atom, monovalent hydrocarbon group, aralkyl, and monovalent heterocyclic group described for R, R_{A}, and R_{B} and (i) to (iv), respectively.

Preferably, the substituents may be:
(i') a halogen atom;
(ii') a C₁₋₁₂ alkyl, a phenyl, or a naphthyl;
(iii') a C₃₋₁₅ aralkyl;
(iv') a 5- or 6-membered heterocycle;
(v') Rₑ-O-, Rₑ-C(=O)-, Rₑ-O-C(=O)-, or Rₑ-C(=O)-O-, (where Rₑ represents a hydrogen atom or a C₁₋₁₂ alkyl);
(vi') NR_{f}R_{g}-, NR_{f}R_{g}-C(=O)-, NR_{f}R_{g}-C(=O)-O-, or R_{f}-C(=O)-NR_{g}- (where R_{f} and R_{g} are the same as or different from each other, and each represent a hydrogen atom or a C₁₋₁₂ alkyl); or
(vii') the same groups as listed in the above (vii').

More preferably, the substituent may be:
(i') a halogen atom;
(ii') a C₁₋₁₂ alkyl;
(iii') Rₑ-O-, Rₐ-C(=O)-, Rₐ-O-C(=O)-, or Rₐ-C(=O)-O-, (where Rₐ represents a hydrogen atom or a C₁₋₁₂ alkyl);
(iv') NR_{f}R_{g}-, NR_{f}R_{g}-C(=O)-, NR_{f}R_{g}-C(=O)-O-, or R_{f}-C(=O)-NR_{g}- (where R_{f} and R_{g} are the same as or different from each other, and each represent a hydrogen atom or a C₁₋₁₂ alkyl); or
(v') the same groups as listed in the above (vii').

Even more preferably, the substituent may be:
(i') a halogen atom;
(ii') a C₁₋₆ alkyl;
(iii') Rₑ-O-, Rₑ-C(=O)-, Rₑ-O-C(=O)-, or Rₑ-C(=O)-O-, (where Rₑ represents a hydrogen atom or a C₁₋₆ alkyl);
(iv') NR_{f}R_{g}-, NR_{f}R_{g}-C(=O)-, NR_{f}R_{g}-C(=O)-O-, or R_{f}-C(=O)-NR_{g}-, (where R_{f} and R_{g} are the same as or different from each other, and each represent a hydrogen atom or a C₁₋₆ alkyl); or
(v') the same groups as listed in the above (vii').

Particularly preferably, the substituent may be:
(i') a halogen atom;
(ii') a C₁₋₄ alkyl;
(iii') Rₑ-O-, Rₑ-C(=O)-, Rₑ-O-C(=O)-, or Rₑ-C(=O)-O-, (where Rₑ represents a hydrogen atom or a C₁₋₄ alkyl);
(iv') NR_{f}R_{g}-, NR_{f}R_{g}-C(=O)-, NR_{f}R_{g}-C(=O)-O-, or R_{f}-C(=O)-NR_{g}-, (where R_{f} and R_{g} are the same as or different from each other, and each represent a hydrogen atom or a C₁₋₄ alkyl); or
(v') the same groups as listed in the above (vii').

Preferably, the compound represented by the above formula (I) may be represented by the following formula (I'): 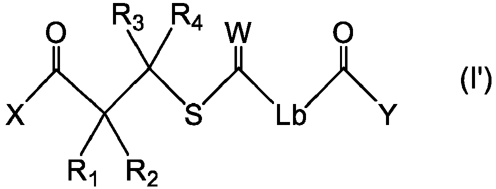 wherein
X, Y, O, S and W are the same as those in the above formula (I),
Lb indicates a second linker having 3 to 5 atoms constituting a main chain, and
R₁, R₂, R₃ and R₄ each independently indicate a hydrogen atom or a substituent.

Definitions, examples and preferred examples of substituents represented by R₁, R₂, R₃ and R₄ in formula (I') are similar to that of the above-mentioned substituent which may be possessed by the groups constituting the main chain in the first linker.

More preferably, the compound represented by the above formula (I') may be represented by the following formula (I''): 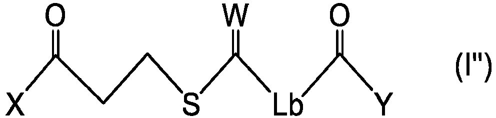 wherein
X, Y, O, S and W are the same as those in the above formula (I), and
Lb is the same as that of the above formula (I').

The compound or salt thereof of the present invention represented by the formulae (I) to (I") can be obtained by, for example, synthesizing a compound or salt thereof in which the portion Y is substituted with a leaving group (a leaving group having a higher leaving ability than X) in the compound or salt thereof represented by formulas (I) to (I"), and then reacting the synthesized compound or salt thereof with an affinity peptide. For example, such a reaction can be carried out in a suitable reaction system (e.g., an organic solvent or aqueous solution or a mixed solvent thereof) at a suitable temperature (e.g., about 15 to 200°C). The reaction system may include a suitable catalyst. The reaction time is, for example, 1 minute to 20 hours, preferably 10 minutes to 15 hours, more preferably 20 minutes to 10 hours, and even more preferably 30 minutes to 8 hours.

Preferably, the compound or salt thereof in which the portion Y is substituted with a leaving group (a leaving group having a higher leaving ability than X) may be a compound or salt thereof represented by the following formula (VI): 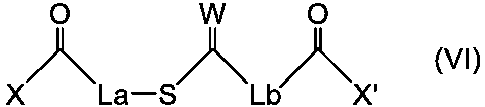 wherein
X indicates a leaving group,
X' indicates a leaving group having a leaving ability which is higher than that of the leaving group X.
O indicates an oxygen atom,
S indicates a sulfur atom,
W indicates an oxygen atom or a sulfur atom,
La indicates a first linker,
Lb indicates a second linker, and
the total number of atoms constituting a main chain in the first linker and the number of atoms constituting a main chain in the second linker are 5 to 7.

The leaving group represented by X', which has a higher leaving ability than the leaving group X, is not particularly limited as long as it is a leaving group having a higher leaving ability than the leaving group X, and examples of the leaving group include a pentafluorophenyloxy group, a tetrafluorophenyloxy group, a paranitrophenyloxy group, and an N-succinimidyloxy group.

The definitions, examples, and preferred examples of symbols, terms and expressions such as X (leaving group), W (oxygen atom or sulfur atom), La (first linker), Lb (second linker) in formula (VI) are the same as those of the above formula.

The compound or salt thereof represented by formula (VI) is useful as, for example, a synthetic intermediate for efficiently producing the compound ofr salt thereof represented by the formula (I).

More preferably, the compound represented by formula (VI) may be a compound represented by the following formula (VI'): 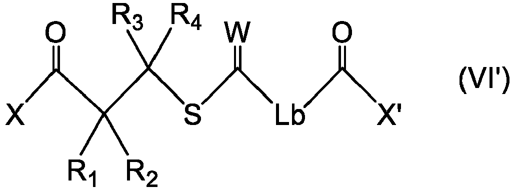 wherein
X, X', O, S and W are the same as those in the above formula (VI),
Lb indicates a second linker having 3 to 5 atoms constituting a main chain, and
R₁, R₂, R₃ and R₄ each independently indicate a hydrogen atom or a substituent.

The definitions, examples and preferred examples of substituents represented by R₁, R₂, R₃ and R₄ in formula (VI') may be the same as those of the above-mentioned substituent which may be possessed by the group constituting the main chain in the first linker.

Even more preferably, the compound represented by the formula (VI') may be a compound represented by the following formula (VI"): 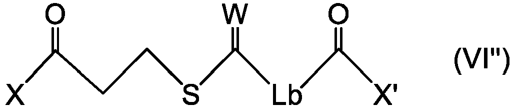 wherein
X, X', O, S and W are the same as those in the above formula (VI), and
Lb is the same as that of the above formula (VI').

Particularly preferably, the compound represented by the formula (VI") may be a compound represented by the following formula (VI"-1) or (VI"-2): 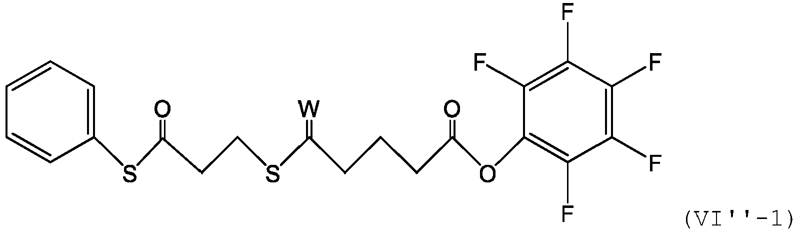 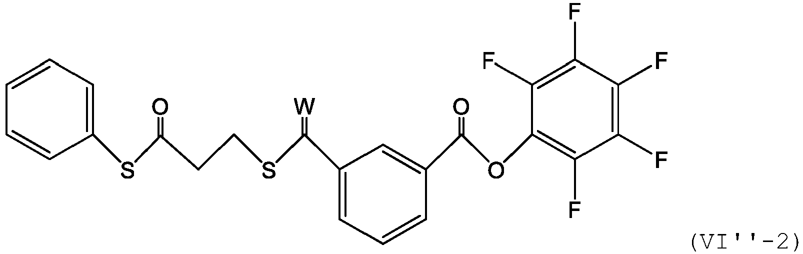 or wherein
O, S and W are the same as those of the above formula (VI), and
F is a fluorine atom.

The compound or a salt thereof represented by the formula (VI) can be produced from the compound or salt thereof represented by the following formula (V) : 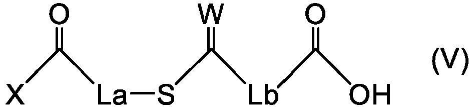 wherein
X indicates a leaving group,
O indicates an oxygen atom,
OH indicates a hydroxy group,
S indicates a sulfur atom,
W indicates an oxygen atom or a sulfur atom,
La indicates a first linker,
Lb indicates a second linker,
the total number of atoms constituting a main chain in the first linker and atoms constituting a main chain in the second linker are 5 to 7.

The definitions, examples, and preferred examples of symbols, terms and expressions such as X (leaving group), W (oxygen atom or sulfur atom), La (first linker), Lb (second linker) in formula (V) are the same as those of the above formula.

The compound or salt thereof represented by formula (VI) can be obtained by reacting the compound or salt thereof represented by the formula (V) with a carboxyl group modifying reagent. Examples of the carboxyl group modifying reagent include a pentafluorophenylation reagent (e.g., pentafluorophenyl trifluoroacetate), a tetrafluorophenylation reagent (e.g., trifluoroacetate tetrafluorophenyl), and a paranitrophenylation reagent (e.g., trifluoroacetate paranitrophenyl), N-succinimidylation reagent (e.g., trifluoroacetate N-succinimidyl). For example, such a reaction may be carried out at a suitable temperature (e.g., about -10 to 30°C) in a suitable organic solvent system (e.g., an organic solvent containing an alkyl halide (e.g., methyl halide) such as CH₂Cl₂ and an amine such as triethylamine). The reaction time is, for example, 1 minute to 20 hours, preferably 10 minutes to 15 hours, more preferably 20 minutes to 10 hours, and even more preferably 30 minutes to 8 hours.

The compound or salt thereof represented by formula (V) is useful as, for example, a synthetic intermediate for efficiently producing the compound or salt thereof represented by the formula (VI).

More preferably, the compound or salt thereof represented by formula (V) may be a compound represented by the following formula (V') : 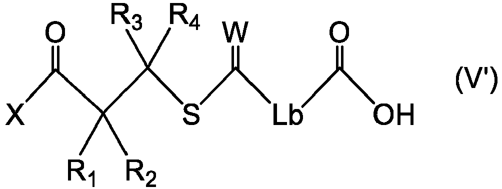 wherein
X, O, OH, S and W are the same as those in the above formula (V),
Lb indicates a second linker having 3 to 5 atoms constituting a main chain, and
R₁, R₂, R₃ and R₄ each independently indicate a hydrogen atom or a substituent.

In formula (V'), the definitions, examples and preferred examples of substituents represented by R₁, R₂, R₃ and R₄ is the same as those of the above-mentioned substituent which may be possessed by the group constituting the main chain in the first linker.

More preferably, the compound represented by the formula (V') may be a compound represented by the following formula (V"): 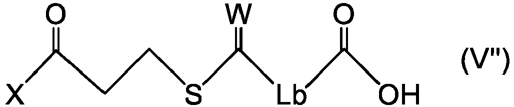 wherein
X, O, OH, S and W are the same as those in the above formula (V), and
Lb is the same as that of the above formula (V').

Particularly preferably, the compound represented by formula (V") may be a compound represented by the following formula (V"-1) or (V"-2) : 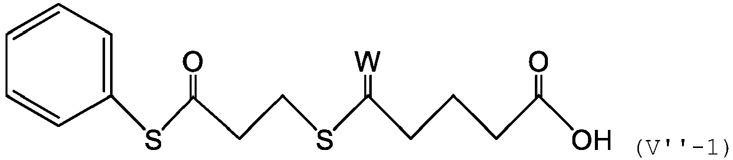 or 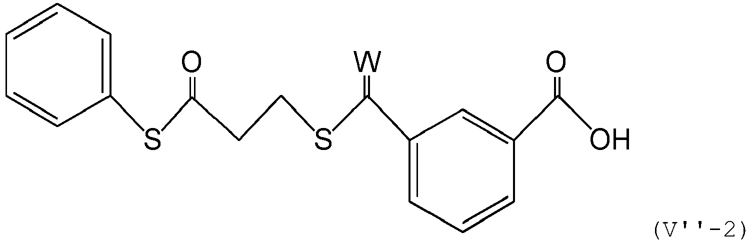 wherein
O, OH, S and W are the same as those in the above formula (V).

The compound represented by formula (V) can be obtained by reacting a compound represented by X-C(=O)-LaSH with a dicarboxylic acid compound represented by HO-C(=W)-Lb-C(=O)-OH or a cyclic compound produced by an intramolecular condensation reaction of the dicarboxylic acid compound (see Examples (1-3) and (2-1)). For example, such a reaction can be carried out in a suitable organic solvent system at a suitable temperature (eg, about 4-60°C). The reaction time is, for example, 1 minute to 20 hours, preferably 10 minutes to 15 hours, more preferably 20 minutes to 10 hours, and even more preferably 30 minutes to 8 hours.

The determination of the formation of the above compounds or salts thereof, which depends on its specific raw materials and the molecular weight of a product, can be performed by electrophoresis, chromatography (e.g., gel permutation chromatography, ion-exchange chromatography, reversed phase column chromatography, and HPLC), or mass spectrometry. The above compounds or salts thereof can be purified as appropriate by any method such as chromatography (e.g., the pieces of chromatography described above and affinity chromatography).

### 3. Antibody Intermediate or salt thereof

The present invention provides an antibody intermediate or salt thereof containing a structural unit represented by the following formula (II): 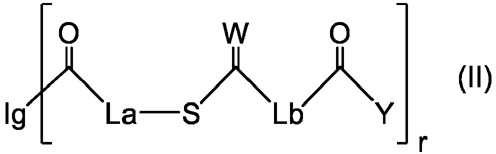 wherein
Ig indicates an immunoglobulin unit containing two heavy chains and two light chains, and forms an amide bond with the carbonyl group adjacent to Ig via an amino group in a side chain of a lysine residue present at the position 288/290 in the two heavy chains according to EU numbering,
Y indicates an affinity peptide having a binding region to a CH2 domain in the immunoglobulin unit,
O indicates an oxygen atom,
S indicates a sulfur atom,
W indicates an oxygen atom or a sulfur atom,
La indicates a first linker,
Lb indicates a second linker,
the total number of atoms constituting a main chain in the first linker and atoms constituting a main chain in the second linker are 5 to 7, and
the average ratio r of the amide bond per the two heavy chains is 1.5 to 2.5.

The immunoglobulin unit indicated by Ig is as described above. The definitions, examples, and preferred examples of symbols, terms and expressions such as Y (affinity peptide), W (oxygen atom or sulfur atom), La (first linker) and Lb (second linker) in formula (II) are the same as those of the above formula.

In the formula (II), the average ratio (r) of the amide bonds per two heavy chains is the average ratio of the bonds between the immunoglobulin unit and the affinity peptide-containing group (number of affinity peptide-containing group/immunoglobulin unit). Such an average ratio is 1.5 to 2.5. Such an average ratio may be preferably 1.6 or more, more preferably 1.7 or more, even more preferably 1.8 or more, and particularly preferably 1.9 or more. Such an average ratio may also be preferably 2.4 or less, more preferably 2.3 or less, even more preferably 2.2 or less, and particularly preferably 2.1 or less. More specifically, such an average ratio is preferably 1.6 to 2.4, more preferably 1.7 to 2.3, even more preferably 1.8 to 2.2, and particularly preferably 1.9 to 2.1.

Preferably, the structural unit represented by the formula (II) may be the structural unit represented by the following formula (II') : 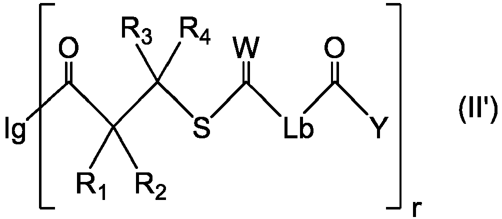 wherein
Ig, Y, O, S, W and r are the same as those of the above formula (II),
Lb indicates a second linker having 3 to 5 atoms constituting a main chain, and
R₁, R₂, R₃ and R₄ each independently indicate a hydrogen atom or a substituent.

The definitions, examples and preferred examples of substituents represented by R₁, R₂, R₃ and R₄ in formula (II') is the same as those of the above-mentioned substituent which may be possessed by the group consituting the main chain in the first linker.

More preferably, the structural unit represented by formula (II') may be a structural unit represented by the following formula (II"): 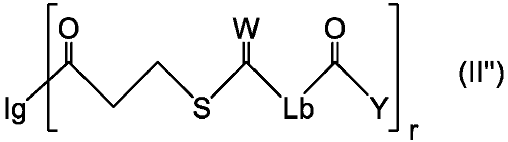 wherein
Ig, Y, O, S, W and r are the same as those of the above formula (II), and
Lb is the same as that of the above formula (II').

The antibody intermediate or salt thereof of the present invention can be obtained by reacting the compound or salt thereof of the present invention with an antibody containing the above-mentioned immunoglobulin unit. In the reaction, first, the compound or salt thereof of the present invention is mixed with the antibody. This allows the compounds or salt thereof of the present invention to associate with the antibody via an affinity peptide that has an affinity for the antibody. Next, after antibody association, a carbonyl group (X-C=O) with a leaving group (X) can be regioselectively reacted with the amino group in the side chain of the lysine residue present at the position 288/290 of the antibody. By such a reaction, the amino group and the carbon atom of the carbonyl group are bonded, and the leaving group (X) is eliminated from the carbonyl group to obtain the antibody intermediate or salt thereof of the present invention. The molar ratio of the compound or salt thereof of the present invention to the antibody (the compound of the present invention or salt thereof/antibody) to the antibody in the reaction is not particularly limited, since it varies depending on factors such as the type of the compound of the present invention or salt thereof and antibody. It is, for example, 1 to 100, preferably 2 to 80, more preferably 4 to 60, even more preferably 5 to 50, and particularly preferably 6 to 30.

Such a reaction can be appropriately carried out under a condition that cannot cause protein denaturation/degradation (e.g., cleavage of amide bond) (mild conditions). For example, such a reaction can be carried out at room temperature (e.g., about 15-30°C) in a suitable reaction system such as buffer. The pH of the buffer is, for example, 5 to 9, preferably 5.5 to 8.5, and more preferably 6.0 to 8.0. The buffer may contain a suitable catalyst. The reaction time is, for example, 1 minute to 20 hours, preferably 10 minutes to 15 hours, more preferably 20 minutes to 10 hours, and even more preferably 30 minutes to 8 hours. For details of such a reaction, see, e.g., G.I.J.L. Bernardes et al., Chem. Rev., 115, 2174(2015); J.L. Bernardes et al., Chem. Asian. J., 4, 630 (2009); B. G. Devices et al., Nat. Commun., 5, 4740(2014); Wagner et al., Bioconjugate. Chem., 25, 825(2014).

The determination of the formation of the antibody intermediate or salt thereof, which depends on its specific raw materials and the molecular weight of a product, can be performed by electrophoresis, chromatography (e.g., gel permutation chromatography, ion-exchange chromatography, reversed phase column chromatography, and high-performance liquid chromatography (HPLC)), or mass spectrometry, for example, and preferably mass spectrometry. Determination of regioselectivity can be performed by peptide mapping, for example. Peptide mapping can be performed by protease (e.g., trypsin and chymotrypsin) treatment and mass spectrometry, for example. For the protease, an endoprotease is preferred. Examples of such an endoprotease include trypsin, chymotrypsin, Glu-C, Lys-N, Lys-C, and Asp-N. Determination of the number of the introduced affinity peptide can be performed by electrophoresis, chromatography, or mass spectrometry, for example, and preferably mass spectrometry. The antibody intermediate or salt thereof can be purified as appropriate by any method such as chromatography (e.g., chromatography described above and affinity chromatography).

### 4. A thiol group-introduced antibody derivative or a salt thereof

The present invention provides a thiol group-introduced antibody derivative or salt thereof, which comprises a structural unit represented by the following formula (III): 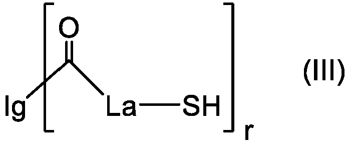 wherein
Ig indicates an immunoglobulin unit containing two heavy chains and two light chains, and forms an amide bond with the carbonyl group adjacent to Ig via an amino group in a side chain of a lysine residue present at the position 288/290 in the two heavy chains according to EU numbering,
O indicates an oxygen atom,
SH indicates a thiol group,
La indicates a first linker,
the number of atoms constituting the main chain in the first linker is 2 to 4, and
the average ratio r of the amide bonds per the two heavy chains is 1.5 to 2.5.

The immunoglobulin unit indicated by Ig is as described above. The definitions, examples, and preferred examples of symbols, terms and expressions such as La (first linker) in formula (III) are the same as those in the above formula.

In the formula (III), the average ratio (r) of the amide bond per two heavy chains indicates the average ratio of the bonds between the immunoglobulin unit and the thiol-containing group (number of thiol-containing groups/immunoglobulin unit). Such an average ratio is 1.5 to 2.5. Such an average ratio may be preferably 1.6 or more, more preferably 1.7 or more, even more preferably 1.8 or more, and particularly preferably 1.9 or more. Such an average ratio may also be preferably 2.4 or less, more preferably 2.3 or less, even more preferably 2.2 or less, and particularly preferably 2.1 or less. More specifically, such an average ratio is preferably 1.6 to 2.4, more preferably 1.7 to 2.3, even more preferably 1.8 to 2.2, and particularly preferably 1.9 to 2.1.

Preferably, the structural unit represented by formula (III) may be the structural unit represented by the following formula (III'): 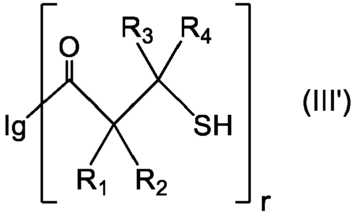 wherein
Ig, O, SH and r are the same as those in the above formula (III), and
R₁, R₂, R₃ and R₄ each independently indicate a hydrogen atom or a substituent.

The definitions, examples and preferred examples of substituents represented by R₁, R₂, R₃ and R₄ in formula (III') is the same as those of the above-mentioned substituent which may be possessed by the group constituting the main chain in the first linker.

More preferably, the structural unit represented by formula (III') may be the structural unit represented by the following formula (III"): 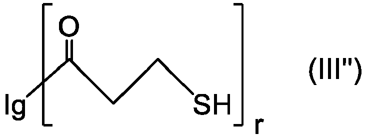 wherein
Ig, O, SH and r are the same as those in the above formula (III).

The thiol group-introduced antibody derivative or salt thereof of the present invention can be obtained by subjecting the antibody intermediate or salt thereof of the present invention to a thioester cleavage reaction. The thioester cleavage reaction can be carried out under conditions that cannot cause denaturation/degradation of proteins (immunoglobulin/antibody) (e.g., cleavage of amide bonds) (mild conditions as described above). More specifically, it can be cleaved by stirring for 1 hour in a hydroxylamine hydrochloride solution in the range of pH 4.0 to pH 8.0, 10 mM to 10 M (e.g., Vance, Net al., Bioconjugate Chem. 2019, 30, 148-160.).

The determination of the formation of the thiol group-introduced antibody derivative or salt thereof, which depends on its specific raw materials and the molecular weight of a product, can be performed by electrophoresis, chromatography (e.g., gel permutation chromatography, ion-exchange chromatography, reversed phase column chromatography, and high-performance liquid chromatography (HPLC)), or mass spectrometry, for example, and preferably mass spectrometry. Determination of regioselectivity can be performed by peptide mapping, for example. Peptide mapping can be performed by protease (e.g., trypsin and chymotrypsin) treatment and mass spectrometry, for example. For the protease, an endoprotease is preferred. Examples of such an endoprotease include trypsin, chymotrypsin, Glu-C, Lys-N, Lys-C, and Asp-N. Determination of the number of the introduced thiol group can be performed by electrophoresis, chromatography, or mass spectrometry, for example, and preferably mass spectrometry. The thiol group-introduced antibody derivative or salt thereof can be purified as appropriate by any method such as chromatography (e.g., chromatography described above and affinity chromatography).

### 5. Conjugates of antibody and functional substances or salt thereof

The present invention provides a conjugate of an antibody and a functional substances or a salt thereof, which comprises a structural unit represented by the following formula (IV): 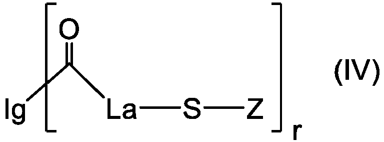 wherein
Ig indicates an immunoglobulin unit containing two heavy chains and two light chains, and forms an amide bond with the carbonyl group adjacent to Ig via an amino group in a side chain of a lysine residue present at the position 288/290 in the two heavy chains according to EU numbering,
O indicates an oxygen atom,
S indicates a sulfur atom,
Z indicates a functional substance,
La indicates a first linker,
the number of atoms constituting a main chain in the first linker is 2 to 4, and
the average ratio r of the amide bonds per the two heavy chains is 1.5 to 2.5.

The immunoglobulin unit indicated by Ig is as described above. The definitions, examples, and preferred examples of symbols, terms and expressions such as La (first linker) in formula (IV) are similar to those in formula above.

The functional substance is not limited to a particular substance as long as it is a substance imparting any function to the antibody; examples thereof include drugs, labelling substances, and stabilizers; preferred are drugs and labelling substances. The functional substance may be a single functional substance or a substance in which two or more functional substances are linked with each other.

The drug may be a drug to any disease. Examples of such a disease include cancer (e.g., lung cancer, stomach cancer, colon cancer, pancreatic cancer, renal cancer, liver cancer, thyroid cancer, prostatic cancer, bladder cancer, ovarian cancer, uterine cancer, bone cancer, skin cancer, a brain tumor, and melanoma), autoimmune diseases and inflammatory diseases (e.g., allergic diseases, articular rheumatism, and systemic lupus erythematosus), brain or nerve diseases (e.g., cerebral infarction, Alzheimer's disease, Parkinson disease, and amyotrophic lateral sclerosis), infectious diseases (e.g., microbial infectious diseases and viral infectious diseases), hereditary rare diseases (e.g., hereditary spherocytosis and nondystrophic myotonia), eye diseases (e.g., age-related macular degeneration, diabetic retinopathy, and retinitis pigmentosa), diseases in the bone and orthopedic field (e.g., osteoarthritis), blood diseases (e.g., leukosis and purpura), and other diseases (e.g., diabetes, metabolic diseases such as hyperlipidemia, liver diseases, renal diseases, lung diseases, circulatory system diseases, and digestive system diseases). The drug may be a prophylactic or therapeutic agent for a disease, or a relief agent for side effects.

More specifically, the drug may be an anti-cancer agent. Examples of the anti-cancer agent include chemotherapeutic agents, toxins, and radioisotopes or substances comprising them. Examples of chemotherapeutic agents include DNA injuring agents, antimetabolites, enzyme inhibitors, DNA intercalating agents, DNA cleaving agents, topoisomerase inhibitors, DNA binding inhibitors, tubulin binding inhibitors, cytotoxic nucleosides, and platinum compounds. Examples of toxins include bacteriotoxins (e.g., diphtheria toxin) and phytotoxins (e.g., ricin). Examples of radioisotopes include radioisotopes of a hydrogen atom (e.g., ³H), radioisotopes of a carbon atom (e.g., ¹⁴C), radioisotopes of a phosphorous atom (e.g., ³²P), radioisotopes of a sulfur atom (e.g., 35^{S}), radioisotopes of yttrium (e.g., ⁹⁰Y), radioisotopes of technetium (e.g., ^{99m}Tc), radioisotopes of indium (e.g., ¹¹¹In), radioisotopes of an iodide atom (e.g., ¹²³I, ¹²⁵I, ¹²⁹I, and ¹³¹I), radioisotopes of samarium (e.g., ¹⁵³Sm), radioisotopes of rhenium (e.g., ¹⁸⁶Re), radioisotopes of astatine (e.g., ²¹¹At), and radioisotopes of bismuth (e.g., ²¹²Bi). More specific examples of the drug include auristatin (MMAE, MMAF), maytansine (DM1, DM4), PBD (pyrrolobenzodiazepine), IGN, camptothecin analogs, calicheamicin, duocarmycin, eribulin, anthracycline, dmDNA31, and tubricin.

The labelling substance is a substance that makes detection of a target (e.g., a tissue, a cell, or a substance) possible. Examples of the labelling substance include enzymes (e.g., peroxidase, alkaline phosphatase, luciferase, and β-galactosidase), affinity substances (e.g., streptavidin, biotin, digoxigenin, and aptamer), fluorescent substances (e.g., fluorescein, fluorescein isothiocyanate, rhodamine, green-fluorescent protein, and red-fluorescent protein), luminescent substances (e.g., luciferin, aequorin, acridinium ester, tris(2,2'-bipyridyl) Duthenium, and luminol), and radioisotopes (e.g., those described above) or substances comprising them.

The stabilizer is a substance that makes stabilization of an antibody possible. Examples of the stabilizer include diols, glycerin, nonionic surfactants, anionic surfactants, natural surfactants, saccharides, and polyols.

The functional substance may also be a peptide, a protein, a nucleic acid, a low molecular weight organic compound, a sugar chain, a lipid, a high molecular polymer, a metal (e.g., gold), or a chelator. Examples of the peptide include a cell membrane permeable peptide, a bloodbrain barrier permeable peptide, and a peptide medicament. Examples of the protein include enzymes, cytokines, fragment antibodies, lectins, interferons, serum albumin, and antibodies. Examples of the nucleic acid include DNA, RNA, and artificial nucleic acid. Examples of the nucleic acid also include RNA interference inducible nucleic acids (e.g., siRNA), aptamers, and antisense. Examples of the low molecular weight organic compound include proteolysis targeting chimeras, dyes, and photodegradable compounds.

When the functional sutstance does not have a functional group that is likely to react with a thiol group, the functional sutstance may be derivatized to have such a functional group. The derivatization is common technical knowledge in the art (e.g., WO2004/010957, US2006/0074008, US2005/02386649). For example, the derivatization may be carried out using any cross-linking agent. Alternatively, the derivatization may be carried out using a specific linker having a desired functional group. For example, such a linker may be capable of separating a functional substance and an antibody in a suitable environment (e.g., intracellular or extracellular) by cleavage of the linker. Such linkers include, for example, peptidyl linkers that are degraded by specific proteases [e.g., intracellular proteases (e.g., proteases present in lysosomes, or endosomes), extracellular proteases (e.g., secretory proteases)] (e.g., USP6,214,345; Dubowchik et al., Pharma. Therapeutics 83: 67-123 (1999)), a linker that can be cleaved at a locally acidic site present in the living body (e.g., USP5,622,929, 5,122,368; 5,824,805). The linker may be self-immolative (e.g., WO02/083180, WO04/043493, WO05/1192919). In the present invention, the derivatized functional substance is also simply referred to as "functional substance".

In the formula (IV), the average ratio (r) of the amide bond per two heavy chains is the average ratio of the bonds between the immunoglobulin unit and the functional substance-containing group (number of functional substance-containing groups/immunoglobulin unit). Such an average ratio is 1.5 to 2.5. Such an average ratio may be preferably 1.6 or more, more preferably 1.7 or more, even more preferably 1.8 or more, and particularly preferably 1.9 or more. Such an average ratio may also be preferably 2.4 or less, more preferably 2.3 or less, even more preferably 2.2 or less, and particularly preferably 2.1 or less. More specifically, such an average ratio is preferably 1.6 to 2.4, more preferably 1.7 to 2.3, even more preferably 1.8 to 2.2, and particularly preferably 1.9 to 2.1.

Preferably, the structural unit represented by formula (IV) may be the structural unit represented by the following formula (IV'): 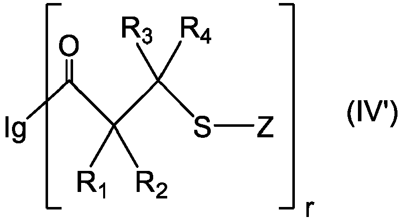 wherein
Ig, O, S, Z and r are the same as those of the above formula (IV), and
R₁, R₂, R₃ and R₄ each independently indicate a hydrogen atom or a substituent.

The definitions, examples and preferred examples of substituents represented by R₁, R₂, R₃ and R₄ in formula (IV') is the same as those of the above-mentioned substituent which may be possessed by the group constituting the main chain in the first linker.

Preferably, the structural unit represented by formula (IV') may be the structural unit represented by the following formula (IV"): 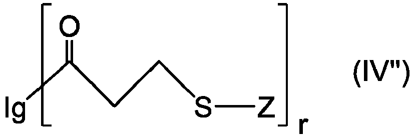 wherein
Ig, O, S, Z and r are the same as those of the above formula (IV).

The conjugate or salt thereof of the present invention can be obtained by reacting the thiol group-introduced antibody derivative or salt thereof of the present invention with a functional substance. Such a reaction can be carried out under a condition that cannot cause denaturation/degradation of a protein (immunoglobulin/antibody) (e.g., cleavage of an amide bond) (mild conditions as described above). As the functional substance, a substance having an arbitrary functional group capable of reacting with a thiol group under mild conditions can be used, but it is preferable to use a functional group that easily reacts with the thiol group. Examples of such a functional group include a maleimide group, a disulfide group, an α-haloketone, an α-haloamide, a benzyl bromide, and an iodoalkyl group. Alternatively, when the functional substance does not have a functional group that easily reacts with the bioorthogonal functional group, the functional substance derivatized as described above can be used. In the reaction, the molar ratio of the functional substance to the thiol group-introduced antibody derivative or salt thereof (functional substance/ thiol group-introduced antibody derivative or salt thereof) is not particularly limited, since it varies depending on factors such as the type of the thiol group-introduced antibody derivative or salt thereof and the functional substance and reaction time. It is, for example, 2 or more, preferably 3 or more, and more preferably 5 or more. In order to sufficiently react a functional substance with the thiol group of the thiol group-introduced antibody derivative in a short reaction time, a sufficient amount (eg, excess amount) of the functional substance with respect to the thiol group-introduced antibody derivative or salt thereof can be used.

The determination of the formation of the conjugate or salt thereof, which depends on its specific raw materials and the molecular weight of a product, can be performed by electrophoresis, chromatography (e.g., gel permutation chromatography, ion-exchange chromatography, reversed phase column chromatography, and high-performance liquid chromatography (HPLC)), or mass spectrometry, for example, and preferably mass spectrometry. Determination of regioselectivity can be performed by peptide mapping, for example. Peptide mapping can be performed by protease (e.g., trypsin and chymotrypsin) treatment and mass spectrometry, for example. For the protease, an endoprotease is preferred. Examples of such an endoprotease include trypsin, chymotrypsin, Glu-C, Lys-N, Lys-C, and Asp-N. Determination of the number of the introduced functional substance can be performed by electrophoresis, chromatography, or mass spectrometry, for example, and preferably mass spectrometry. The conjugate or salt thereof can be purified as appropriate by any method such as chromatography (e.g., chromatography described above and affinity chromatography).

### 6. Uses

The compound or salt thereof of the present invention can regioselectively modify an antibody at the position288/290, for example. Consequently, the present invention provides a reagent of derivatizing an antibody, comprising the compound or salt thereof of the present invention.

The reagent of the present invention may be provided in a form of a composition further comprising other components. Examples of such other compounds include solutions and stabilizers (e.g., antioxidants and preservatives). Among solutions, aqueous solutions are preferred. Examples of aqueous solutions include water (e.g., distilled water, sterilized distilled water, purified water, and a physiological saline solution) and buffers (e.g., an aqueous phosphoric acid solution, a Trishydrochloric acid buffer, a carbonic acid-bicarbonic acid buffer, an aqueous boric acid solution, a glycine-sodium hydroxide buffer, and a citric acid buffer); buffers are preferred. The pH of solutions is e.g., 5.0 to 9.0 and preferably 5.5 to 8.5. The reagent of the present invention can be provided in a liquid form or a powder form (e.g., freeze-dried powder).

The intermediate antibody or salt thereof of the present invention and the thiol group-introduced antibody derivative or salt thereof of the present invention are useful as intermediates for preparing a conjugate of an antibody and a functional substance or a salt thereof, for example.

The conjugate or salt thereof of the present invention is useful as pharmaceuticals or reagents (e.g., diagnostic reagents and reagents for research), for example. In particular, the conjugate or salt thereof of the present invention regioselectively modified with a functional substance and having an average bonding ratio of an antibody and a functional substance which is in a desired rage (1.5 to 2.5) is useful as pharmaceuticals. It is reported that when the number of bonds and the bond positions of a drug of an antibody drug conjugate (ADC) are changed, pharmacokinetics, a releasing rate of the drug, and effects change. Given these circumstances, nextgeneration ADCs are required to control the number and positions of a drug to be conjugated. It is believed that when the number and positions are constant, the problems of expected efficacy, variations in conjugate medicines, and lot difference, or what is called regulation, will be solved. Therefore, the conjugate or salt thereof of the present invention can solve such a problem of regulation.

The conjugate or salt thereof of the present invention having a functional substance may be provided in the form of a pharmaceutical composition. The pharmaceutical composition may comprise a pharmaceutically allowable carrier in addition to the conjugate or salt thereof of the present invention. Examples of the pharmaceutically allowable carrier include, but are not limited to, excipients such as sucrose, starch, mannitol, sorbitol, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrators such as starch, carboxymethylcellulose, hydroxypropyl starch, sodium hydrogencarbonate, calcium phosphate, and calcium citrate; lubricants such as magnesium stearate, Aerosil, talc, sodium lauryl sulfate; aromatics such as citric acid, menthol, glycyl lysine ammonium salts, glycine, and orange powder; preservatives such as sodium benzoate, sodium hydrogen sulfite, methylparaben, and propylparaben; stabilizers such as citric acid, sodium citrate, and acetic acid, suspensions such as methylcellulose, polyvinylpyrrolidone, and aluminum stearate; dispersants such as surfactants; diluents such as water, a physiological saline solution, and orange juice; and base waxes such as cacao butter, polyethylene glycol, and refined kerosene. The conjugate or salt thereof of the present invention may also have any modification (e.g., PEGylation) achieving stability.

Examples of preparations suitable for oral administration include liquid medicines dissolving an effective amount of a ligand in a diluted solution such as water, a physiological saline solution, or orange juice; capsules, sachets, and tablets comprising an effective amount of a ligand as a solid or granules; suspension medicines suspending an effective amount of an active ingredient in an appropriate dispersion medium; and emulsions dispersing a solution dissolving an effective amount of an active ingredient in an appropriate dispersion medium to be emulsified.

The pharmaceutical composition is suitable for nonoral administration (e.g., intravenous injection, hypodermic injection, intramuscular injection, local injection, and intraperitoneal administration). Examples of the pharmaceutical composition suitable for such nonoral administration include aqueous or nonaqueous, isotonic, aseptic injection medicines, which may comprise an antioxidant, a buffer, a bacteriostat, a tonicity agent, or the like. Examples thereof also include aqueous or nonaqueous, aseptic suspension medicines, which may comprise a suspension, a solubilizing agent, a thickener, a stabilizer, an antiseptic, or the like.

The dose of the pharmaceutical composition, which varies by the type and activity of an active ingredient, the severity of diseases, an animal type as a subject to be dosed, the drug receptivity, body weight, and age of a subject to be dosed, or the like, can be set as appropriate.

### EXAMPLES

The present invention is explained by the following Examples in more detail, but the present invention is not limited to the following Examples.

### [Example 1: Synthesis of an affinity substance to a soluble protein, a cleavable moiety and a reactive group (linked product of peptide thioester linker-thiophenol activator), and modification of an anti-HER2 antibody using the compound and analysis thereof]

### (1-1) Synthesis of IgG1 Fc Binding Peptide

The peptide of Ac-FNMQCQRRFYEALHDPNLNEEQRNARIRSIKDDC-NH₂ (SEQ ID NO: 2), which are affinity substance for soluble proteins, were synthesized by Fmoc solid-phase synthetic methods. A Liberty Blue manufactured by CEM was used as a peptide synthesizer. All reagents from Watanabe Chemical Industries were used. Resin were double-coupled with Fmoc-NH-SAL-PEG Resin, HL, arginine (R), cysteine (C), and histidine (H). The excision from Resin was carried out under stirring for 3 hours in trifluoroacetic acid:water:triisopropylsilane:ethanedithiol=94:2.5:1.0:2.5. After excision, Resin was removed by filtration and trifluoroacetic acid was removed. Diethyl ether was added to the resulting crystals, followed by ether precipitation, and the resulting white crystals were collected by filtration. This was dissolved in 0.1% trifluoroacetic acid aqueous solution and subjected to reverse phase high performance liquid chromatography using octadodecyl group chemically bonded silica gel as a filler, and eluted with a mixed solution of water and acetonitrile containing 0.1% trifluoroacetic acid, and the fractions were confirmed by LC-MS. Fractions containing the product were collected, and only acetonitrile was removed by concentration under reduced pressure, followed by lyophilization.

### (1-2) Forming an intramolecular disulfide bond at Cys of positions 5 and 34 of Ac-FNMQCQRRFYEALHDPNLNEEQRNARIRSIKDDC-NH₂ (SEQ ID NO: 2)

The peptide synthesized in (1-1) was dissolved in DMSO and 0.1 M Tris-HCl pH 8.0 was added. To this solution was added the glutathione oxidized form and stirred at room temperature for 20 hours. The reaction was stopped by adding an aqueous 2 M trifluoroacetic acid solution to the reaction solution, which was dissolved in a 0.05% aqueous trifluoroacetic acid solution, and subjected to reverse phase high performance liquid chromatography using octadodecyl group chemically bonded silica gel as a filler, and eluted with a mixed solution of water and acetonitrile containing 0.05% trifluoroacetic acid, and the fractions were confirmed by LC-MS. The fraction containing the product was collected, and the acetonitrile was removed by concentration under reduced pressure, followed by lyophilization to obtain the target substance (20.0 mg, 4.70 µmol).
MS(ESI) m/z:z=4 1063.65[M+4H]⁴⁺, z=5 851.15[M+5H]⁵⁺

### (1-3) Synthesis of the thioester linker

(1-3-1)

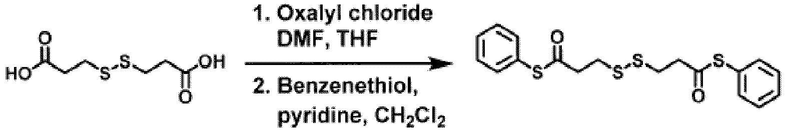

3,3'-Dithiodipropionic acid (1 g, 5.0 mmol) was solved in THF(10 mL) and DMF (100 µL) and OxalylChloride(1.5 mL, 15.0 mmol) was added at 0°C and stirred at 0°C for 15 minutes and at room temperature for 1 hour. Then, Benzenethiol (1.53 mL, 15.0 mmol), Pyridine (4 mL, 50 mmol) and CH₂Cl₂ (10 mL) were added at 0°C, and the mixture was stirred at room temperature for 3 hours. After confirming the reaction with TLC (hexane/ethyl acetate=5/1), the crystals were removed, extracted with ethyl acetate and 1 M hydrochloric acid aqueous solution, and the organic layers were concentrated. It was eluted with a mixed solution of hexane and ethyl acetate and the fractions were confirmed by TLC (hexane/ethyl acetate=5/1). Fractions containing the product were collected and concentrated under reduced pressure to remove the organic solvent, followed by vacuum-drying to obtain the above compound (1.2 g, 3.04 mmol).

(1-3-2)

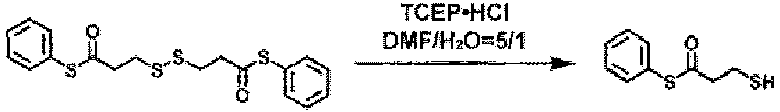

The compound (1.2 g, 3.04 mmol) synthesized in (1-3-1) was dissolved in a mixed solvent of DMF/H₂O=5/1, TCEP•HCl (1.74 g, 6.08 mmol) was added, and the mixture was stirred at room temperature for 1 hour. After confirming the reaction with TLC (hexane/ethyl acetate=5/1), the reaction was extracted with ethyl acetate and water, and then the organic layer was concentrated. It was eluted with a mixed solution of hexane and ethyl acetate and the fractions were confirmed by TLC (hexane/ethyl acetate=5/1). Fractions containing the product were collected and concentrated under reduced pressure to remove the organic solvents, followed by vacuum-drying to obtain the above compound (1.18 g, 6.5 mmol).

(1-3-3)

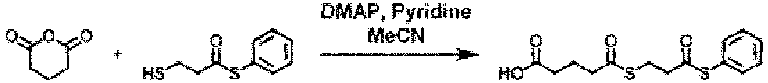

The compound (200 mg, 1.10 mmol) synthesized in (1-3-2) was dissolved in acetonitrile (2.0 mL), and Glutaric Anhydride(125.5 mg, 1.10 mmol), DMAP(6.72 mg, 0.06 mmol), and Pyridine(0.22 mL) was added, and the mixture was stirred at room temperature for 4 hours. After confirming the reaction with TLC (dichloromethane/methanol=10/1), the reaction was extracted with ethylacetate and 0.5 M HCl, and the organic layers were concentrated. It was eluted with a mixed solution of dichloromethane and methanol, and the fractions were confirmed by TLC (dichloromethane/methanol=10/1). Fractions containing the product were collected and concentrated under reduced pressure to remove the organic solvents, followed by vacuum-drying to obtain the above compound (135 mg, 0.45 mmol).

(1-3-4)

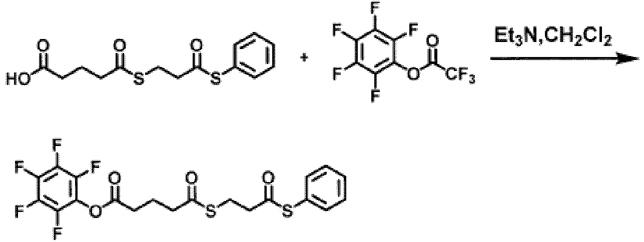

To the compound (134 mg, 0.45 mmol) synthesized in (1-3-3), CH₂Cl₂ (2.25 mL) and triethylamine (157 µL, 1.13 mmol) were added and dissolved. Pentafluorophenyl trifluoroacetic acid (154 µL, 0.90 mmol) was added at 0°C and stirred for 1 h. After confirming the reaction with TLC (hexane/ethyl acetate=3/1), the reaction solutions were concentrated. It was eluted with a mixed solution of hexane and ethyl acetate and the fractions were confirmed by TLC (hexane/ethyl acetate=3/1). Fractions containing the product were collected and concentrated under reduced pressure to remove the organic solvents, followed by vacuum-drying to obtain the above compound (96 mg, 0.20 mmol) .
¹H NMR (400 MHz, Chloroform-d) δ = 7.44 (s, 5H), 3.23 (t, J=6.9, 2H), 3.01 (t, J=6.9, 2H), 2.77 (dt, J=14.5, 7.3, 4H), 2.16 (t, J=7.3, 2H).

### (1-4) Linkage between peptide and linker

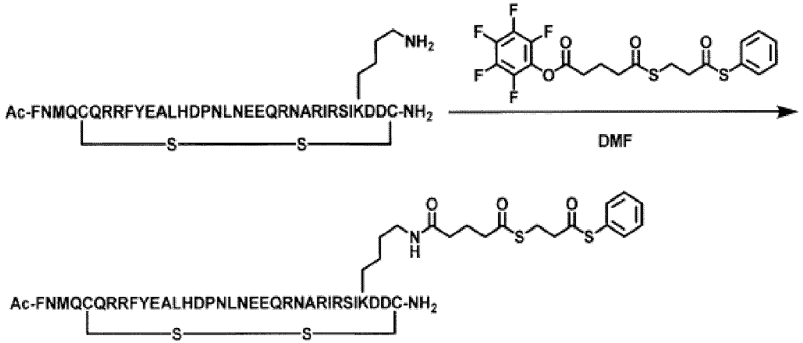

Both of the two aforementioned amino acid sequences are the amino acid sequence of SEQ ID NO: 2.

Ac-FNMQCQRRFYEALHDPNLNEEQRNARIRSIKDDC-NH₂ (SEQ ID NO: 2) synthesized in (1-2) (30.0 mg, 7.06 µmol, where two cysteine at positions 5 and 34 each forms a disulfide bond in the molecule) was dissolved in N,N-dimethylformamide (1.00 mL), a thioester linker (96.0 mg, 201 µmol) synthesized in (1-3) was added, and the mixture was stirred at room temperature for 24 hours. This was dissolved in a 0.05% aqueous trifluoroacetic acid solution and subjected to reverse phase high performance liquid chromatography using octadodecyl group chemically bonded silica gel as a filler, and eluted with a mixed solution of water and acetonitrile containing 0.05% trifluoroacetic acid, and the fractions were confirmed by LC-MS. The fraction containing the product was collected, and the acetonitrile was removed by concentration under reduced pressure, followed by lyophilization to obtain the above linked product of peptide thioester linker-thiophenol activator (15.8 mg, 3.48 µmol).
MS(ESI) m/z: z=4 1136.80[M+4H]⁴⁺

### (1-5) Specific Modification of the Anti HER2 IgG Antibody Trastuzumab and Analysis by ESI-TOFMS

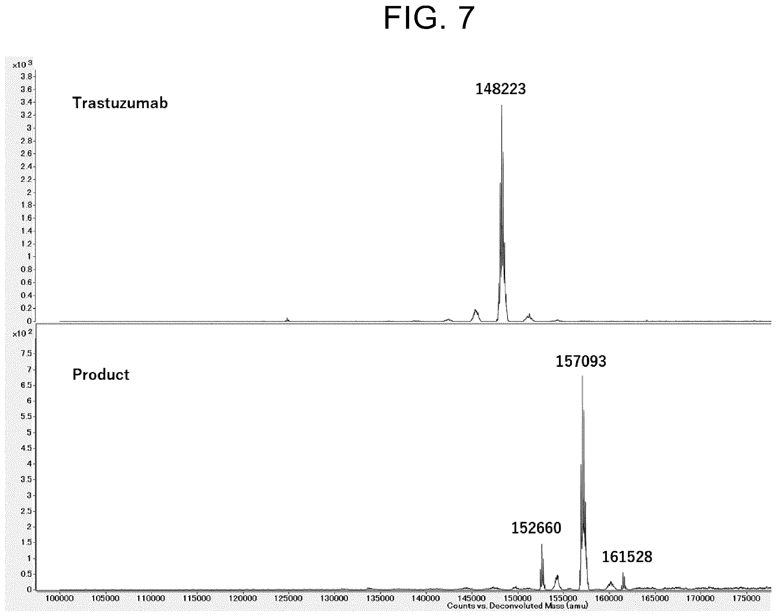

The linked product of peptide linker synthesized in (1-4) was dissolved in dimethyl sulfoxide so as to be 10 mM. 500 µg of the anti-HER2 antibody trastuzumab (Chugai Pharmaceutical Co., Ltd.) was dissolved in 200 µL (20 µM) of HEPES buffer (pH 8.2), and 3.38µL of 10 mM peptide reagent (10 equivalents to the antibody) was added and stirred at room temperature for 1 hour. The reactant was replaced with ammonium 20 mM acetate buffer and the masses were measured by ESI-TOFMS. As a result, trastuzumab as a raw material was observed to show a peak at 148223. The product identified a peak 152660 with one binding peptide introduced, a peak 157093 with two binding peptides introduced, and a peak 161528 with three introduced (FIG. 7) .

### (1-6) Confirmation of Heavy Chain Selectivity of Specific Modified Trastuzumab by ESI-TOFMS Analysis under Reducing Conditions

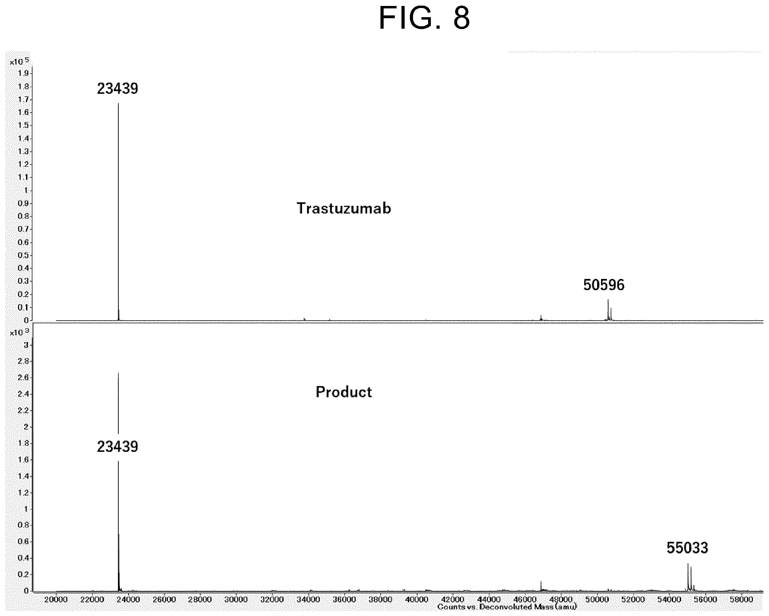

To the antibody-peptide complex produced in (1-5), 2 µL of 100 mM tris(2-carboxyethyl)phosphine hydrochloride (equivalent to the antibody) was added, and the mixture was stirred at room temperature for 15 minutes. The masses were measured by ESI-TOFMS. As a result, it is observed that the raw material trastuzumab had a heavy chain peak at 50596 and a light chain peak at 23439, and the product shows peak at 55033 in which one linker was introduced into the heavy chain and the light chain had a peak at 23439 which was the same as the raw material (FIG. 8).

### (1-7) Confirmation of Peptide/Antibody Bonding Ratio of Specific Modifications of Trastuzumab by DAR calculator

For MS data analyzed in (1-5), the peptide/antibody bonding ratio was confirmed by DAR calculator (Agilent Software), and the results are shown in Table 2. The average peptide/antibody bonding ratio calculated from DAR peak and % Area in Table-2 was 2.0. Therefore, the production of an antibody intermediate represented by the following structural formula (average peptide/antibody bonding ratio: 2.0) was confirmed. 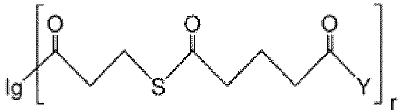 [where Ig represents an immunoglobulin unit (IgG) comprising two heavy chains and two light chains and forms an amide bond with a carbonyl group adjacent to Ig via an amino group in the side chain of the lysine residue present at position 288/290 in the two heavy chains according to EU numbering;
Y represents an affinity peptide represented by the amino acid sequence of SEQ ID NO: 2,
the average ratio r of the amide bonds per two heavy chains is 2.0.]

**Table 2**

| DAR Peak | Observed mass (Da) | Peak Area | %Area |
|---|---|---|---|
| 1 | 152660 | 6.23E+003 | 4.56 |
| 2 | 157093 | 1.22E+005 | 89.26 |
| 3 | 161528 | 8.44E+003 | 6.18 |

### (1-8) Preparation of Thiol Group-Introduced Antibody Derivatives by Cleavage of Thioester Group

Hydroxylamine solutions were added to the antibodyintermediates obtained in (1-7) in accordance with the previous report (WO2019/240287A1) and allowed to stand at room temperature for 1 hour. After 2 hours, the compound was replaced with 20 mM PBS buffer, 10 mM EDTA (pH 7.4) to obtain a thiol group-introduced antibody derivative. The masses were measured by ESI-TOFMS. As a result, the peak was confirmed at 148409 at which the cleavage proceeded. 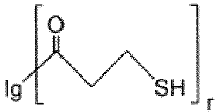 [where Ig represents an immunoglobulin unit (IgG) comprising two heavy chains and two light chains and forms an amide bond with a carbonyl group adjacent to Ig via an amino group in the side chain of the lysine residue present at position 288/290 in the two heavy chains according to EU numbering,
the average ratio r of the amide bonds per two heavy chains is 2.0.]

### (1-9) Peptide mapping by trypsinization

The thiol-introduced trastuzumab obtained in (1-8) was subjected to peptide mapping in the following steps.

### (1-9-1) Trypsinization of the thiol-introduced trastuzumab

To 1.5 mL low-adsorption microtest tube, 10 µL of the sample solution, 50 mM ammonium hydrogencarbonate buffer solution, and 10 µL of 20 mM dithiothreitol aqueous solution dissolved in 40% trifluoroethanol were added, and after heating at 65°C for 1 hour, 10µL of an 50 mM iodoacetamide aqueous solution was added, and the mixture was allowed to react for 30 minutes at room temperature under light shielding. After the reaction, 40µL of 50 mM ammonium bicarbonate buffer was added and stirred, and 10 µL of 20 ng/µL of trypsin aqueous solution was added and enzymatically digested at 37°C for 16 hours. After digestion, 2 µL of 20% aqueous trifluoroacetic acid solution was added to stop the reaction, and LC-MS/MS was measured.

### (1-9-2) LC-MS/MS determination of trastuzumab (Analyzer)

Nano HPLC:EASY-nLC 1000 (Thermo Fisher Scientific)
Mass Spectrometer: Tribrid Mass Spectrometer Orbitrap Fusion (Thermo Fisher Scientific)

### (HPLC Assay)

Trap Column : Acclaim PepMap^{®} 100,75 µ m × 2 cm (Thermo Fisher Scientific)
Analytical columns: ESI-column(NTCC-360/75-3-125, 75 µm × 12.5 cm, 3 µm (Nikkyo Technos Co., Ltd.)
Mobile phase A: 0.1% formic acid in water
Mobile phase B: 0.1% formic acid, acetonitrile solution
Loading solution: 0.1% aqueous trifluoroacetic acid solution
Flow rate: 300 nL/min
Sample injection volume: 1 µL
Gradient conditions (B%): 2% (0.0-0.5 min), 2% → 30% (0.5-23.5 min), 30% → 75% (23.5-25.5 min), 75% (25.5-35.0 min)

### (Mass Spectrometer Analysis Conditions)

Ionization method: ESI, Positive
Scan Type: Data Dependent Aquisition
Activation Type:Collision Induced Dissociation(CID)

Data were acquired using the attached software Xcalibur 3.0 (Thermo Fisher Scientific) and Thermo Orbitrap Fusion Tune Application 2.0 (Thermo Fisher Scientific).

### (1-9-3) Analysis of trastuzumab modification sites

Modification site analysis on the LC-MS/MS measurement result was performed using BioPharma Finder 3.0 (Thermo Fisher Scientific).

Analysis with BioPharma Finder was performed with S/N Threshold set to 1 and MS Noise Level set to 0.01% of the peak-top strength. The digestive enzyme was set to Trypsin and Specificity to High. Static Modification was set as a modification of cysteine residue by iodoacetamide, Carbamidomethyl (+57.021Da). For Dynamic Modifications, oxidations of methionine residues (+15.995Da) and modifications to lysine residues (thiol-introduced product that is carbamidomethylated with iodoacetamide (+145.019Da)) were established. In addition, filters were set so that Confidence Score was 80 or more, Mass Accuracy at the time of identification was 5ppm or less, and only those for which MS/MS was observable. Regarding the residue number of the lysine residue, the heavy chain VH domain and the light chain are indicated by the number in the sequence (i.e., the first amino acid at the N-terminal end; the same shall apply hereinafter), and the heavy chain CH1, CH2, CH3 domain is indicated by EU numbering.

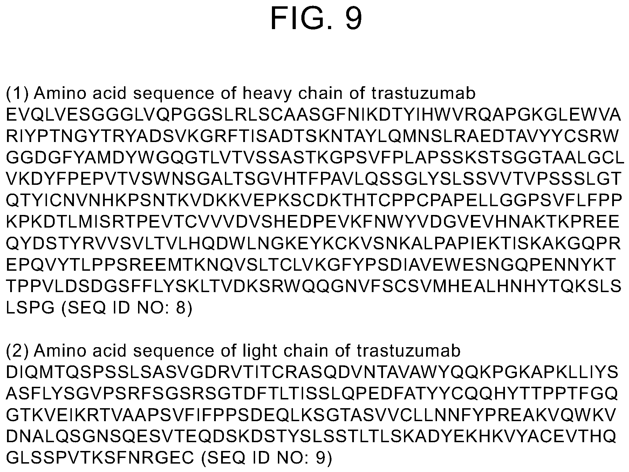

In addition, (1) and (2) shown in FIG. 9 were used as the data of the amino acid sequence to be searched for the modification site.

### (1-9-4) Analysis of trastuzumab modification sites by LC-MS/MS

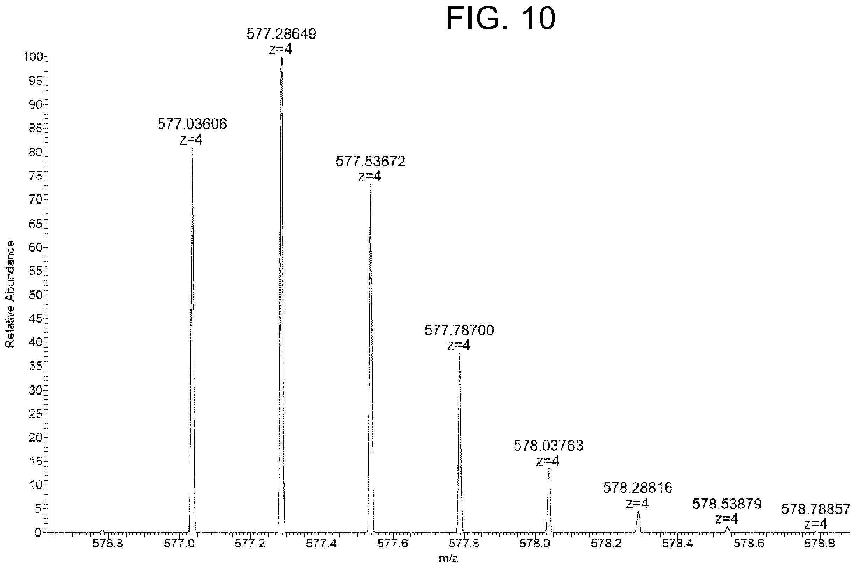

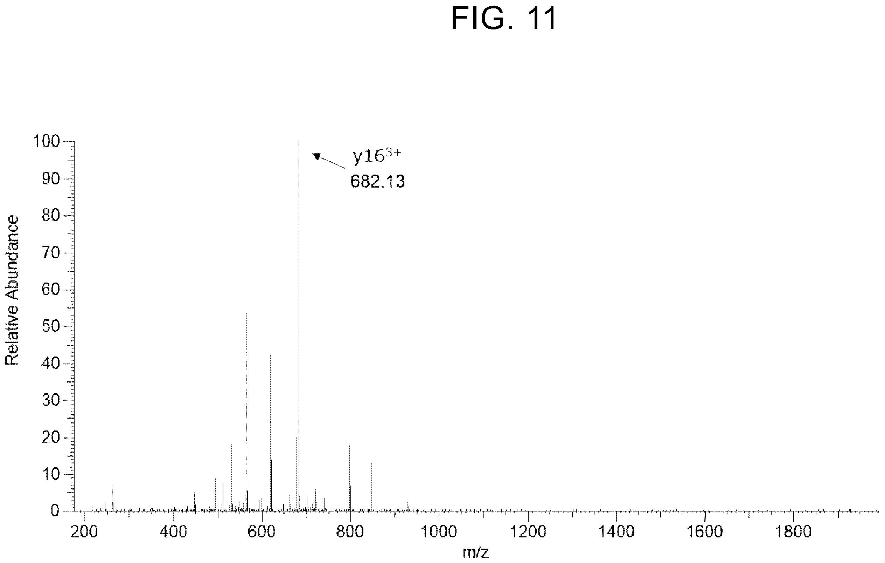

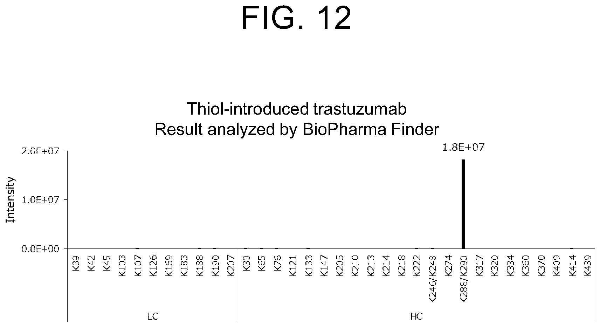

Analysis using LC-MS/MS revealed that MS spectra (found: m/z 577.03606, theoretical value: 577.03557, 4 values) of the peptide fragment FNWYVDGVEVHNAKTKPR (SEQ ID NO: 10) which is a peptide consisting of 18 amino acids including a modification site of trastuzumab to lysine residue by tryptic digestion of trastuzumab (thiol introduced product (+145.019Da) which is carbamidomethylated by iodoacetamide) were observed (FIG. 10), and m/z 682.13 (theoretical value: 682.01) corresponding to a trivalent y16 showing modification of the lysine residue at position 288 or 290 of the heavy chain according to EU numbering was confirmed from CID spectrum (FIG. 11). Analysis with BioPharma Finder also showed that the modification to the lysine residue at position 288 or 290 occurred highly selectively (FIG. 12).

The results showed that the thiol-introduced trastuzumab obtained in (1-8) above were conjugating at Lys288 and Lys290 of the heavy chain of the antibody according to EU numbering.

### [Example 2: Synthesis of an affinity substance to a soluble protein, a cleavable moiety and a reactive group (linked product of peptide thioester linker-thiophenol activator), and modification and analysis of an anti-HER2 antibody trastuzumab using the compound]

### (2-1) Synthesis of thioester linker

(2-1-1)

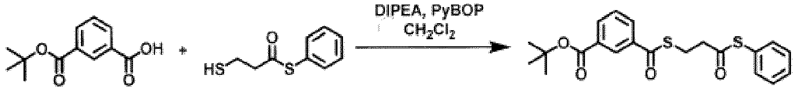

The compound (295 mg, 1.62 mmol) synthesized in (1-3-2) was dissolved in CH₂Cl₂ (13.5 mL) and 3-(tert-Butoxycarbonyl)benzoic acid (300 mg, 1.35 mmol), DIPEA (700 µL, 2.03 mmol), and PyBOP (843 mg, 1.62 mmol) were added and stirred at normal temperature for 1 h. After confirming the reaction with TLC (hexane/ethyl acetate=5/1), the reaction solutions were concentrated. It was eluted with a mixed solution of hexane and ethyl acetate and the fractions were confirmed by TLC (hexane/ethyl acetate=5/1). Fractions containing the product were collected and concentrated under reduced pressure to remove the organic solvents, followed by vacuum-drying to obtain the above compound (259 mg, 0.64 mmol).

(2-1-2)

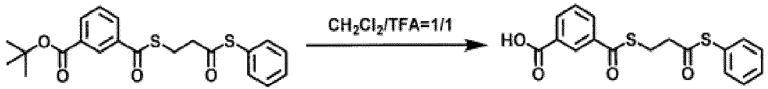

The compound (259 mg, 0.64 mmol) synthesized in (2-1-1) was dissolved in a mixed solution of CH₂Cl₂ and TFA=1/1, and stirred at room temperature for 1 hour. After confirming that the reaction solution had fallen to the starting point with TLC (hexane/ethyl acetate=5/1), the reaction solution was concentrated and then dried under reduced pressure to obtain the above compound (227 mg, 0.66 mmol).

(2-1-3)

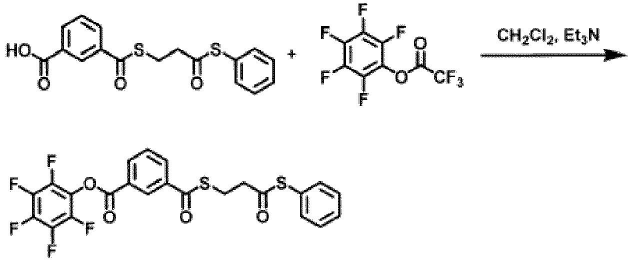

To the compound (227 mg, 0.66 mmol) synthesized in (2-1-2), CH₂Cl₂ (3.3 mL) and triethylamine (230 µL, 1.65 mmol) were added and dissolved. Pentafluorophenyl trifluoroacetic acid (225 µL, 1.32 mmol) was added at 0°C and stirred for 1 h. After confirming the reaction with TLC (hexane/ethyl acetate=3/1), the reaction solutions were concentrated. It was eluted with a mixed solution of hexane and ethyl acetate and the fractions were confirmed by TLC (hexane/ethyl acetate=3/1). Fractions containing the product were collected and concentrated under reduced pressure to remove the organic solvents, followed by vacuum-drying to obtain the above compound (175.3 mg, 0.34 mmol) .
¹H NMR (400 MHz, Chloroform-d) δ = 8.79 (t, J=1.8, 1H), 8.43 (dt, J=7.8, 1.5, 1H), 8.30 (dt, J=7.9, 1.5, 1H), 7.70 (t, J=7.8, 1H), 7.45 (s, 4H), 3.45 (t, J=6.9, 2H), 3.14 (t, J=6.9, 2H).

### (2-2) Linkage between peptide and linker

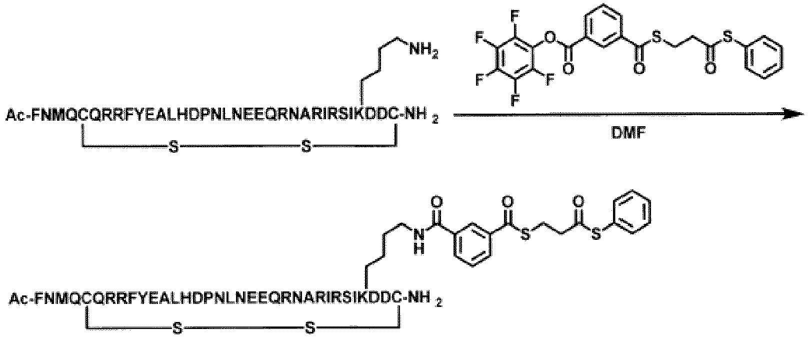

Both of the two aforementioned amino acid sequences are the amino acid sequence of SEQ ID NO: 2.

Ac-FNMQCQRRFYEALHDPNLNEEQRNARIRSIKDDC-NH₂ (SEQ ID NO: 2) synthesized in (1-2) (SEQ ID NO: 2) (30.0 mg,7. 06 µmol, where two cysteine at positions 5 and 34 each forms a disulfide bond in the molecule) was dissolved in N,N-dimethylformamide (1.00 mL), a linker (72.0 mg, 141 µmol) was added, and the mixture was stirred at room temperature for 24 hours. This was dissolved in a 0.05% aqueous trifluoroacetic acid solution and subjected to reverse phase high performance liquid chromatography using octadodecyl group chemically bonded silica gel as a filler, and eluted with a mixed solution of water and acetonitrile containing 0.05% trifluoroacetic acid, and the fractions were confirmed by LC-MS. The fraction containing the product was collected, and the acetonitrile was removed by concentration under reduced pressure, followed by lyophilization to obtain the aforementioned linked product of peptide thioester-thiophenol activator (10.0 mg, 2.19 µmol).
MS(ESI) m/z: z=4 1145.6[M+4H]⁴⁺

### (2-3) Specific Modification of Anti-HER2 Antibody Trastuzumab and Analysis by ESI-TOFMS

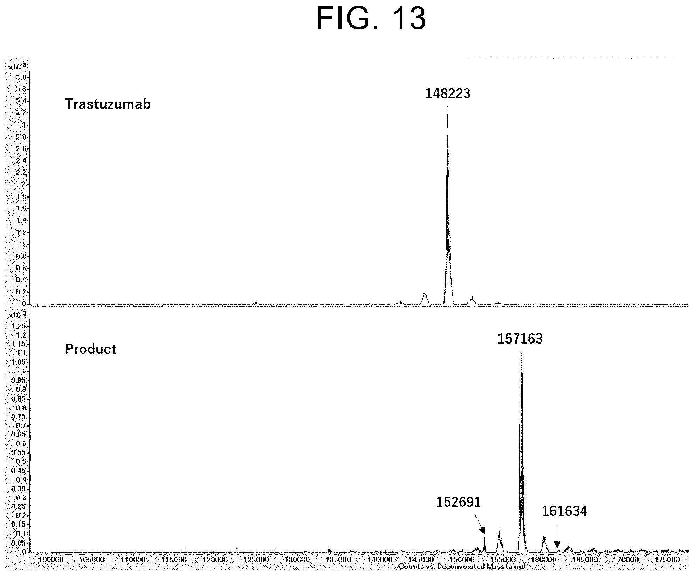

The linked product of peptide linker synthesized in (2-2) was dissolved in dimethyl sulfoxide so as to be 10 mM. 500 µg of the anti-HER2 antibody trastuzumab (Chugai Pharmaceutical Co., Ltd.) was dissolved in 200 µL (20 µM) of 50 mM HEPES buffer (pH 8.2), and 3.38 µL of 10 mM peptide reagent (10 equivalents to the antibody) was added and stirred at room temperature for 1 hour. The reaction solution was replaced with 20 mM ammonium acetate buffer. Mass is measured by ESI-TOFMS. As a result, it was observed that the raw material trastuzumab showed a peak at 148223 and a peak at 152691 with one bonding peptide introduced, a peak 157163 with two bonding peptides introduced, and a peak at 161634 with three introduced were confirmed (FIG. 13).

### (2-4) Confirmation of Heavy Chain Selectivity of Specific Modified Trastuzumab under Reducing Conditions by ESI-TOFMS Analysis under Reducing Conditions

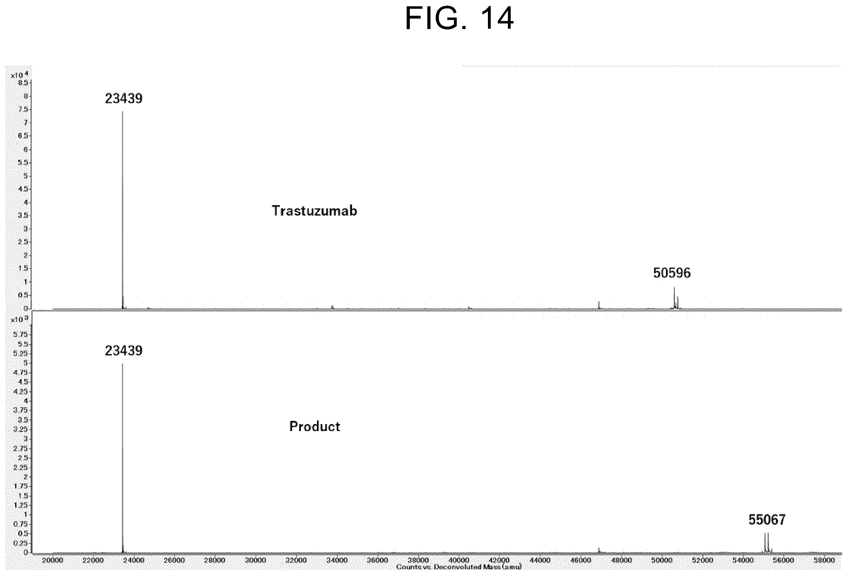

To the antibody-peptide complex produced in (2-3), 2 µL of 100 mM tris(2-carboxyethyl)phosphine hydrochloride (equivalent to the antibody) was added, and the mixture was stirred at room temperature for 15 minutes. The masses were measured by ESI-TOFMS. As a result, the raw material trastuzumab was observed to have a heavy chain peak at 50596 and a light chain peak at 23439. The reactants had a peak at 55067 in which one linker was introduced into the heavy chain and a peak at 23439 in which the light chain was the same as the starting material (FIG. 14).

### (2-5) Confirmation of Peptide/Antibody Bonding Ratio of Specific Modifications of Trastuzumab by DAR calculator

For MS data analyzed in (2-3), the peptide/antibody bonding ratio was confirmed by DAR calculator (Agilent Software). The results are shown in Table 3. The average peptide/antibody bonding ratio calculated from DAR peak and % Area in Table-3 was 2.0. Therefore, the production of an antibody intermediate represented by the following structural formula (average peptide/antibody bonding ratio: 2.0) was confirmed. 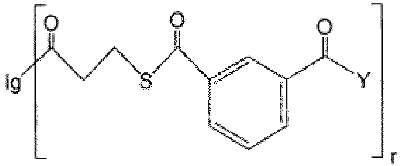 [where Ig represents an immunoglobulin unit (IgG) comprising two heavy chains and two light chains and forms an amide bond with a carbonyl group adjacent to Ig via an amino group in the side chain of the lysine residue present at position 288/290 in the two heavy chains according to EU numbering;
Y represents an affinity peptide represented by the amino acid sequence of SEQ ID NO: 2,
The average ratio r of the amide bonds per two heavy chains is 2.0.]

**Table 3**

| DAR Peak | Observed mass(Da) | Peak Area | %Area |
|---|---|---|---|
| 1 | 152691 | 5.74E+003 | 4.25 |
| 2 | 157163 | 1.26E+005 | 93.33 |
| 3 | 161634 | 3.27E+003 | 2.42 |

### (2-6) Preparation of Thiol Group-Introduced Antibody Derivatives by Cleavage of Thioester Group

The thiol group-introduced antibody derivative was obtained by subjecting the antibody intermediate obtained in (2-5) to the cleavage reaction of the thioester group described in (1-8) to obtain the thiol group-introduced antibody derivative described in (1-8).

### (2-7) Peptide mapping by trypsinization

The thiol-introduced trastuzumab obtained in (2-6) was subjected to peptide mapping in the following step.

### (2-7-1) Trypsinization of the thiol-introduced trastuzumab

Trypsinization of the thiol-introduced trastuzumab obtained in (2-6) was performed in the same manner as in (1-9-1).

### (2-7-2) LC-MS/MS determination of trastuzumab

LC-MS/MS was measured in the same manner as in (1-9-2) .

### (2-7-3) Analysis of trastuzumab modification sites

Analysis was performed in the same manner as in (1-9-3) .

### (2-7-4) Analysis of trastuzumab modification sites by LC-MS/MS

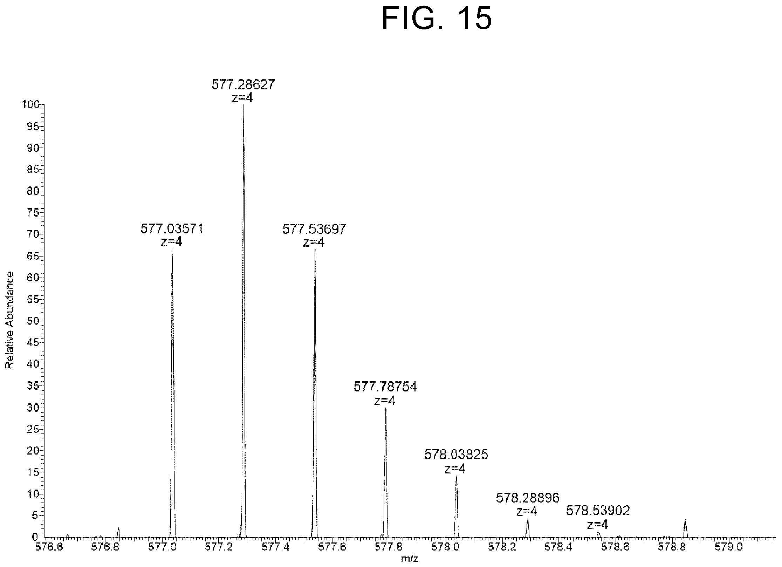

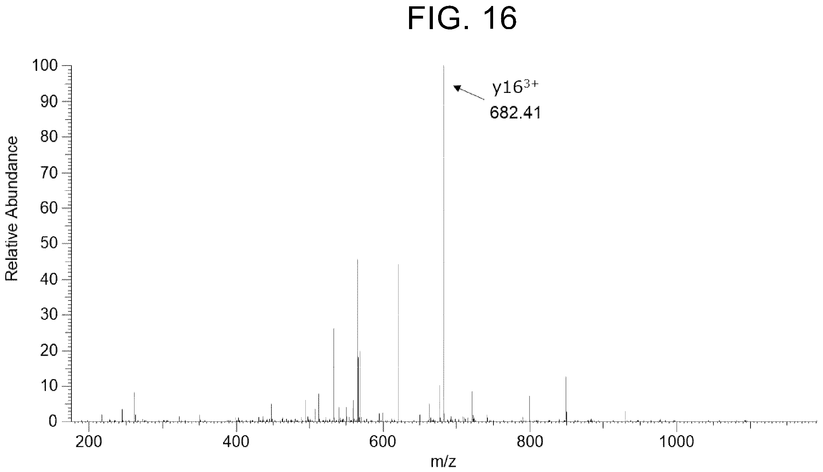

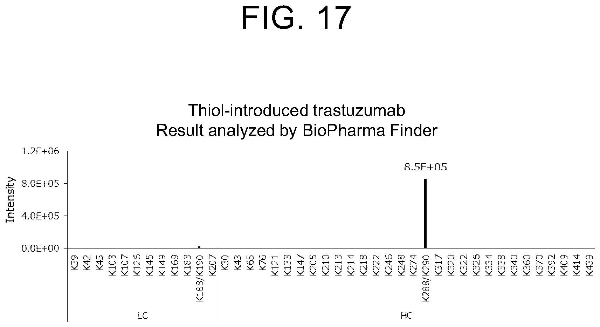

Analysis using LC-MS/MS revealed that MS spectra (found: m/z 577.03571, theoretical value: 577.03557, tetravalent) of the peptide fragment FNWYVDGVEVHNAKTKPR (SEQ ID NO: 10) which is a peptide consisting of 18 amino acids including a modification site of trastuzumab to lysine residue by tryptic digestion of trastuzumab (thiol introduced product (+145.019Da) which is carbamidomethylated by iodoacetamide) were observed (FIG. 15), and m/z 682.41 (theoretical value: 682.01) corresponding to a trivalent y16 showing modification of the lysine residue at position 288 or 290 of the heavy chain according to EU numbering was confirmed from CID spectrum (FIG. 16). Analysis with BioPharma Finder also showed that the modification to the lysine residue at position 288 or 290 occurred highly selectively (FIG. 17).

From the above, it was found that the thiol-introduced trastuzumab obtained in the above (2-6) were conjugated to Lys288 and Lys290 of the heavy chain according to EU numbering in a regioselective manner.

### [Example 3: Synthesis of an affinity substance to a soluble protein, a cleavable moiety and a reactive group (linked product of peptide thioester linker-thiophenol activator), and modification of an anti-HER2 antibody using the compound and analysis thereof]

### (3-1) Synthesis of thioester linker

(3-1-1)

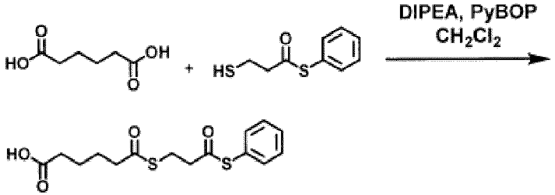

The compound (220 mg, 1.21 mmol) synthesized in (1-3-2) was dissolved in CH₂Cl₂ (12.0 mL) and Adipic Acid (530 mg, 3.63 mmol), DIPEA (314 µL, 1.82 mmol), and PyBOP (755 mg, 1.45 mmol) were added and stirred for 1 h at normal temperature. After confirming the reaction with TLC (dichloromethane/methanol=10/1), the reaction solutions were concentrated. It was eluted with a mixed solution of dichloromethane and methanol, and the fractions were confirmed by TLC (dichloromethane/methanol=10/1). Fractions containing the product were collected and concentrated under reduced pressure to remove the organic solvents, followed by vacuum-drying to obtain the above compound (205 mg, 0.63 mmol).

(3-1-2)

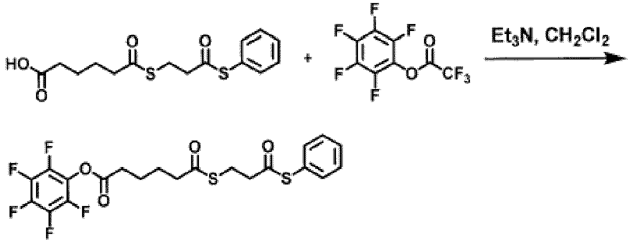

To the compound (205 mg, 0.63 mmol) synthesized in (3-1-1), CH₂Cl₂ (3.15 mL) and triethylamine (220 µL, 1.58 mmol) were added and dissolved. Pentafluorophenyl trifluoroacetic acid (215µL, 1.26mmol) was added at 0°C and stirred for 1 h. After confirming the reaction with TLC (hexane/ethyl acetate=3/1), the reaction solutions were concentrated. It was eluted with a mixed solution of hexane and ethyl acetate and the fractions were confirmed by TLC (hexane/ethyl acetate=3/1). Fractions containing the product were collected and concentrated under reduced pressure to remove the organic solvents, followed by vacuum-drying to obtain the above compound (200 mg, 0.41 mmol).
¹H NMR (400 MHz, Chloroform-d) δ = 7.44 (s, 5H), 3.20 (t, J=7.0, 2H), 3.00 (t, J=6.9, 2H), 2.66 (dt, J=28.3, 7.4, 5H), 1.79 (ddt, J=20.4, 15.2, 7.5, 5H), 1.57 - 1.38 (m, 3H) .

### (3-2) Linkage between peptide and linker

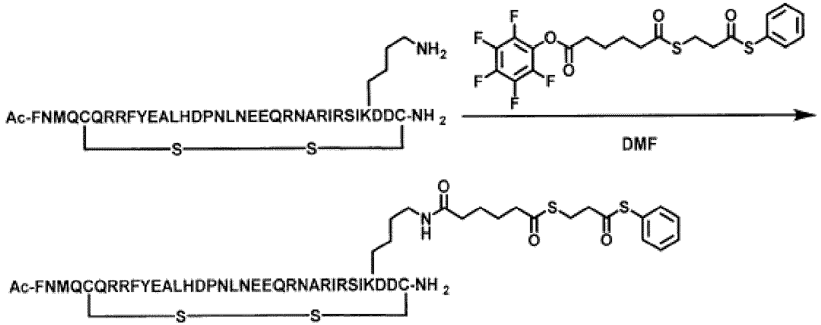

Both of the two aforementioned amino acid sequences are the amino acid sequence of SEQ ID NO: 2.

Ac-FNMQCQRRFYEALHDPNLNEEQRNARIRSIKDDC-NH₂ (SEQ ID NO: 2) (SEQ ID NO: 2) synthesized in (1-2) (30.0 mg, 7. 06 µmol, where two cysteines at positions 5 and 34 each forms a disulfide bond in the molecule) was dissolved in N,N-dimethylformamide (1.00 mL), a linker (69.0 mg, 141 µmol) was added, and the mixture was stirred at room temperature for 24 hours. This was dissolved in a 0.05% aqueous trifluoroacetic acid solution and subjected to reverse phase high performance liquid chromatography using octadodecyl group chemically bonded silica gel as a filler, and eluted with a mixed solution of water and acetonitrile containing 0.05% trifluoroacetic acid, and the fractions were confirmed by LC-MS. The fraction containing the product was collected, and the acetonitrile was removed by concentration under reduced pressure, followed by lyophilization to obtain the aforementioned linked product of peptide thioester-thiophenol activator (7.5 mg, 1.65 µmol).
MS(ESI) m/z: z=4 1140.50[M+4H]⁴⁺

### (3-3) Specific Modification of the Anti-HER2 Antibody Trastuzumab and Analysis by ESI-TOFMS

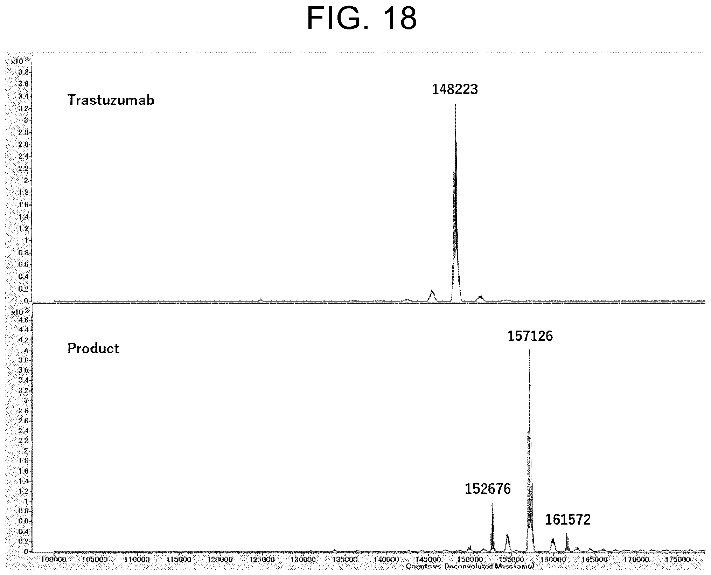

The linked product of peptide linker synthesized in (3-2) was dissolved in dimethyl sulfoxide so as to be 10 mM. 500 µg of the anti-HER2 antibody trastuzumab (Chugai Pharmaceutical Co., Ltd.) was dissolved in 200 µL (20 µM) of 50 mM HEPES buffer (pH 8.2), and 3.38 µL of 10 mM peptide reagent (10 equivalents to the antibody) was added and stirred at room temperature for 1 hour. The reaction solution was replaced with 20 mM ammonium acetate buffer. Mass is measured by ESI-TOFMS. As a result, the raw material trastuzumab had a peak at 148223. A peak 152676 with one binding peptide introduced, a peak 157126 with two binding peptides introduced, and a peak 161572 with three introduced were confirmed (FIG. 18).

### (3-4) Confirmation of Heavy Chain Selectivity of Specific Modified Trastuzumab by ESI-TOFMS Analysis under Reducing Conditions

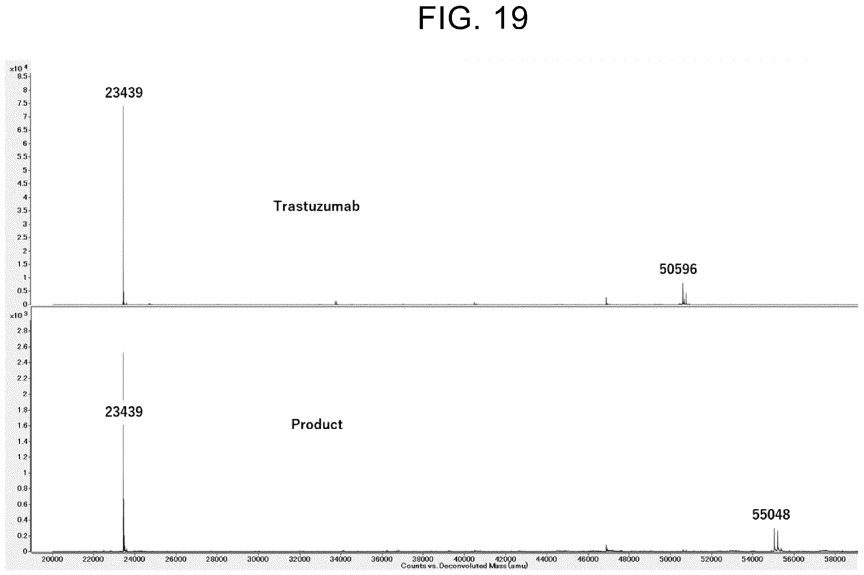

To the antibody-peptide complex produced in (3-3), 2 µL of 100 mM tris(2-carboxyethyl)phosphine hydrochloride (equivalent to the antibody) was added, and the mixture was stirred at room temperature for 15 minutes. The masses were measured by ESI-TOFMS. As a result, the raw material trastuzumab was observed to have a heavy chain peak at 50596 and a light chain peak at 23439. The reactants had a peak at 55048 in which one linker was introduced into the heavy chain and a peak at 23439 in which the light chain was the same as the starting material (FIG. 19).

### (3-5) Confirmation of Peptide/Antibody Bonding Ratio of Specific Modifications of Trastuzumab by DAR calculator

For MS data analyzed in (3-3), the peptide/antibody bonding ratio was confirmed by DAR calculator (Agilent Software). The results are shown in Table 4. The average peptide/antibody bonding ratio calculated from DAR peak and % Area in Table-4 was 1.9. Therefore, the production of an antibody intermediate represented by the following structural formula (average peptide/antibody bonding ratio: 1.9) was confirmed. 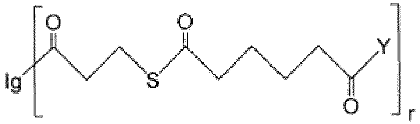 [where Ig represents an immunoglobulin unit (IgG) comprising two heavy chains and two light chains and forms an amide bond with a carbonyl group adjacent to Ig via an amino group in the side chain of the lysine residue present at position 288/290 in the two heavy chains according to EU numbering;
Y represents an affinity peptide represented by the amino acid sequence of SEQ ID NO: 2,
The average ratio r of the amide bonds per two heavy chains is 1.9.]

**Table 4**

| DAR Peak | Observed mass (Da) | Peak Area | %Area |
|---|---|---|---|
| 1 | 152676 | 1.71E+004 | 13.70 |
| 2 | 157126 | 1.01E+005 | 81.15 |
| 3 | 161572 | 6.43E+003 | 5.15 |

### (3-6) Preparation of Thiol Group-Introduced Antibody Derivatives by Cleavage of Thioester Group

The thiol group-introduced antibody derivative was obtained by subjecting the antibody intermediate obtained in (3-5) to the cleavage reaction of the thioester group described in (1-8) to obtain the thiol group-introduced antibody derivative described in (1-8).

### [Example 4: Synthesis of an affinity substance to a soluble protein, a cleavable moiety and a reactive group (linked product of peptide thioester linker-thiophenol activator), and modification of an anti-HER2 antibody using the compound and analysis thereof]

### (4-1) Synthesis of the thioester linker

(4-1-1)

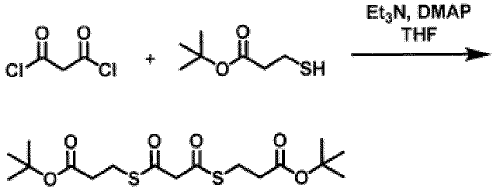

tBu-3-Sulfanylpropanoate(500 mg, 3.08 mmol) was dissolved in tetrahydrofuran (7 mL), triethylamine (0.64 mL, 4.62 mmol) was added, and then malonyl chloride (0.15 mg, 1.54 mmol) was added at 0°C and stirred for 3 hours. After confirming the reaction with TLC (hexane/ethyl acetate=5/1), the reaction solutions were concentrated. It was eluted with a mixed solution of hexane and ethyl acetate and the fractions were confirmed by TLC (hexane/ethyl acetate=5/1). Fractions containing the product were collected and concentrated under reduced pressure to remove the organic solvents, followed by vacuum-drying to obtain the above compound (104 mg, 0.26 mmol).

(4-1-2)

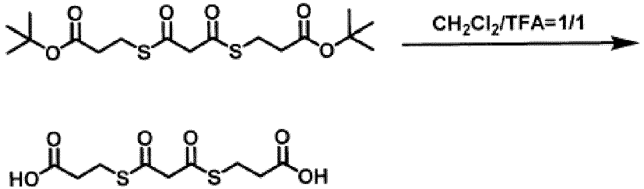

The compound (104 mg, 0.26 mmol) synthesized in (4-1-1) was dissolved in a mixed solution of CH₂Cl₂ and TFA=1/1, and stirred at room temperature for 1 hour. After confirming that the reaction solution had fallen to the starting point with TLC (hexane/ethyl acetate=5/1), the reaction solution was concentrated and then dried under reduced pressure to obtain the above compound (104 mg, 0.37 mmol).

(4-1-3)

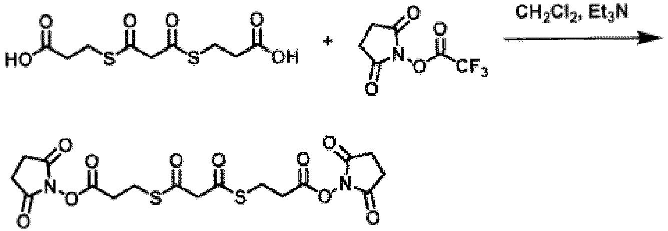

To the compound (104 mg, 0.37 mmol) synthesized in (4-1-2), CH₂Cl₂ (1.85 mL) and triethylamine (130 µL, 0.93 mmol) were added and dissolved. N-SuccinimidylTrifluoroacetate (156 mg, 0.74 mmol) was added at 0°C and stirred for 1 h. After confirming the reaction with TLC (hexane/ethyl acetate=1/1), the reaction solutions were concentrated. It was eluted with a mixed solution of hexane and ethyl acetate, and the fractions were confirmed by TLC (hexane/ethyl acetate=1/1). Fractions containing the product were collected and concentrated under reduced pressure to remove the organic solvents, followed by vacuum-drying to obtain the above compound (66.8 mg, 0.14 mmol).
¹H NMR (400 MHz, Chloroform-d) δ = 3.84 (s, 2H), 3.28 (t, J=6.9, 2H), 3.19 (t, J=6.8, 2H), 3.00 (t, J=6.8, 2H), 2.74 (t, J=6.8, 2H).

### (4-2) Linkage between peptide and linker

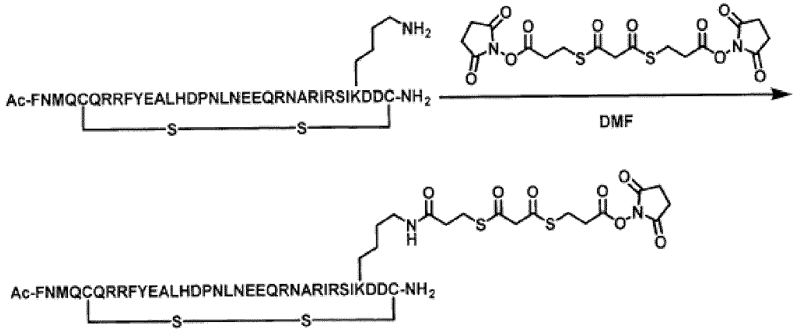

Both of the two aforementioned amino acid sequences are the amino acid sequence of SEQ ID NO: 2.

Ac-FNMQCQRRFYEALHDPNLNEEQRNARIRSIKDDC-NH₂ (SEQ ID NO: 2) synthesized in (1-2) (SEQ ID NO: 2) (29.7 mg, 7.00 µmol, where two cysteines at positions 5 and 34 each forms a disulfide bond in the molecule) was dissolved in N,N-dimethylformamide (1.00 mL), a linker (66.8 mg, 0.14 mmol) was added, and the mixture was stirred at room temperature for 4 hours. This was dissolved in a 0.05% aqueous trifluoroacetic acid solution and subjected to reverse phase high performance liquid chromatography using octadodecyl group chemically bonded silica gel as a filler, and eluted with a mixed solution of water and acetonitrile containing 0.05% trifluoroacetic acid, and the fractions were confirmed by LC-MS. The fraction containing the product was collected, and the acetonitrile was removed by concentration under reduced pressure, followed by lyophilization to obtain the linked product of thioester linker-NHS activator (13 mg, 2.82 µmol).
MS(ESI) m/z: z=4 1153.10[M+4H]⁴⁺

### (4-3) Specific Modification of the Anti-HER2 Antibody Trastuzumab and Analysis by ESI-TOFMS

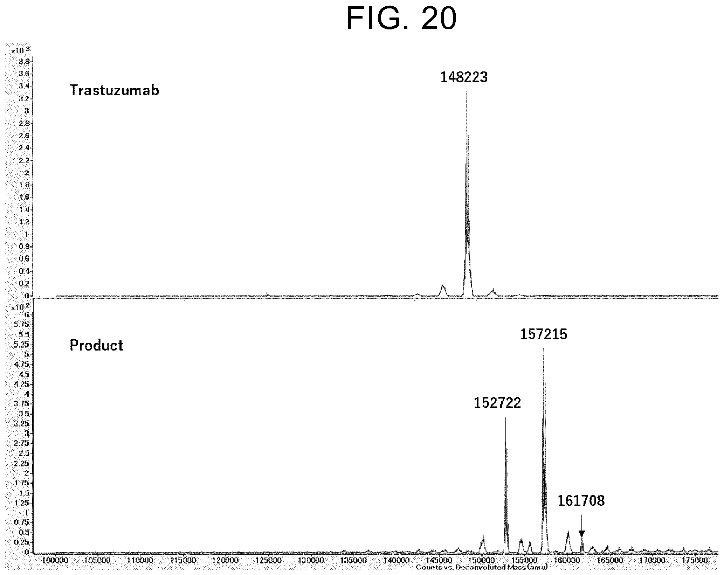

The linked product of peptide linker synthesized in (4-2) was dissolved in dimethyl sulfoxide so as to be 10 mM. 500 µg of the anti-HER2 antibody trastuzumab (Chugai Pharmaceutical Co., Ltd.) was dissolved in 200 µL (20 µM) of 50 mM HEPES buffer (pH 8.2), and 3.38 µL of 10 mM peptide reagent (10 equivalents to the antibody) was added and stirred at room temperature for 1 hour. The reaction solution was replaced with 20 mM ammonium acetate buffer. Mass was measured by ESI-TOFMS. As a result, the raw material trastuzumab had a peak at 148223. A peak 152722 with one binding peptide introduced, a peak 157215 with two binding peptides introduced, and a peak 161708 with three introduced were confirmed (FIG. 20).

### (4-4) Confirmation of Heavy Chain Selectivity of Specific Modified Trastuzumab by ESI-TOFMS Analysis under Reducing Conditions

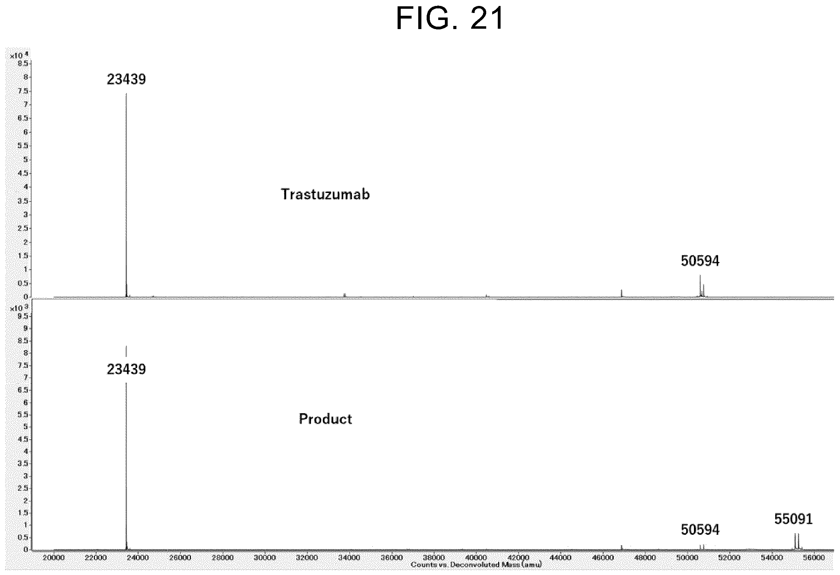

To the antibody-peptide complex produced in (4-3), 2 µL of 100 mM tris(2-carboxyethyl)phosphine hydrochloride (equivalent to the antibody) was added, and the mixture was stirred at room temperature for 15 minutes. The masses were measured by ESI-TOFMS. As a result, the raw material trastuzumab was observed to have a heavy chain peak at 50594 and a light chain peak at 23439. The reactants had a peak at 55091 in which one linker was introduced into the heavy chain and a peak at 23439 in which the light chain was the same as the starting material (FIG. 21).

### (4-5) Confirmation of Peptide/Antibody Bonding Ratio of Specific Modifications of Trastuzumab by DAR calculator

For MS data analyzed in (4-3), the peptide/antibody bonding ratio was confirmed by DAR calculator (Agilent Software). The results are shown in Table 5. The average peptide/antibody bonding ratio calculated from DAR peak and % Area in Table-5 was 1.8. Therefore, the production of an antibody intermediate represented by the following structural formula (average peptide/antibody bonding ratio: 1.8) was confirmed. 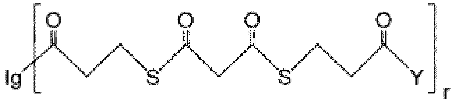 [where Ig represents an immunoglobulin unit (IgG) comprising two heavy chains and two light chains and forms an amide bond with a carbonyl group adjacent to Ig via an amino group in the side chain of the lysine residue present at position 288/290 in the two heavy chains according to EU numbering;
Y represents an affinity peptide represented by the amino acid sequence of SEQ ID NO: 2,
the average ratio r of the amide bonds per two heavy chains is 1.8.]

**Table 5**

| DAR Peak | Observed mass (Da) | Peak Area | %Area |
|---|---|---|---|
| 1 | 152722 | 4.47E+005 | 24.92 |
| 2 | 157215 | 1.21E+006 | 67.20 |
| 3 | 161708 | 1.22E+005 | 6.78 |

### (4-6) Preparation of Thiol Group-Introduced Antibody Derivatives by Cleavage of Thioester Group

The thiol group-introduced antibody derivative was obtained by subjecting the antibody intermediate obtained in (4-5) to the cleavage reaction of the thioester group described in (1-8) to obtain the thiol group-introduced antibody derivative described in (1-8).

### [Example 5: Synthesis of an affinity substance to a soluble protein, a cleavable moiety and a reactive group (linked product of peptide thioester linker-thiophenol activator), and modification of an anti-HER2 antibody using the compound and analysis thereof]

### (5-1) Linkage between peptide and linker

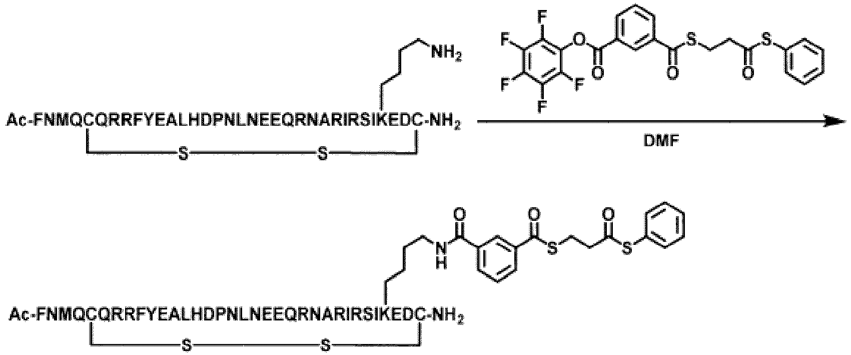

Both of the two aforementioned amino acid sequences are the amino acid sequence of SEQ ID NO: 3.

The linker was attached to Ac-FNMQCQRRFYEALHDPNLNEEQRNARIRSIKEDC-NH₂ (SEQ ID NO: 3) synthesized by the method described in (1-1) (30.0 mg, 7. 06 µmol, where two cysteines at positions 5 and 34 each formed an intramolecular disulfide bond) in the same manner as in Example 2 (2-2) to obtain the linked product of peptide thioester-thiophenol activator (10.0 mg, 2.19 µmol).
MS(ESI)m/z:z=4 1145.6[M+4H]⁴⁺

### (5-2) Specific Modification of the Anti-HER2 Antibody Trastuzumab and Analysis by ESI-TOFMS

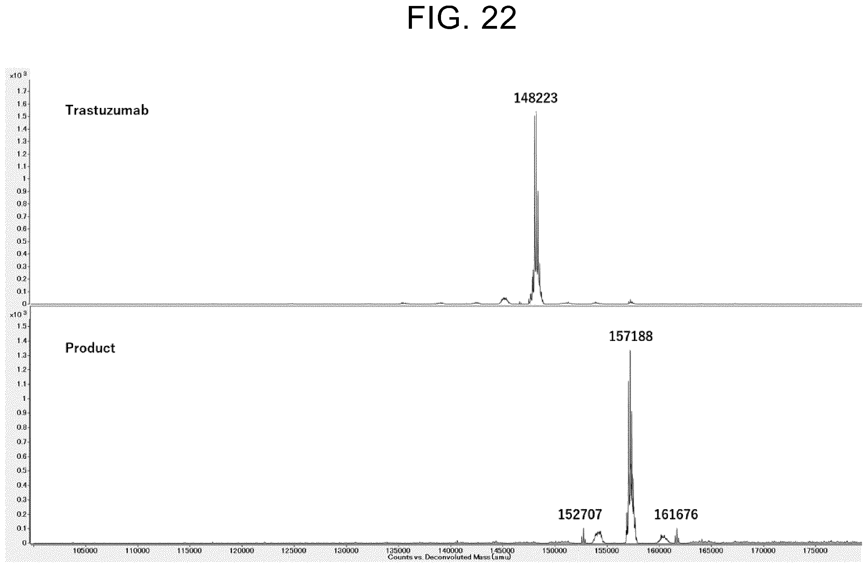

The linked product of peptide linker synthesized in (5-1) was dissolved in dimethyl sulfoxide so as to be 10 mM. 500 µg of the anti-HER2 antibody trastuzumab (Chugai Pharmaceutical Co., Ltd.) was dissolved in 200 µL (20 µM) of 50 mM HEPES buffer (pH 8.2), and 3.38 µL of 10 mM peptide reagent (10 equivalents to the antibody) was added and stirred at room temperature for 1 hour. The reaction solution was replaced with 20 mM ammonium acetate buffer. Mass was measured by ESI-TOFMS. As a result, the raw material trastuzumab had a peak at 148223. A peak 152707 with one binding peptide introduced, a peak 157188 with two binding peptides introduced, and a peak 161676 with three introduced were confirmed (FIG. 22).

### (5-3) Confirmation of Heavy Chain Selectivity of Specific Modified Trastuzumab by ESI-TOFMS Analysis under Reducing Conditions

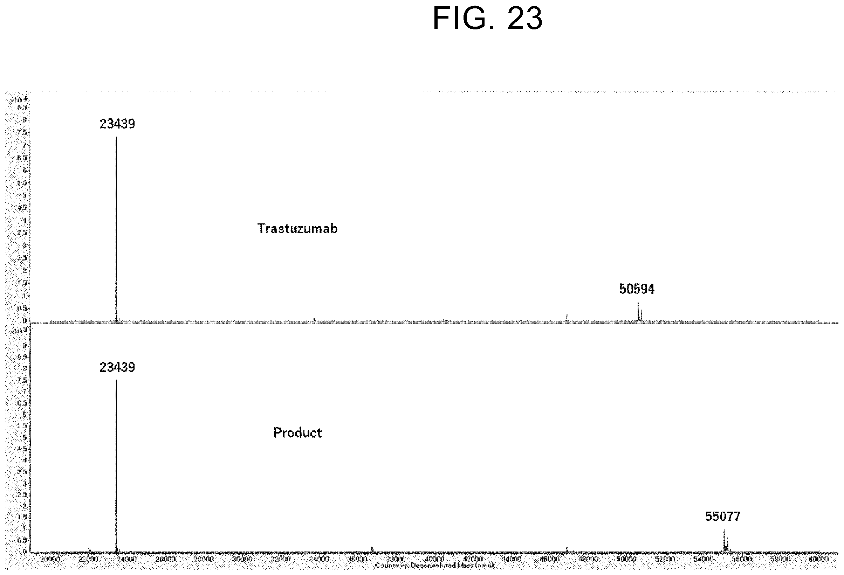

To the antibody-peptide complex produced in (5-2), 2 µL of 100 mM tris(2-carboxyethyl)phosphine hydrochloride (equivalent to the antibody) was added, and the mixture was stirred at room temperature for 15 minutes. The masses were measured by ESI-TOFMS. As a result, the raw material trastuzumab was observed to have a heavy chain peak at 50596 and a light chain peak at 23439. The reactants had a peak at 55077 in which one linker was introduced into the heavy chain and at 23439 in which the light chain was the same as the starting material (FIG. 23).

### (5-4) Confirmation of Peptide/Antibody Bonding Ratio of Specific Modifications of Trastuzumab by DAR calculator

For MS data analyzed in (5-2), the peptide/antibody bonding ratio was confirmed by DAR calculator (Agilent Software). The results are shown in Table 6. The average peptide/antibody bonding ratio calculated from DAR peak and % Area in Table-6 was 2.0. Therefore, the production of an antibody intermediate represented by the following structural formula (average peptide/antibody bonding ratio: 2.0) was confirmed. 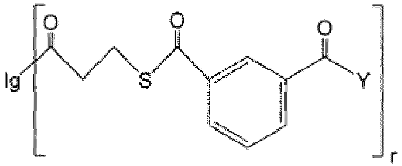 [where Ig represents an immunoglobulin unit (IgG) comprising two heavy chains and two light chains and forms an amide bond with a carbonyl group adjacent to Ig via an amino group in the side chain of the lysine residue present at position 288/290 in the two heavy chains according to EU numbering;
Y represents an affinity peptide represented by the amino acid sequence of SEQ ID NO: 3,
The average ratio r of the amide bonds per two heavy chains is 2.0.]

**Table 6**

| DAR Peak | Observed mass (Da) | Peak Area | %Area |
|---|---|---|---|
| 1 | 152707 | 6.90E+003 | 2.22 |
| 2 | 157188 | 2.96E+005 | 95.07 |
| 3 | 161676 | 8.47E+003 | 2.72 |

### (5-5) Preparation of Thiol Group-Introduced Antibody Derivatives by Cleavage of Thioester Group

The thiol group-introduced antibody derivative was obtained by subjecting the antibody intermediate obtained in (5-4) to the cleavage reaction of the thioester group described in (1-8) to obtain the thiol group-introduced antibody derivative described in (1-8).

### [Example 6: Synthesis of an affinity substance to a soluble protein, a cleavable moiety and a reactive group (linked product of peptide thioester linker-thiophenol activator), and modification of an anti-HER2 antibody using the compound and analysis thereof]

### (6-1) Linkage between peptide and linker

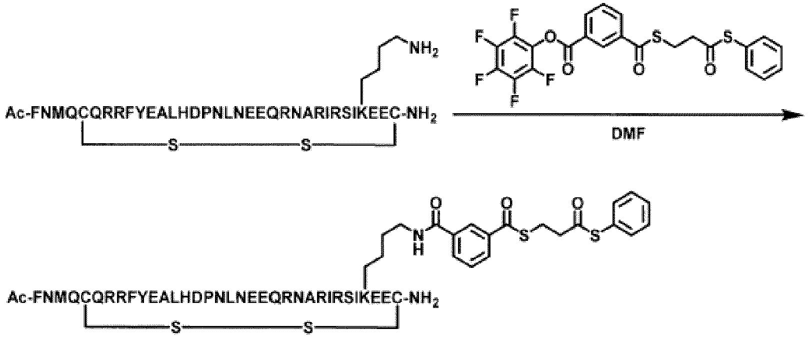

Both of the two aforementioned amino acid sequences are the amino acid sequence of SEQ ID NO: 4.

The linker was attached to Ac-FNMQCQRRFYEALHDPNLNEEQRNARIRSIKEEC-NH₂ (SEQ ID NO: 4) synthesized by the method described in (1-1) (30.0 mg, 7.06 µmol, where two cysteines at positions 5 and 34 each formed an intramolecular disulfide bond) in the same manner as in Example 2 (2-2) to obtain the linked product of thioester linker-thiophenol activator (22.2 mg, 4.82 µmol).
MS(ESI) m/z: z=4 1152.4[M+4H]⁴⁺

### (6-2) Specific Modification of the Anti-HER2 Antibody Trastuzumab and Analysis by ESI-TOFMS

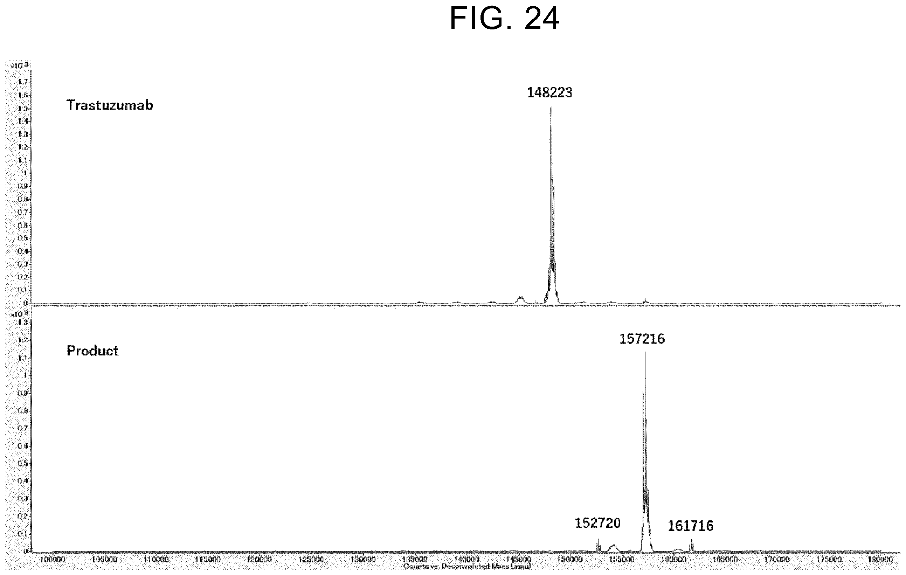

The linked product of peptide linker synthesized in (6-1) was dissolved in dimethyl sulfoxide so as to be 10 mM. 500 µg of the anti-HER2 antibody trastuzumab (Chugai Pharmaceutical Co., Ltd.) was dissolved in 200 µL (20 µM) of 50 mM HEPES buffer (pH 8.2), and 3.38 µL of 10 mM peptide reagent (10 equivalents to the antibody) was added and stirred at room temperature for 1 hour. The reaction solution was replaced with 20 mM ammonium acetate buffer. Mass was measured by ESI-TOFMS. As a result, the raw material trastuzumab had a peak at 148223. A peak 152720 with one binding peptide introduced, a peak 157216 with two binding peptides introduced, and a peak 161716 with three introduced were confirmed (FIG. 24).

### (6-3) Confirmation of Heavy Chain Selectivity of Specific Modified Trastuzumab under by ESI-TOFMS Analysis under Reducing Conditions

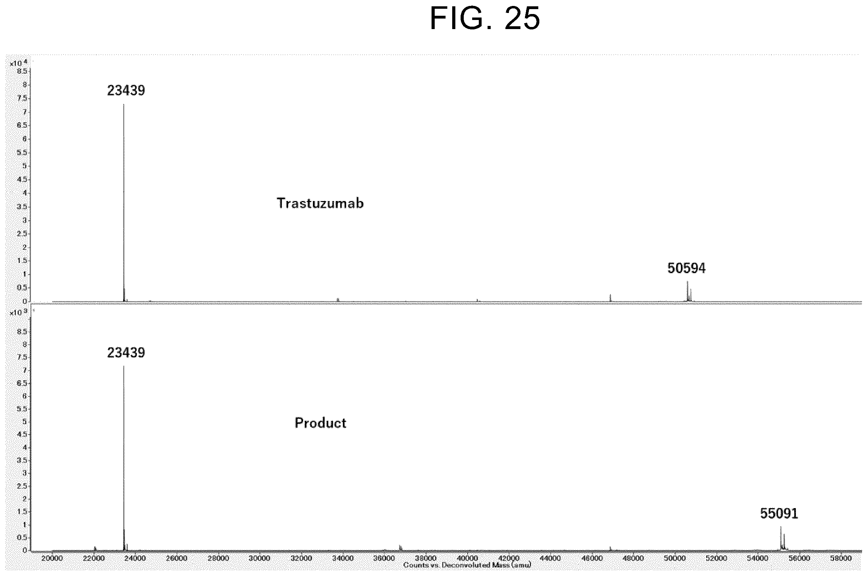

To the antibody-peptide complex produced in (6-2), 2 µL of 100 mM tris(2-carboxyethyl)phosphine hydrochloride (equivalent to the antibody) was added, and the mixture was stirred at room temperature for 15 minutes. The masses were measured by ESI-TOFMS. As a result, the raw material trastuzumab was observed to have a heavy chain peak at 50596 and a light chain peak at 23439. The reactants had a peak at 55091 in which one linker was introduced into the heavy chain and a peak at 23439 in which the light chain was the same as the starting material (FIG. 25).

### (6-4) Confirmation of the peptide/antibody bonding ratio of the specific modified form of trastuzumab by DAR calculator

For MS data analyzed in (6-2), the peptide/antibody bonding ratio was confirmed by DAR calculator (Agilent software). The results are shown in Tables 7. The average peptide/antibody bonding ratio calculated from DAR peak and % Area in Table-7 was 2.0. Therefore, the production of an antibody intermediate represented by the following structural formula (average peptide/antibody bonding ratio: 2.0) was confirmed. 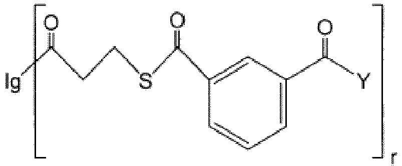 [where Ig represents an immunoglobulin unit (IgG) comprising two heavy chains and two light chains and forms an amide bond with a carbonyl group adjacent to Ig via an amino group in the side chain of the lysine residue present at position 288/290 in the two heavy chains according to EU numbering;
Y represents an affinity peptide represented by the amino acid sequence of SEQ ID NO: 4,
The average ratio r of the amide bonds per two heavy chains is 2.0.]

**Table 7**

| DAR Peak | Observed mass (Da) | Peak Area | %Area |
|---|---|---|---|
| 1 | 152720 | 5.84E+003 | 2.31 |
| 2 | 157216 | 2.41E+005 | 95.07 |
| 3 | 161716 | 6.65E+003 | 2.63 |

### (6-5) Preparation of Thiol Group-Introduced Antibody Derivatives by Cleavage of Thioester Group

The thiol group-introduced antibody derivative was obtained by subjecting the antibody intermediate obtained in (6-4) to the cleavage reaction of the thioester group described in (1-8) to obtain the thiol group-introduced antibody derivative described in (1-8).

### [Example 7: Synthesis of an affinity substance to a soluble protein, a cleavable moiety, and a reactive group (linked product of peptide thioester linker-thiophenol activator), and modification of an anti-antibody HER2 trastuzumab using the compound and analysis thereof]

### (7-1) Linkage between peptide and linker

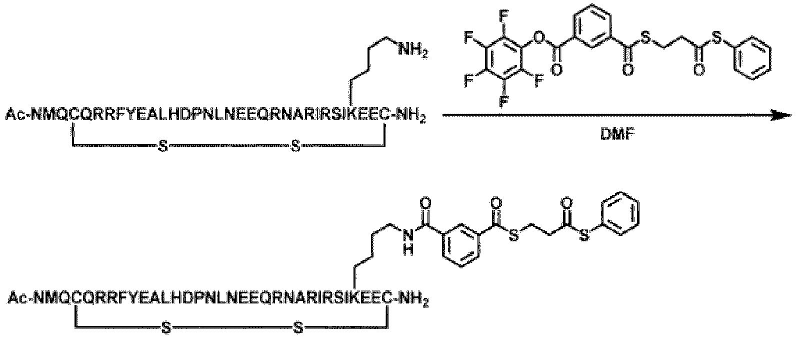

Both of the two aforementioned amino acid sequences are the amino acid sequence of SEQ ID NO: 5.

The linker was attached to Ac-NMQCQRRFYEALHDPNLNEEQRNARIRSIKEEC-NH₂ (SEQ ID NO: 5) synthesized by the method described in (1-1) (30.0 mg, 7.06 µmol, where two cysteines at positions 5 and 34 each form a disulfide bond in the molecule) in the same manner as in Example 2 (2-2) to obtain the linked product of peptide thioester-thiophenol activator (12.0 mg, 2.69 µmol).
MS(ESI) m/z: z=4 1115.8[M+4H]⁴⁺

### (7-2) Specific Modification of the Anti-HER2 Antibody Trastuzumab and Analysis by ESI-TOFMS

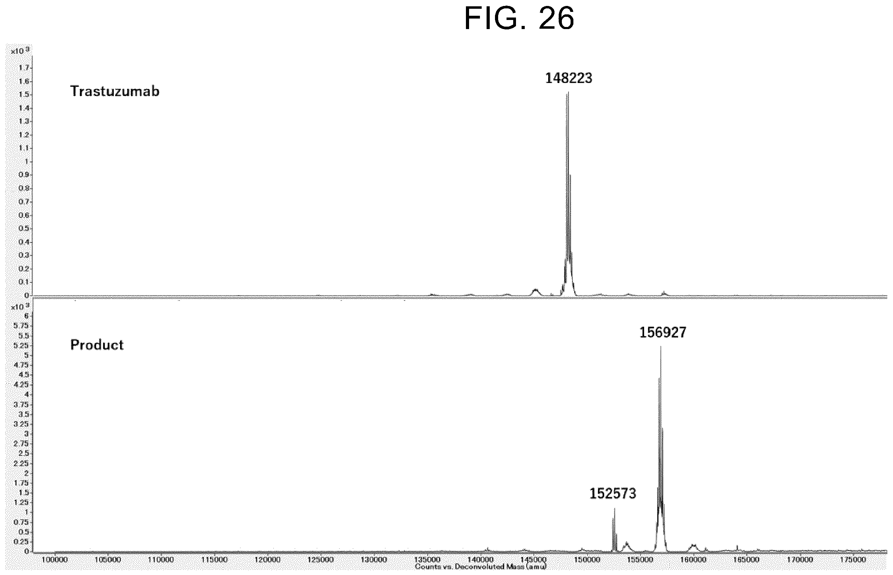

The linked product of peptide linker synthesized in (7-1) was dissolved in dimethyl sulfoxide so as to be 10 mM. 500 µg of the anti-HER2 antibody trastuzumab (Chugai Pharmaceutical Co., Ltd.) was dissolved in 200 µL (20 µM) of 50 mM HEPES buffer (pH 8.2), and 3.38 µL of 10 mM peptide reagent (10 equivalents to the antibody) was added and stirred at room temperature for 1 hour. The reaction solution was replaced with 20 mM ammonium acetate buffer. Mass was measured by ESI-TOFMS. As a result, the raw material trastuzumab peaked at 148223. A peak 152573 in which one binding peptide was introduced and a peak 156927 in which two binding peptides were introduced were confirmed (FIG. 26).

### (7-3) Confirmation of Heavy Chain Selectivity of Specific Modified Trastuzumab under Reducing Conditions by ESI-TOFMS Analysis under Reducing Conditions

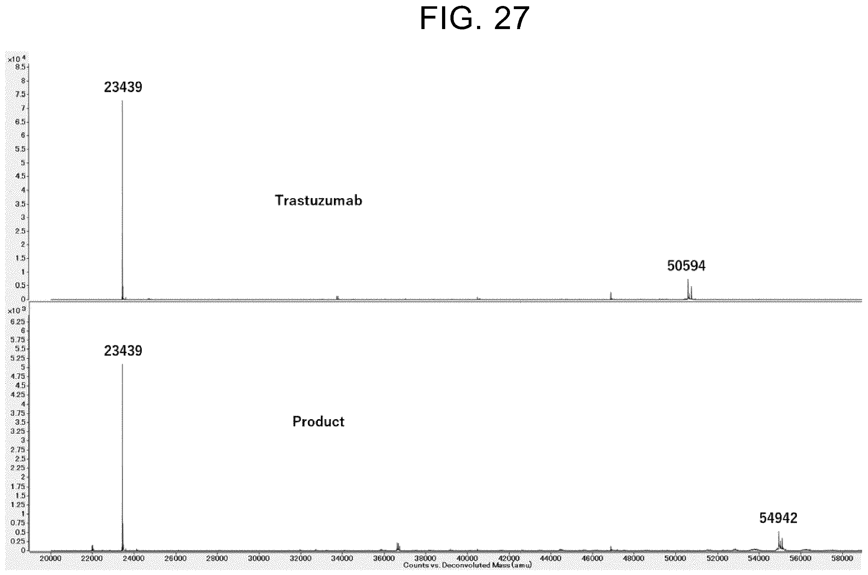

To the antibody-peptide complex produced in (7-2), 2 µL of 100 mM tris(2-carboxyethyl)phosphine hydrochloride (equivalent to the antibody) was added, and the mixture was stirred at room temperature for 15 minutes. The masses were measured by ESI-TOFMS. As a result, the raw material trastuzumab was observed to have a heavy chain peak at 50596 and a light chain peak at 23439. The reactants had a peak at 54942 in which one linker was introduced into the heavy chain and a peak at 23439 in which the light chain was the same as the starting material (FIG. 27).

### (7-4) Confirmation of Peptide/Antibody Bonding Ratio of Specific Modifications of Trastuzumab by DAR calculator

For MS data analyzed in (7-2), the peptide/antibody bonding ratio was confirmed by DAR calculator (Agilent Software). The results are shown in Table 8. The average peptide/antibody bonding ratio calculated from DAR peak and % Area in Table-8 was 2.0. Therefore, the production of an antibody intermediate represented by the following structural formula (average peptide/antibody bonding ratio: 2.0) was confirmed. 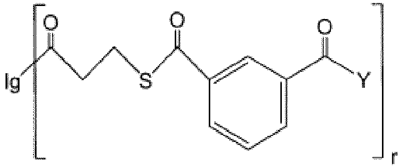 [where Ig represents an immunoglobulin unit (IgG) comprising two heavy chains and two light chains and forms an amide bond with a carbonyl group adjacent to Ig via an amino group in the side chain of the lysine residue present at position 288/290 in the two heavy chains according to EU numbering;
Y represents an affinity peptide represented by the amino acid sequence of SEQ ID NO: 5,
the average ratio r of the amide bonds per two heavy chains is 2.0.]

**Table 8**

| DAR Peak | Observed mass (Da) | Peak Area | %Area |
|---|---|---|---|
| 1 | 152573 | 9.84E+004 | 9.08 |
| 2 | 156927 | 1.11E+006 | 90.92 |

### (7-5) Preparation of Thiol Group-Introduced Antibody Derivatives by Cleavage of Thioester Group

The thiol group-introduced antibody derivative was obtained by subjecting the antibody intermediate obtained in (7-4) to the cleavage reaction of the thioester group described in (1-8) to obtain the thiol group-introduced antibody derivative described in (1-8).

### (7-6) Peptide mapping by trypsinization

The thiol-introduced trastuzumab obtained in (7-5) was subjected to peptide mapping in the following step.

### (7-6-1) Trypsinization of the thiol-introduced trastuzumab

Trypsinization of the thiol-introduced trastuzumab obtained in (7-5) was performed in the same manner as in (1-9-1).

### (7-6-2) LC-MS/MS determination of trastuzumab

LC-MS/MS was measured in the same manner as in (1-9-2) .

### (7-6-3) Analysis of trastuzumab modification sites

Analysis was performed in the same manner as in (1-9-3) .

### (7-6-4) Analysis of trastuzumab modification sites by LC-MS/MS

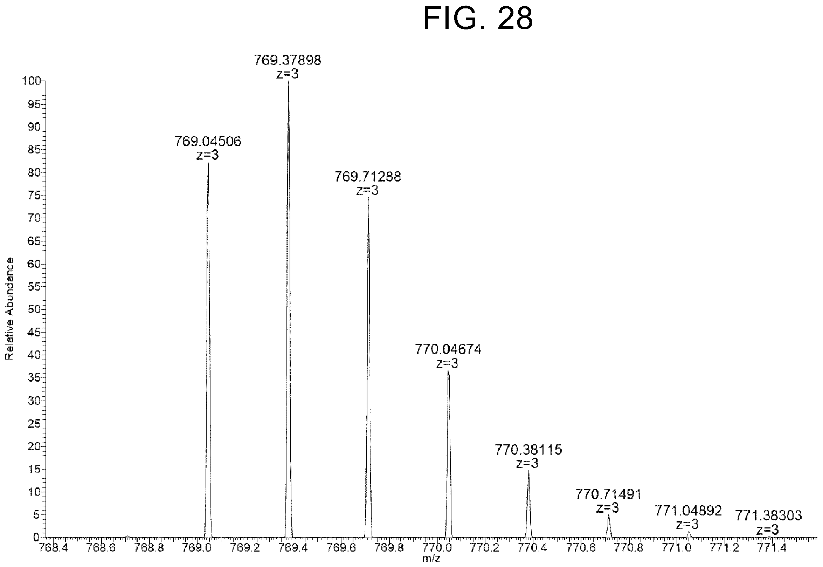

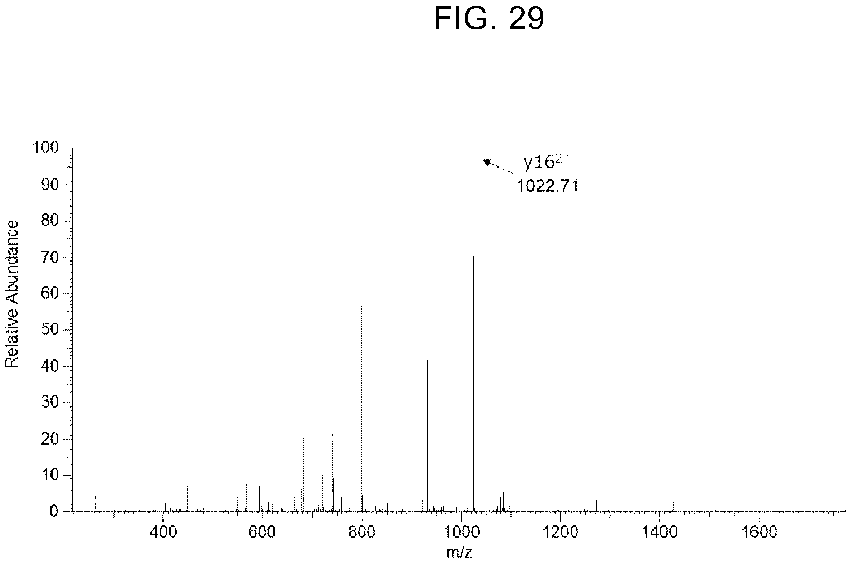

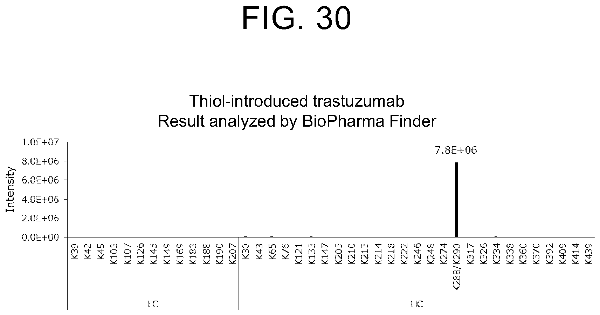

Analysis using LC-MS/MS revealed that MS spectrum (found: m/z 769.04506, theoretical value: 769.04482, trivalent) of the peptide fragment of FNWYVDGVEVHNAKTKPR (SEQ ID NO: 10) which is a peptide consisting of 18 amino acids including a modification site of trastuzumab to lysine residue by tryptic digestion of trastuzumab (thiol introduced product (+145.019 Da) which is carbamidomethylated by iodoacetamide) were observed (FIG. 28), and a product ion of m/z 1022.71 (theoretical value: 1022.51) corresponding to a divalent y16 showing modification of the lysine residue at position 288 or 290 of the heavy chain according to EU numbering was confirmed from CID spectrum (FIG. 29). Analysis with BioPharma Finder also showed that the modification to the lysine residue at position 288 or 290 occurred highly selectively (FIG. 30).

The results showed that the thiol-introduced trastuzumab obtained in the above (7-5) were regioselectively conjugating to Lys288 and Lys290 of the heavy chain according to EU numbering.

### [Example 8: Synthesis of an affinity substance to a soluble protein, a cleavable moiety and a reactive group (linked product of peptide thioester linker-thiophenol activator), and modification of an anti-HER2 antibody using the compound and analysis thereof]

### (8-1) Linkage between peptide and linker

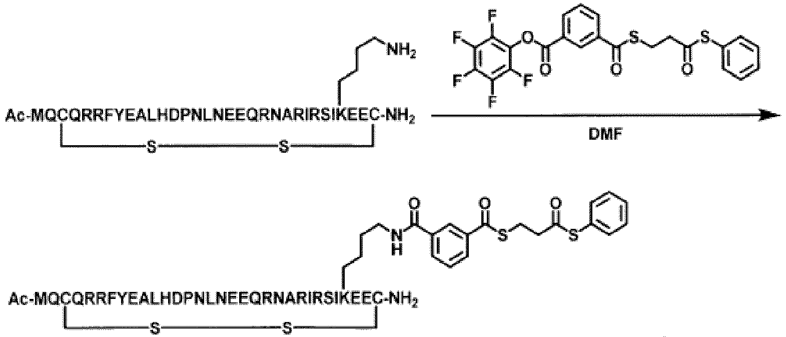

Both of the two aforementioned amino acid sequences are the amino acid sequence of SEQ ID NO: 6.

The linker was attached to Ac-MQCQRRFYEALHDPNLNEEQRNARIRSIKEEC-NH₂ (SEQ ID NO: 6) synthesized by the method described in (1-1) (30.0 mg, 7.06 µmol, where two cysteines at positions 5 and 34 each formed a disulfide bond in the molecule) in the same manner as in Example 2 (2-2) to obtain the linked product of peptide thioester-thiophenol activator (16.8 mg, 3.87 µmol).
MS(ESI) m/z: z=4 1087.3[M+4H]⁴⁺

### (8-2) Specific Modification of the Anti-HER2 Antibody Trastuzumab and Analysis by ESI-TOFMS

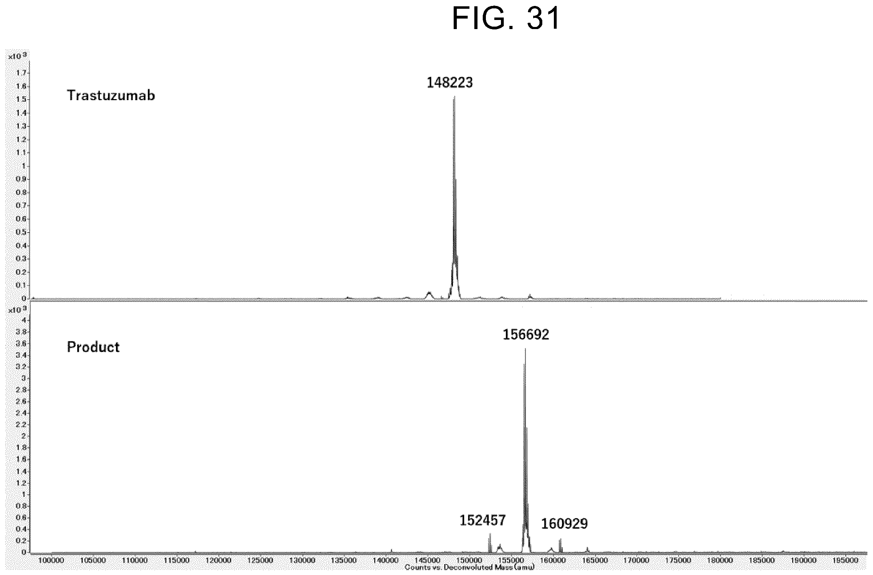

The linked product of peptide linker synthesized in (8-1) was dissolved in dimethyl sulfoxide so as to be 10 mM. 500 µg of the anti-HER2 antibody trastuzumab (Chugai Pharmaceutical Co., Ltd.) was dissolved in 200 µL (20 µM) of 50 mM HEPES buffer (pH 8.2), and 3.38 µL of 10 mM peptide reagent (10 equivalents to the antibody) was added and stirred at room temperature for 1 hour. The reaction solution was replaced with 20 mM ammonium acetate buffer. The masses were measured by ESI-TOFMS. As a result, the raw material trastuzumab had a peak at 148223. A peak 152457 with one binding peptide introduced, a peak 156692 with two binding peptides introduced, and a peak 160929 with three introduced were confirmed (FIG. 31).

### (8-3) Confirmation of Heavy Chain Selectivity of Specific Modified Trastuzumab by ESI-TOFMS Analysis under Reducing Conditions

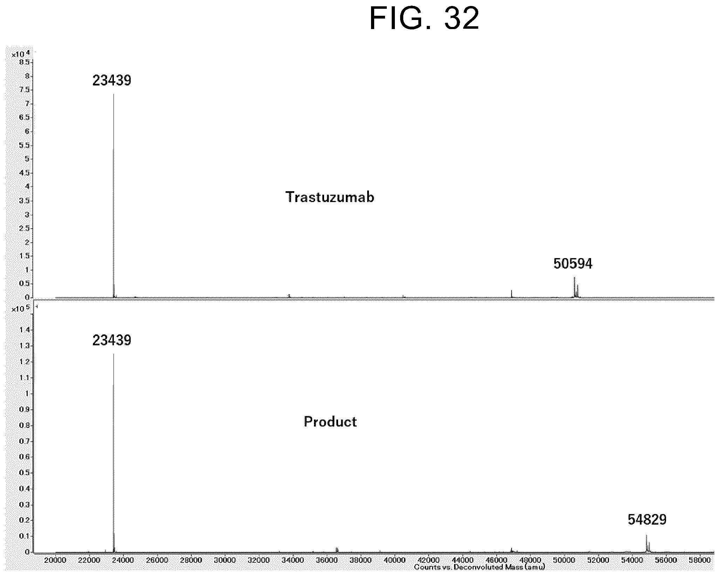

To the antibody-peptide complex produced in (8-2), 2 µL of 100 mM tris(2-carboxyethyl)phosphine hydrochloride (equivalent to the antibody) was added, and the mixture was stirred at room temperature for 15 minutes. The masses were measured by ESI-TOFMS. As a result, the raw material trastuzumab was observed to have a heavy chain peak at 50596 and a light chain peak at 23439. The reactants had a peak at 54829 in which one linker was introduced into the heavy chain and a peak at 23439 in which the light chain was the same as the starting material (FIG. 32).

### (8-4) Confirmation of Peptide/Antibody Bonding Ratio of Specific Modifications of Trastuzumab by DAR calculator

For MS data analyzed in (8-2), the peptide/antibody bonding ratio was confirmed by DAR calculator (Agilent Software). The results are shown in Table 9. The average peptide/antibody bonding ratio calculated from DAR peak and % Area in FIG. 9 was 2.0. Therefore, the production of an antibody intermediate represented by the following structural formula (average peptide/antibody bonding ratio: 2.0) was confirmed. 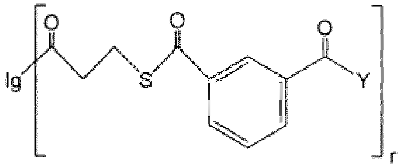 [where Ig represents an immunoglobulin unit (IgG) comprising two heavy chains and two light chains and forms an amide bond with a carbonyl group adjacent to Ig via an amino group in the side chain of the lysine residue present at position 288/290 of the two heavy chains according to EU numbering;
Y represents an affinity peptide represented by the amino acid sequence of SEQ ID NO: 6,
the average ratio r of the amide bonds per two heavy chains is 2.0.]

**Table 9**

| DAR Peak | Observed mass (Da) | Peak Area | %Area |
|---|---|---|---|
| 1 | 152457 | 1.74E+004 | 3.08 |
| 2 | 156692 | 5.22E+005 | 93.53 |
| 3 | 160929 | 1.89E+004 | 3.39 |

### (8-5) Preparation of Thiol Group-Introduced Antibody Derivatives by Cleavage of Thioester Group

The thiol group-introduced antibody derivative was obtained by subjecting the antibody intermediate obtained in (8-4) to the cleavage reaction of the thioester group described in (1-8) to obtain the thiol group-introduced antibody derivative described in (1-8).

### [Example 9: Synthesis of an affinity substance to a soluble protein, a cleavable moiety and a reactive group (linked product of peptide thioester linker-thiophenol activator), and modification of an anti-HER2 antibody using the compound and analysis thereof]

### (9-1) Linkage between peptide and linker

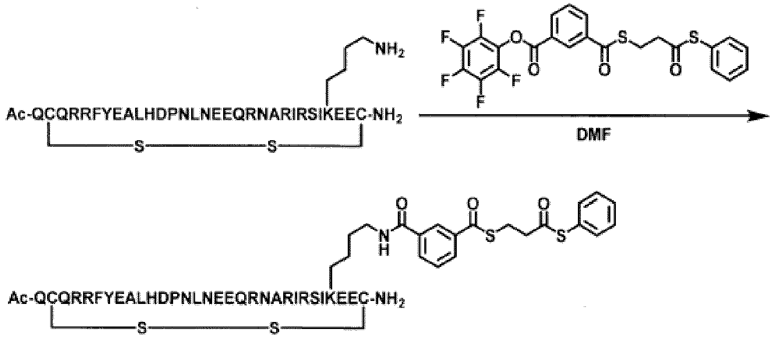

Both of the above two amino acid sequences are the amino acid sequence of SEQ ID NO: 7.

The linker was attached to Ac-QCQRRFYEALHDPNLNEEQRNARIRSIKEEC-NH₂ (SEQ ID NO: 7) synthesized by the method described in (9-1) (30.0 mg, 7.06 µmol, where two cysteines at positions 5 and 34 each formed an intramolecular disulfide bond) in the same manner as in Example 2 (2-2) to obtain the linked product of thioester linker-thiophenol activator (14.1 mg, 3.35 µmol).
MS(ESI) m/z: z=4 1054.4[M+4H]⁴⁺

### (9-2) Specific Modification of the Anti-HER2 Antibody Trastuzumab and Analysis by ESI-TOFMS

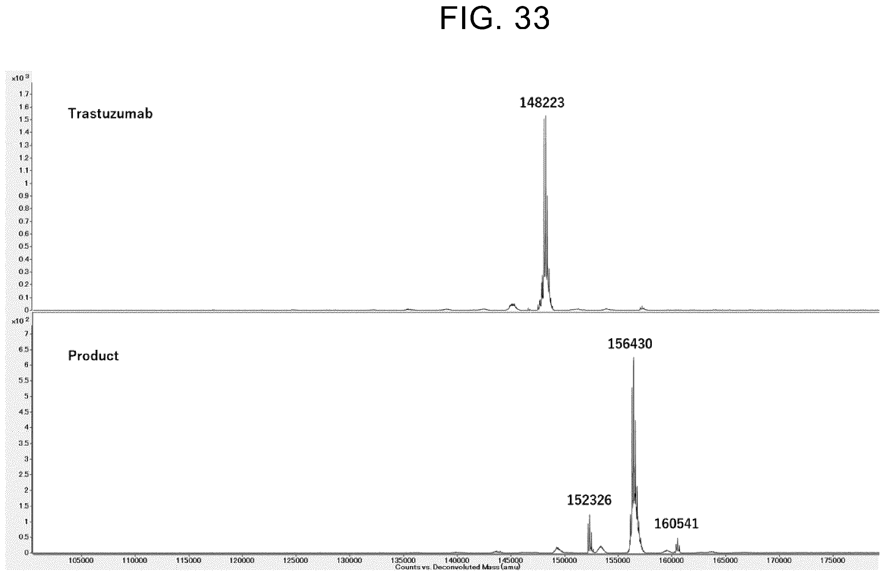

The linked product of peptide linker synthesized in (9-1) was dissolved in dimethyl sulfoxide so as to be 10 mM. 500 µg of the anti-HER2 antibody trastuzumab (Chugai Pharmaceutical Co., Ltd.) was dissolved in 200 µL (20 µM) of 50 mM HEPES buffer (pH 8.2), and 3.38 µL of 10 mM peptide reagent (10 equivalents to the antibody) was added and stirred at room temperature for 1 hour. The reaction solution was replaced with 20 mM ammonium acetate buffer. The raw material trastuzumab peaked at 148223 as measured by ESI-TOFMS. A peak of 152236 in which one binding peptide was introduced, 156430 in which two binding peptides were introduced, and 160541 in which three binding peptides were introduced were confirmed (FIG. 33).

### (9-3) Confirmation of Heavy Chain Selectivity of Specific Modified Trastuzumab by ESI-TOFMS Analysis under Reducing Conditions

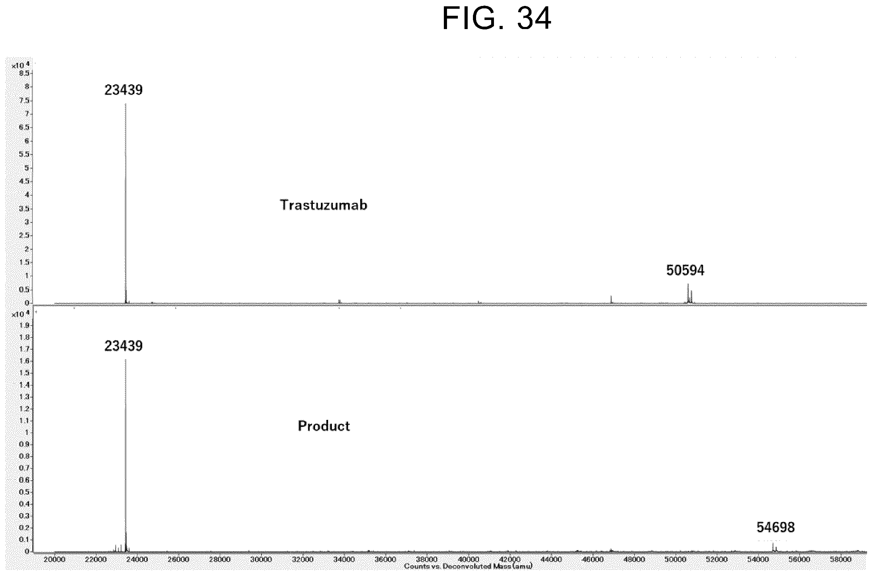

To the antibody-peptide complex produced in (9-2), 2 µL of 100 mM tris(2-carboxyethyl)phosphine hydrochloride (equivalent to the antibody) was added, and the mixture was stirred at room temperature for 15 minutes. The masses were measured by ESI-TOFMS. The raw material trastuzumab was observed to have a heavy chain peak at 50596 and a light chain peak at 23439. The reactants had a peak at 54698 in which one linker was introduced into the heavy chain and a peak at 23439 in which the light chain was the same as the starting material (FIG. 34).

### (9-4) Confirmation of Peptide/Antibody Bonding Ratio of Specific Modifications of Trastuzumab by DAR calculator

For MS data analyzed in (9-2), the peptide/antibody bonding ratio was confirmed by DAR calculator (Agilent Software). The results are shown in Table 10. The average peptide/antibody bonding ratio calculated from DAR peak and % Area in Table-10 was 2.0. Therefore, the production of an antibody intermediate represented by the following structural formula (average peptide/antibody bonding ratio: 2.0) was confirmed. 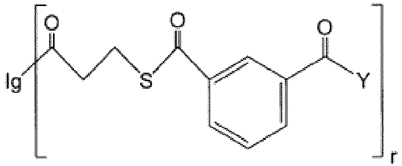 [where Ig represents an immunoglobulin unit (IgG) comprising two heavy chains and two light chains and forms an amide bond with a carbonyl group adjacent to Ig via an amino group in the side chain of the lysine residue present at position 288/290 in the two heavy chains according to EU numbering;
Y represents an affinity peptide represented by the amino acid sequence of SEQ ID NO: 7,
The average ratio r of the amide bonds per two heavy chains is 2.0.]

**Table 10**

| DAR Peak | Observed mass (Da) | Peak Area | %Area |
|---|---|---|---|
| 1 | 152236 | 1.24E+004 | 7.09 |
| 2 | 156430 | 1.57E+005 | 89.82 |
| 3 | 160541 | 5.41E+003 | 3.09 |

### (9-5) Preparation of Thiol Group-Introduced Antibody Derivatives by Cleavage of Thioester Group

The thiol group-introduced antibody derivative was obtained by subjecting the antibody intermediate obtained in (9-4) to the cleavage reaction of the thioester group described in (1-8) to obtain the thiol group-introduced antibody derivative described in (1-8).

### (Summary of Examples 1 to 9)

The relationship between the linker length and the peptide/antibody bonding ratio (PAR) in the compounds of the Examples was examined. Compounds of formula (I) in which the number of atoms constituting the main chain linking the reactive moiety with the lysine residue at position 288/290 of the heavy chain in the immunoglobulin unit [X (leaving group)-C=O] and the binding moiety with the affinity peptide [O=C-Y (affinity peptide)] is 7 to 9 (in the compound of formula (I), the total number of atoms constituting the main chain in the first linker and atoms constituting the main chain in the second linker correspond to the number of atoms of 5 to 7) showed the average PAR in the desired range (1.5 to 2.0).

**Table 11. Relationship between linker length and bonding ratio in Example compounds (1)**

| Example | Chemical structure | Linker length ¹⁾ | Bonding ratio ²⁾ |
|---|---|---|---|
| 1 | 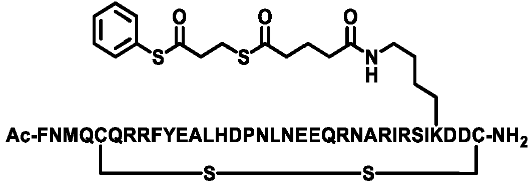 | 7 | 2.0 |
| | The above amino acid sequence: SEQ ID NO: 2 | | |
| 2 | 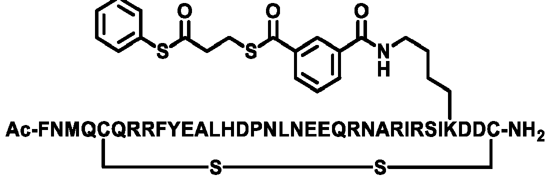 | 7 | 2.0 |
| | The above amino acid sequence: SEQ ID NO: 2 | | |
| 3 | 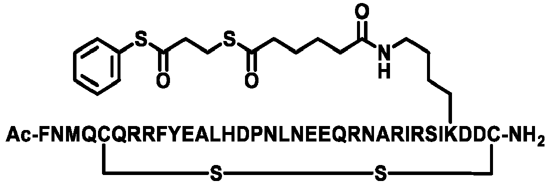 | 8 | 1.9 |
| | The above amino acid sequence: SEQ ID NO: 2 | | |
| 4 | 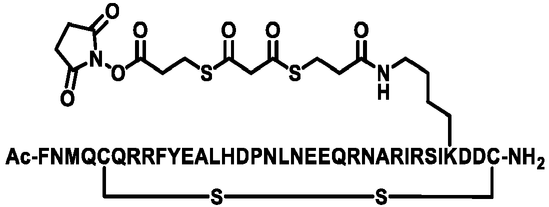 | 9 | 1. 8 |
| | The above amino acid sequence: SEQ ID NO: 2 | | |
| 5 | 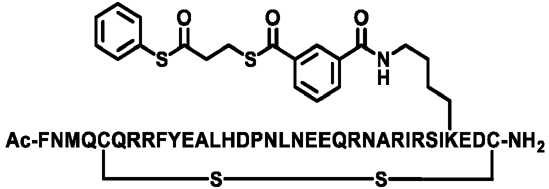 | 7 | 2.0 |
| | The above amino acid sequence: SEQ ID NO: 3 | | |

| | | | |
|---|---|---|---|
| 1) Linker length: the number of atoms constituting the main chain connecting the reactive moiety with the lysine residue at position 288/290 of the heavy chain in the immunoglobulin unit [X (leaving group)-C=O] and the binding moiety with the affinity peptide [O=C-Y (affinity peptide)] 2) Bonding ratio: bonding ratio of peptide to antibody (peptide/antibody) | | | |

(The same shall apply hereinafter)

**Table 12. Relationship between linker length and bonding ratio in Example compounds (2)**

| Example | Chemical structure | Linker length ¹⁾ | Bonding ratio ²⁾ |
|---|---|---|---|
| 6 | 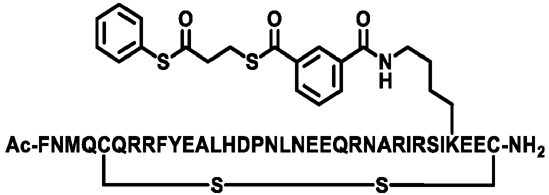 | 7 | 2.0 |
| | The above amino acid sequence: SEQ ID NO: 4 | | |
| 7 | 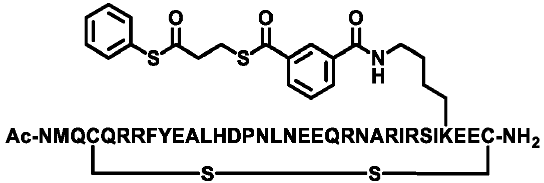 | 7 | 2.0 |
| | The above amino acid sequence: SEQ ID NO: 5 | | |
| 8 | 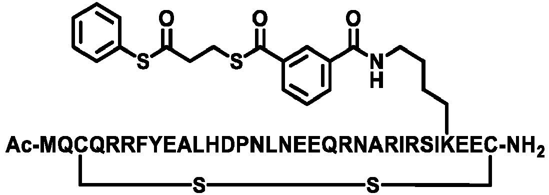 | 7 | 2.0 |
| | The above amino acid sequence: SEQ ID NO: 6 | | |
| 9 | 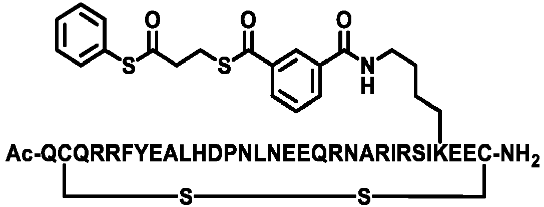 | 7 | 2.0 |
| | The above amino acid sequence: SEQ ID NO: 7 | | |

### [Reference Examples 1 to 5]

The following linked product of peptide thioester-NHS activator or linked product of peptide thioester-thiophenol activator was synthesized with reference to the above embodiment, and the anti-HER2 antibody trastuzumab was analyzed by specific modification and ESI-TOFMS. In addition, as in the examples, the peptide/antibody bonding ratio (PAR) of a specific modification of trastuzumab was confirmed by DAR calculator. As a result, a compound having a linker length of 6 or less or 11 or more atoms (in the compound represented by the formula (I), the total number of atoms constituting the main chain in the first linker and atoms constituting the main chain in the second linker correspond to the number of atoms of less than 4, or 9 or more atoms) exhibited average PAR of 0.5 or less.

**Table 13. Relationship between linker length and bonding ratio in reference example compounds**

| Ref. example | Chemical structure | Linker length ¹⁾ | Bonding ratio ²⁾ |
|---|---|---|---|
| 1 | 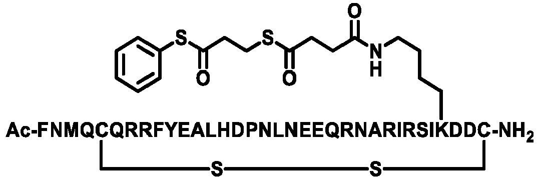 | 6 | 0.5 or less |
| | The above amino acid sequence: SEQ ID NO: 2 | | |
| 2 | 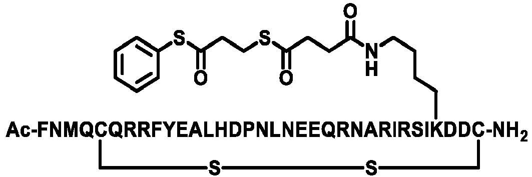 | 6 | |
| | The above amino acid sequence: SEQ ID NO: 2 | | |
| 3 | 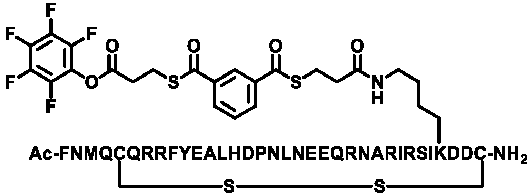 | 11 | |
| | The above amino acid sequence: SEQ ID NO: 2 | | |
| 4 | 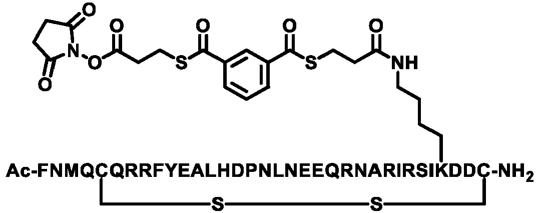 | 11 | |
| | The above amino acid sequence: SEQ ID NO: 2 | | |
| 5 | 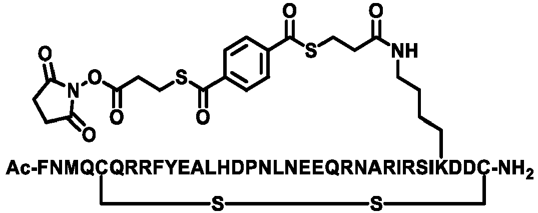 | 12 | |
| | The above amino acid sequence: SEQ ID NO: 2 | | |

### [Example 10: Regioselective Modification of Different Multiple Target Regions with IgG1 Fc Affinity Peptide Reagents and Synthesis of Antibody-Drug Conjugates]

### (10-1) Preparation of Thiol-group-Introduced Antibody Derivatives by Regioselective Modification of the Anti-HER2 Antibody Trastuzumab and Subsequent Cleavage of the Thioester Group

The peptide reagent described in the previous report (WO2019/240287A1) was dissolved in dimethylformamide so as to be 10 mM. 500 µg of the anti-HER2 antibody trastuzumab (Chugai Pharmaceutical Co., Ltd.) was dissolved in 200 µL (20 µM) of 20 mM sodium acetate buffer (pH 5.5), and 3.38 µL of 10 mM peptide reagent (10 equivalents to the antibody) was added and stirred at room temperature for 1 hour. Subsequently, hydroxylamine solutions were added according to the previous report (WO2019/240287A1) and allowed to stand at room temperature for 1 hour. The resulting thiol-introduced antibody was measured by ESI-TOFMS. A peak at 148760 at which cleavage by hydroxylamine proceeds was confirmed. Given that the peptide reagents described below are reagents that allow modification of the lysine residue at positions 246/248 of IgG heavy chain, it is believed that the lysine residue at positions 246/248 of IgG heavy chain was modified with the peptide reagents described below. 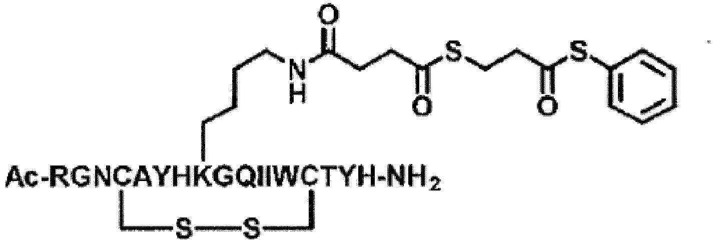 The aforementioned amino acid sequence is the amino acid sequence of SEQ ID NO: 11.

### (10-2) Regioselective Modification of Different Multiple Target Regions of the Anti-HER2 Antibody Trastuzumab

Thiol-transfected antibody solutions synthesized in (10-1) were replaced with 50 mM HEPES buffer (pH 8.2). To this solution, 10 equivalents of a dimethylformamide solution of the linked product of peptide linker synthesized in (3-2) was added to the antibody, and the mixture was stirred at room temperature for 1 hour. The reaction solution was replaced with 20 mM ammonium acetate buffer. Mass of the obtained antibody was measured by ESI-TOFMS. As a result, a peak 157531 in which two binding peptides were introduced to the thiol-introduced antibody synthesized in (3-2) was confirmed.

### (10-3) Preparation of Thiol Group-Introduced Antibody Derivatives by Cleavage of Thioester Group

Hydroxylamine solutions were added to the antibody-intermediates obtained in (10-2) in accordance with the previous report (WO2019/240287A1) and allowed to stand at room temperature for 1 hour. After 2 hours, the compound was replaced with 20 mM PBS buffer, 10 mM EDTA (pH 7.4) to obtain a thiol-group-introduced antibody derivative. The masses were measured by ESI-TOFMS. The peak was confirmed at 148760 at which the cleavage proceeded. Therefore, it was confirmed that the obtained thiol group-introduced antibody derivative had the following structure. 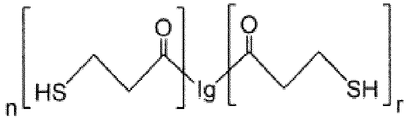 [Where Ig represents an immunoglobulin unit (IgG) comprising two heavy chains and two light chains, and forms an amide bond with the carbonyl group adjacent to Ig via the amino group in the side chain of the lysine residue present at position 288/290 in the two heavy chains according to EU numbering, and also forms an amide bond with the carbonyl group adjacent to Ig via the amino group in the side chain of the lysine residue present at position 246/248 in the two heavy chains according to EU numbering. The average ratios r (288/290) and n (246/248) of the amide bonds per two heavy chains as assessed were 1.9 and 2.0, respectively.]

### (10-4) Preparation of Thiol-introduced Antibody Derivatives by Cleavage of Drug mimic Thioester Group

To a solution (20 µM) of the thiol-group-introduced antibody obtained in (10-3) in buffer (pH 7.4, PBS buffer) was added 10 equivalents of a DMF solution (1.25 mM) of a MC-VC-PAB-MMAE of (Org. Process Res. Dev. 2019, 23 ,12, 2647-2654), and the mixture was allowed to stand at room temperature for 2 hours, followed by purification using NAP-5 Columns (GE Healthcare Co., Ltd.) to obtain a ADC. The masses were measured by ESI-TOFMS and peaked at 154029 where four MC-VC-PAB-MMAE were introduced.

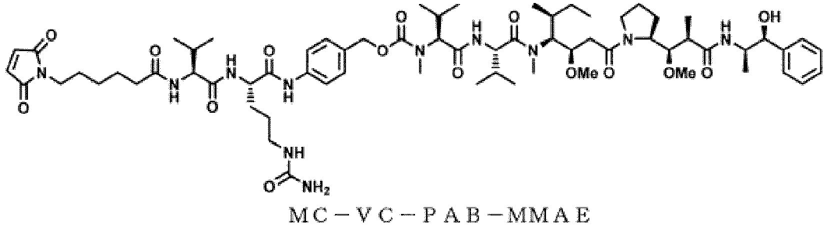

[Sequence Listing]

## Claims

1. A compound or salt thereof represented by the following formula (I): 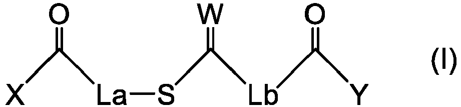 wherein
X indicates a leaving group,
Y indicates an affinity peptide having a binding region to a CH2 domain in an immunoglobulin unit containing two heavy chains and two light chains,
O indicates an oxygen atom,
S indicates a sulfur atom,
W indicates an oxygen atom or a sulfur atom,
La indicates a first linker,
Lb indicates a second linker, and
the total number of atoms constituting a main chain in the first linker and atoms constituting a main chain in the second linker are 5 to 7.

2. The compound or salt thereof of claim 1, wherein the leaving group is selected from the following:
(A) R-S wherein R indicates a hydrogen atom, a monovalent hydrocarbon group which may have a substituent, or a monovalent heterocyclic group which may have a substituent, and S indicates a sulfur atom;
(B) R-O wherein R indicates a hydrogen atom, a monovalent hydrocarbon group which may have a substituent, or a monovalent heterocyclic group which may have a substituent, and O indicates an oxygen atom;
(C) R_{A}-(R_{B}-)N wherein R_{A} and R_{B} each independently indicate a hydrogen atoms, a monovalent hydrocarbon group which may have a substituent, or a monovalent heterocyclic group which may have a substituent, and N indicates a nitrogen atom; or
(d) a halogen atom.

3. The compound or salt thereof of claim 1 or 2, wherein the immunoglobulin unit is a human immunoglobulin unit.

4. The compound or salt thereof of any one of claims 1 to 3, wherein the immunoglobulin unit is human IgG.

5. The compound or salt thereof of any one of claims 1 to 4, wherein the affinity peptide contains a lysine residue and forms an amide bond with a carbonyl group (C=O) adjacent to Y via an amino group in a side chain of the lysine residue.

6. The compound or salt thereof of claims 1 to 5, wherein the affinity peptide is the following:
(A) Affinity peptide comprising the amino acid sequence of CQRRFYEALHDPNLNEEQRNARIRSIKDDC (SEQ ID NO: 1);
(B) An affinity peptide comprising an amino acid sequence comprising a mutation of 1 to 5 amino acid residues which is selected from the group consisting of substitution, insertion, deletion and addition of amino acid residues in the amino acid sequence of CQRRFYEALHDPNLNEEQRNARIRSIKDDC (SEQ ID NO: 1),
wherein the lysine residue at the position 27 and the two cysteine residues at the positions 1 and 30 are maintained.

7. The compound or salt thereof of claim 6, wherein the affinity peptide (B) is selected from the group consisting of the following:
(A) Affinity peptide comprising the amino acid sequence of FNMQCQRRFYEALHDPNLNEEQRNARIRSIKDDC (SEQ ID NO: 2);
(B) Affinity peptide comprising the amino acid sequence of FNMQCQRRFYEALHDPNLNEEQRNARIRSIKEDC (SEQ ID NO: 3);
(C) Affinity peptide comprising the amino acid sequence of FNMQCQRRFYEALHDPNLNEEQRNARIRSIKEEC (SEQ ID NO: 4);
(D) Affinity peptide comprising the amino acid sequence of NMQCQRRFYEALHDPNLNEEQRNARIRSIKEEC (SEQ ID NO: 5);
(E) Affinity peptide comprising the amino acid sequence of MQCQRRFYEALHDPNLNEEQRNARIRSIKEEC (SEQ ID NO: 6); and
(f) Affinity peptide comprising the amino acid sequence of QCQRRFYEALHDPNLNEEQRNARIRSIKEEC (SEQ ID NO: 7).

8. The compound or salt thereof of claim 6 or 7, wherein the N-terminal and C-terminal amino acid residues in the affinity peptide may be protected, and the two thiol groups in side chains of the two cysteine residues (C) in the affinity peptide may be linked by a disulfide bond, or via a linker.

9. The compound or salt thereof of any one of claims 1 to 8, wherein the compound represented by the above formula (I) is represented by the following formula (I'): 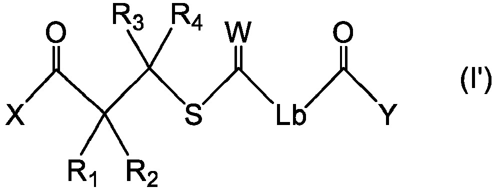 wherein
X, Y, O, S and W are the same as those in the above formula (I),
Lb indicates a second linker having 3 to 5 atoms constituting a main chain, and
R₁, R₂, R₃ and R₄ each independently indicate a hydrogen atom or a substituent.

10. The compound or salt thereof of claim 9, wherein the compound represented by the above formula (I') is represented by the following formula (I''): 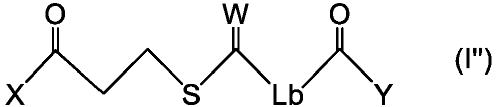 wherein
X, Y, O, S and W are the same as those in the above formula (I), and
Lb is the same as that of the above formula (I').

11. A reagent for derivatizing an antibody, which comprises a compound or salt thereof represented by the following formula (I): 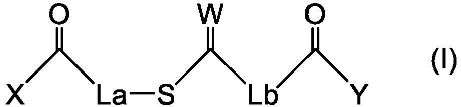 wherein
X indicates a leaving group,
Y indicates an affinity peptide having a binding region to a CH2 domain in an immunoglobulin unit containing two heavy chains and two light chains,
O indicates an oxygen atom,
S indicates a sulfur atom,
W indicates an oxygen atom or a sulfur atom,
La indicates a first linker,
Lb indicates a second linker, and
the total number of atoms constituting a main chain in the first linker and atoms constituting a main chain in the second linker are 5 to 7.

12. The reagent of claim 11, wherein the compound represented by the above formula (I) is represented by the following formula (I'): 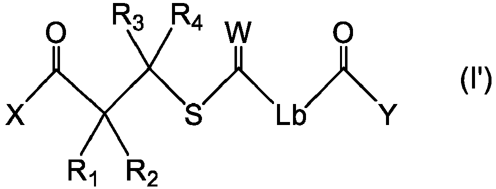 wherein
X, Y, O, S and W are the same as those in the above formula (I),
Lb indicates a second linker having 3 to 5 atoms constituting a main chain, and
R₁, R₂, R₃ and R₄ each independently indicate a hydrogen atom or a substituent.

13. The reagent according to claim 12, wherein the compound represented by the above formula (I') is represented by the following formula (I"): 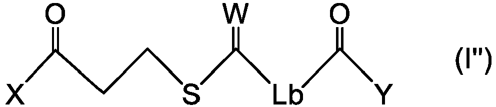 wherein
X, Y, O, S and W are the same as those in the above formula (I), and
Lb is the same as that of the above formula (I').

14. An antibody intermediate or salt thereof comprising a structural unit represented by the following formula (II): 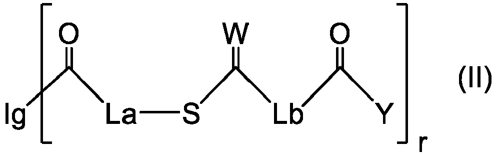 wherein
Ig indicates an immunoglobulin unit containing two heavy chains and two light chains, and forms an amide bond with the carbonyl group adjacent to Ig via an amino group in a side chain of a lysine residue present at the position 288/290 in the two heavy chains according to Eu numbering,
Y indicates an affinity peptide having a binding region to a CH2 domain in the immunoglobulin unit,
O indicates an oxygen atom,
S indicates a sulfur atom,
W indicates an oxygen atom or a sulfur atom,
La indicates a first linker,
Lb indicates a second linker,
the total number of atoms constituting a main chain in the first linker and atoms constituting a main chain in the second linker are 5 to 7, and
the average ratio r of the amide bond per the two heavy chains is 1.5 to 2.5.

15. The antibody intermediate or salt thereof of claim 14, wherein the antibody intermediate is a human antibody intermediate.

16. The antibody intermediate or salt thereof of claim 14 or 15, wherein the antibody intermediate is a human IgG intermediate.

17. The antibody intermediate or salt thereof of any one of claims 14 to 16, wherein the structural unit represented by the above formula (II) is represented by the following formula (II'): 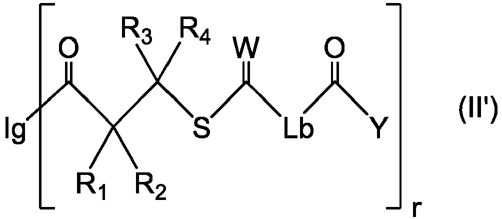 wherein
Ig, Y, O, S, W and r are the same as those of the above formula (II),
Lb indicates a second linker having 3 to 5 atoms constituting a main chain, and
R₁, R₂, R₃ and R₄ each independently indicate a hydrogen atom or a substituent.

18. The antibody intermediate or salt thereof of claim 17, wherein the structural unit represented by the above formula (II') is represented by the following formula (II") : 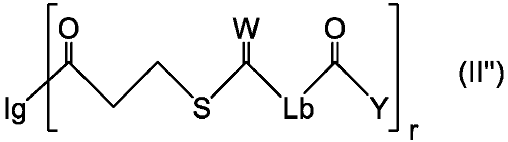 wherein
Ig, Y, O, S, W and r are the same as those of the above formula (II), and
Lb is the same as that of the above formula (II').

19. A thiol group-introduced antibody derivative or salt thereof, which comprises a structural unit represented by the following formula (III): 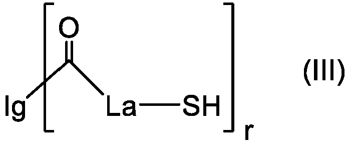 wherein
Ig indicates an immunoglobulin unit containing two heavy chains and two light chains, and forms an amide bond with the carbonyl group adjacent to Ig via an amino group in a side chain of a lysine residue present at the position 288/290 in the two heavy chains according to Eu numbering,
O indicates an oxygen atom,
SH indicates a thiol group,
La indicates a first linker,
the number of atoms constituting the main chain in the first linker is 2 to 4, and
the average ratio r of the amide bonds per the two heavy chains is 1.5 to 2.5.

20. The thiol group-introduced antibody derivative or salt thereof of claim 19, wherein a specific amino acid residue other than the lysine residue present at the position 288/290 in the two heavy chains is further modified.

21. The thiol group-introduced antibody derivative or salt thereof of claim 20, wherein the specific amino acid residue is a lysine residue present at the position 246/248 in the two heavy chains.

22. The thiol group-introduced antibody derivative or salt thereof of any one of claims 19 to 21, wherein the structural unit represented by the above formula (III) is the following formula (III'): 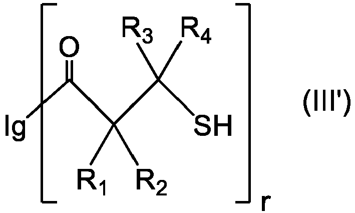 wherein
Ig, O, SH and r are the same as those in the above formula (III), and
R₁, R₂, R₃ and R₄ each independently indicate a hydrogen atom or a substituent.

23. The thiol group-introduced antibody derivative or salt thereof of claim 22, wherein the structural unit represented by the above formula (III') is the following formula (III"): 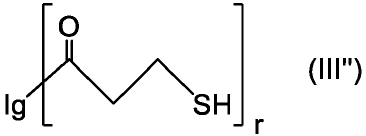 wherein
Ig, O, SH and r are the same as those in the above formula (III).

24. A conjugate of an antibody and a functional substance or a salt thereof, which comprises a structural unit represented by the following formula (IV): 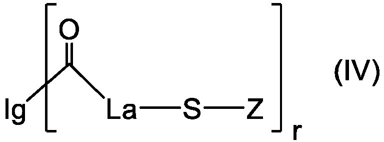 wherein
Ig indicates an immunoglobulin unit containing two heavy chains and two light chains, and forms an amide bond with the carbonyl group adjacent to Ig via an amino group in a side chain of a lysine residue present at the position 288/290 in the two heavy chains according to Eu numbering,
O indicates an oxygen atom,
S indicates a sulfur atom,
Z indicates a functional substance,
La indicates a first linker,
the number of atoms constituting a main chain in the first linker is 2 to 4, and
the average ratio r of the amide bonds per the two heavy chains is 1.5 to 2.5.

25. The conjugate or salt thereof of claim 24, wherein a specific amino acid residue other than the lysine residue present at the position 288/290 in the two heavy chains is further modified.

26. The conjugate or salt thereof of claim 25, wherein the specific amino acid residue is a lysine residue present at position 246/248 in two heavy chains.

27. The conjugate or salt thereof of any one of claims 24 to 26, wherein the structural unit represented by the above formula (IV) is represented by the following formula (IV'): 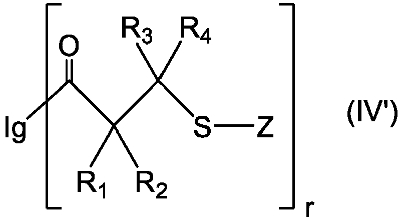 wherein
Ig, O, S, Z and r are the same as those of the above formula (IV), and
R₁, R₂, R₃ and R₄ each independently indicate a hydrogen atom or a substituent.

28. The conjugate or salt thereof of claim 27, wherein the structural unit represented by the above formula (IV') is represented by the following formula (IV"): 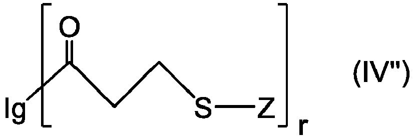 wherein
Ig, O, S, Z and r are the same as those of the above formula (IV).

29. A compound or salt thereof represented by the following formula (V): 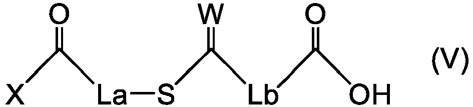 wherein
X indicates a leaving group,
O indicates an oxygen atom,
OH indicates a hydroxy group,
S indicates a sulfur atom,
W indicates an oxygen atom or a sulfur atom,
La indicates a first linker,
Lb indicates a second linker,
the total number of atoms constituting a main chain in the first linker and atoms constituting a main chain in the second linker are 5 to 7.

30. The compound or salt thereof of claim 29, wherein the compound represented by the above formula (V) is represented by the following formula (V'): 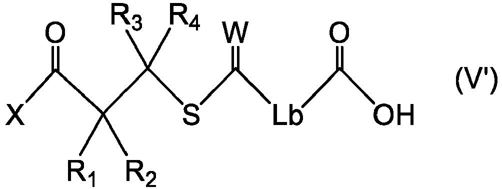 wherein
X, O, OH, S and W are the same as those in the above formula (V),
Lb indicates a second linker having 3 to 5 atoms constituting a main chain, and
R₁, R₂, R₃ and R₄ each independently indicate a hydrogen atom or a substituent.

31. The compound or salt thereof of claim 30, wherein the compound represented by the above formula (V') is represented by the following formula (V''): 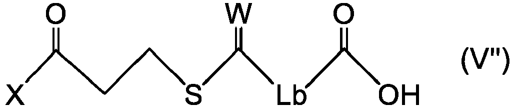 wherein
X, O, OH, S and W are the same as those in the above formula (V), and
Lb is the same as that of the above formula (V').

32. The compound represented by the above formula (V") is the following formula (V"-1) or (V"-2): 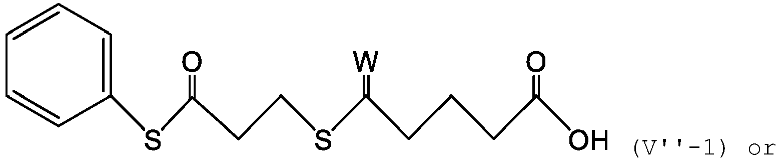 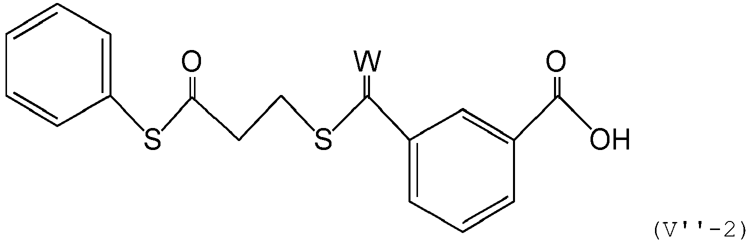 wherein
O, OH, S and W are the same as those in the above formula (V).

33. A compound or salt thereof represented by the following formula (VI): 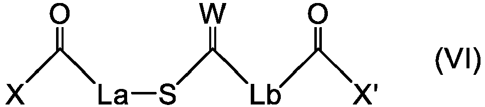 wherein
X indicates a leaving group,
X' indicates a leaving group having a leaving ability which is higher than that of the leaving group X.
O indicates an oxygen atom,
S indicates a sulfur atom,
W indicates an oxygen atom or a sulfur atom,
La indicates a first linker,
Lb indicates a second linker, and
the total number of atoms constituting a main chain in the first linker and the number of atoms constituting a main chain in the second linker are 5 to 7.

34. The compound or salt thereof of claim 33, wherein the leaving group having a leaving ability which is higher than that of the leaving group X is a pentafluorophenyloxy group, a tetrafluorophenyloxy group, a paranitrophenyloxy group, or an N-succinimidyloxy group.

35. The compound or salt thereof of claim 33 or 34, wherein the compound represented by the above formula (VI) is represented by the following formula (VI'): 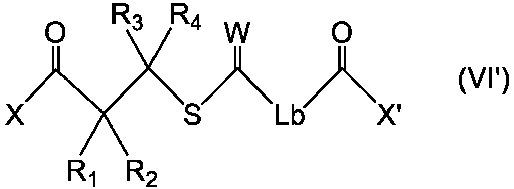 wherein
X, X', O, S and W are the same as those in the above formula (VI),
Lb indicates a second linker having 3 to 5 atoms constituting a main chain, and
R₁, R₂, R₃ and R₄ each independently indicate a hydrogen atom or a substituent.

36. The compound or salt thereof of claim 35, wherein the compound represented by the above formula (VI') is represented by the following formula (VI''): 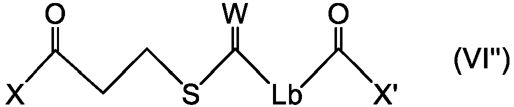 wherein
X, X', O, S and W are the same as those in the above formula (VI), and
Lb is the same as that of the above formula (VI').

37. The compound or salt thereof of claim 36, wherein the compound represented by the above formula (VI'') is represented by the following formula (VI''-1) or (VI''-2): 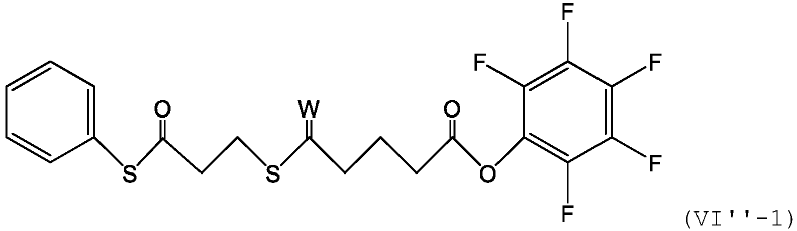 or 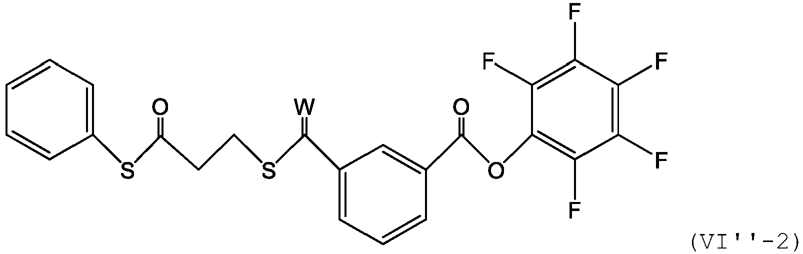 wherein
O, S and W are the same as those of the above formula (VI), and
F is a fluorine atom.

38. A method for producing an antibody intermediate or salt thereof, which comprises reacting a compound or salt thereof represented by the following formula (I): 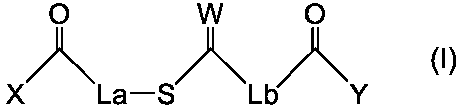 wherein
X indicates a leaving group,
Y indicates an affinity peptide having a binding region to a CH2 domain in an immunoglobulin unit containing two heavy chains and two light chains,
O indicates an oxygen atom,
S indicates a sulfur atom,
W indicates an oxygen atom or a sulfur atom,
La indicates a first linker,
Lb indicates a second linker, and
the total number of atoms constituting a main chain in the first linker and atoms constituting a main chain in the second linker are 5 to 7,
with an antibody comprising the immunoglobulin unit to give an antibody intermediate or salt thereof represented by the following formula (II): 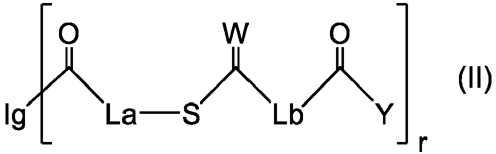
wherein
Ig indicates an immunoglobulin unit containing two heavy chains and two light chains, and forms an amide bond with the carbonyl group adjacent to Ig via an amino group in a side chain of a lysine residue present at the position 288/290 in the two heavy chains according to Eu numbering,
Y, O, S, W, La and Lb are the same as those of the above formula (I), and
the average ratio r of the amide bond per the two heavy chains is 1.5 to 2.5.

39. A method for producing a thiol group-introduced antibody derivative or salt thereof, which comprises
(1) reacting a compound or salt thereof represented by the following formula (I): 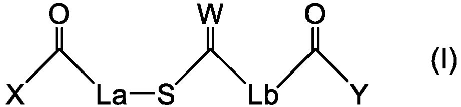 wherein
X indicates a leaving group,
Y indicates an affinity peptide having a binding region to a CH2 domain in an immunoglobulin unit containing two heavy chains and two light chains,
O indicates an oxygen atom,
S indicates a sulfur atom,
W indicates an oxygen atom or a sulfur atom,
La indicates a first linker,
Lb indicates a second linker, and
the total number of atoms constituting a main chain in the first linker and atoms constituting a main chain in the second linker are 5 to 7,
with an antibody comprising the immunoglobulin unit to give an antibody intermediate or salt thereof represented by the following formula (II): 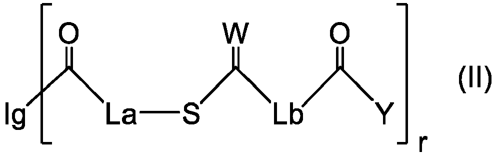
wherein
Ig indicates an immunoglobulin unit containing two heavy chains and two light chains, and forms an amide bond with the carbonyl group adjacent to Ig via an amino group in a side chain of a lysine residue present at the position 288/290 in the two heavy chains according to Eu numbering,
Y, O, S, W, La and Lb are the same as those of the above formula (I), and
the average ratio r of the amide bond per the two heavy chains is 1.5 to 2.5; and
(2) subjecting the antibody intermediate or salt thereof to a thioester cleavage reaction to give a thiol group-introduced antibody derivative or salt thereof comprising a structural unit represented by the following formula (III): 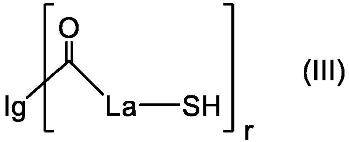 wherein
SH indicates a thiol group, and
Ig, La and r are the same as those of the above formula (II).

40. A method for producing a conjugate of an antibody and a functional substance or a salt thereof, which comprises
(1) reacting a compound or salt thereof represented by the following formula (I): 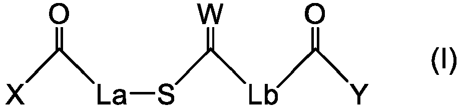 wherein
X indicates a leaving group,
Y indicates an affinity peptide having a binding region to a CH2 domain in an immunoglobulin unit containing two heavy chains and two light chains,
O indicates an oxygen atom,
S indicates a sulfur atom,
W indicates an oxygen atom or a sulfur atom,
La indicates a first linker,
Lb indicates a second linker, and
the total number of atoms constituting a main chain in the first linker and atoms constituting a main chain in the second linker are 5 to 7,
with an antibody comprising the immunoglobulin unit to give an antibody intermediate or salt thereof represented by the following formula (II): 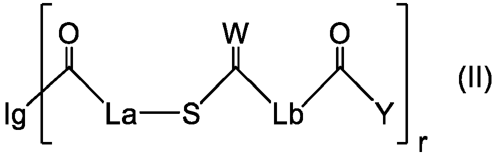
wherein
Ig indicates an immunoglobulin unit containing two heavy chains and two light chains, and forms an amide bond with the carbonyl group adjacent to Ig via an amino group in a side chain of a lysine residue present at the position 288/290 in the two heavy chains according to Eu numbering,
Y, O, S, W, La and Lb are the same as those of the above formula (I), and
the average ratio r of the amide bond per the two heavy chains is 1.5 to 2.5;
(2) subjecting the antibody intermediate or salt thereof to a thioester cleavage reaction to give a thiol group-introduced antibody derivative or salt thereof comprising a structural unit represented by the following formula (III): 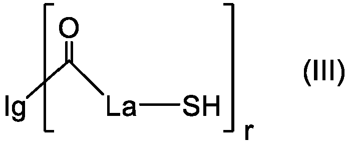 wherein
SH indicates a thiol group, and
Ig, La and r are the same as those of the above formula (II); and
(3) reacting the thiol group-introduced antibody derivative or salt thereof with a functional substance to give a conjugate of an antibody and a functional substance or a salt thereof, which comprises a structural unit represented by the following formula (IV): 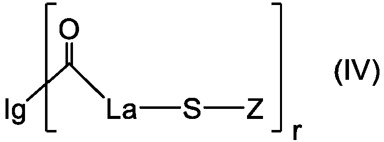 wherein
S indicates a sulfur atom,
Z indicates a functional substance,
Ig, O, La and r are the same as those in the above formula (III).

41. The method of any one of claims 38 to 40, further comprising reacting a compound or salt thereof represented by the following formula (VI): 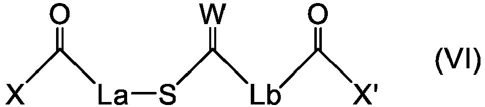 wherein
X indicates a leaving group,
X' indicates a leaving group having a leaving ability which is higher than that of the leaving group X.
O indicates an oxygen atom,
S indicates a sulfur atom,
W indicates an oxygen atom or a sulfur atom,
La indicates a first linker,
Lb indicates a second linker, and
the total number of atoms constituting a main chain in the first linker and the number of atoms constituting a main chain in the second linker are 5 to 7,
with the affinity peptide having a binding region to the CH2 domain in the immunoglobulin unit containing two heavy chains and two light chains
to give the compound or salt thereof represented by the formula (I).

42. The method of any one of claims 38 to 40, further comprising
(1') reacting a compound or salt thereof represented by the following formula (V): 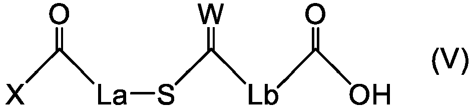 wherein
X indicates a leaving group,
O indicates an oxygen atom,
OH indicates a hydroxy group,
S indicates a sulfur atom,
W indicates an oxygen atom or a sulfur atom,
La indicates a first linker,
Lb indicates a second linker,
the total number of atoms constituting a main chain in the first linker and atoms constituting a main chain in the second linker are 5 to 7,
with a carboxyl group modifying reagent to give a compound or salt thereof represented by the following formula (VI): 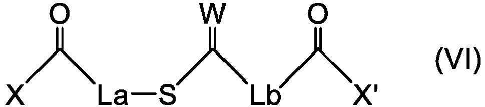
wherein
X, O, S, W, La and Lb are the same as those in the above formula (V), and
X' indicates a leaving group having a leaving ability which is higher than that of the leaving group X; and
(2') reacting the compound or salt thereof represented by the formula (VI) with an immunoglobulin unit containing two heavy chains and two light chains to give a compound or salt thereof represented by the above formula (I).

43. A method for producing a compound or salt thereof represented by formula (VI), which comprises reacting a compound or salt thereof represented by the following formula (V): 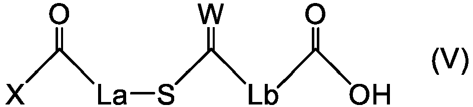 wherein
X indicates a leaving group,
O indicates an oxygen atom,
OH indicates a hydroxy group,
S indicates a sulfur atom,
W indicates an oxygen atom or a sulfur atom,
La indicates a first linker,
Lb indicates a second linker,
the total number of atoms constituting a main chain in the first linker and atoms constituting a main chain in the second linker are 5 to 7,
with a carboxyl group modifying reagent to give the compound or salt thereof represented by the following formula (VI): 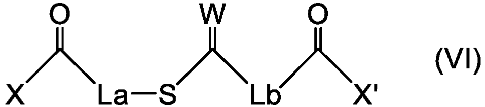
wherein
X, O, S, W, La and Lb are the same as those in the above formula (V), and
X' indicates a leaving group having a leaving ability which is higher than that of the leaving group X.

44. A method for producing a thiol group-introduced antibody derivative or salt thereof, which comprises subjecting an antibody intermediate or salt thereof comprising a structural unit represented by the following formula (II): 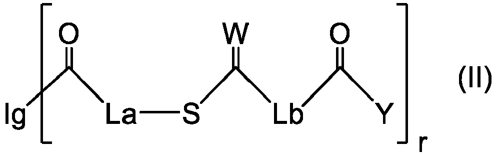 wherein
Ig indicates an immunoglobulin unit containing two heavy chains and two light chains, and forms an amide bond with the carbonyl group adjacent to Ig via an amino group in a side chain of a lysine residue present at the position 288/290 in the two heavy chains according to Eu numbering,
Y indicates an affinity peptide having a binding region to a CH2 domain in the immunoglobulin unit,
O indicates an oxygen atom,
S indicates a sulfur atom,
W indicates an oxygen atom or a sulfur atom,
La indicates a first linker,
Lb indicates a second linker,
the total number of atoms constituting a main chain in the first linker and atoms constituting a main chain in the second linker are 5 to 7, and
the average ratio r of the amide bond per the two heavy chains is 1.5 to 2.5,
to a thioester cleavage reaction to give a thiol group-introduced antibody derivative or salt thereof, which comprises a structural unit represented by the following formula (III): 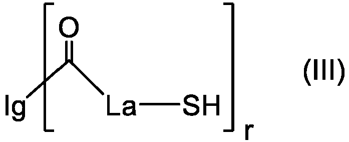
wherein
SH indicates a thiol group, and
Ig, O, La and r are the same as those of the above formula (II).

45. A method for producing a conjugate of an antibody and a functional substance or a salt thereof, which comprises
(1) subjecting an antibody intermediate or salt thereof comprising a structural unit represented by the following formula (II): 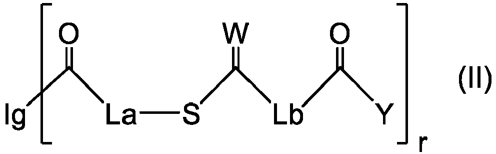 wherein
Ig indicates an immunoglobulin unit containing two heavy chains and two light chains, and forms an amide bond with the carbonyl group adjacent to Ig via an amino group in a side chain of a lysine residue present at the position 288/290 in the two heavy chains according to Eu numbering,
Y indicates an affinity peptide having a binding region to a CH2 domain in the immunoglobulin unit,
O indicates an oxygen atom,
S indicates a sulfur atom,
W indicates an oxygen atom or a sulfur atom,
La indicates a first linker,
Lb indicates a second linker,
the total number of atoms constituting a main chain in the first linker and atoms constituting a main chain in the second linker are 5 to 7, and
the average ratio r of the amide bond per the two heavy chains is 1.5 to 2.5,
to a thioester cleavage reaction to give a thiol group-introduced antibody derivative or salt thereof, which comprises a structural unit represented by the following formula (III): 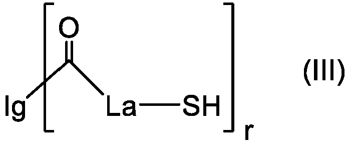
wherein
SH indicates a thiol group, and
Ig, O, La and r are the same as those of the above formula (II); and
(2) reacting the thiol group-introduced antibody derivative or salt thereof with a functional substance to give the conjugate of the antibody and the functional substance or a salt thereof which is represented by the following formula (IV) : 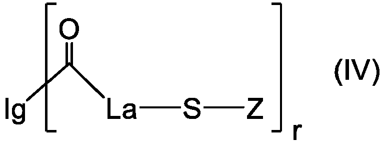 wherein
S indicates a sulfur atom,
Z indicates a functional substance, and
Ig, O, La and r are the same as those in the above formula (III).

46. A method for producing a conjugate of an antibody and a functional substance or a salt thereof, which comprises reacting a thiol group-introduced antibody derivative or salt thereof, which comprises a structural unit represented by the following formula (III): 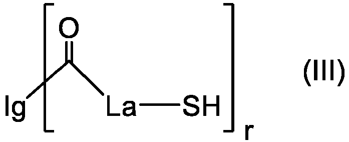 wherein
Ig indicates an immunoglobulin unit containing two heavy chains and two light chains, and forms an amide bond with the carbonyl group adjacent to Ig via an amino group in a side chain of a lysine residue present at the position 288/290 in the two heavy chains according to Eu numbering,
O indicates an oxygen atom,
SH indicates a thiol group,
La indicates a first linker,
the number of atoms constituting the main chain in the first linker is 2 to 4, and
the average ratio r of the amide bonds per the two heavy chains is 1.5 to 2.5,
with a functional substance to give a conjugate of an antibody and a functional substance or a salt thereof, which comprises a structural unit represented by the following formula (IV): 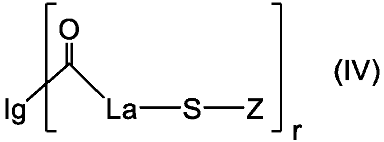
wherein
S indicates a sulfur atom,
Z indicates a functional substance, and
Ig, O, La and r are the same as those in the above formula (III).
